(19) Europäisches Patentamt
European Patent Office
Office européen des brevets

(11) EP 2 958 928 B1

(12) **EUROPÄISCHE PATENTSCHRIFT**

(45) Veröffentlichungstag und Bekanntmachung des Hinweises auf die Patenterteilung:
**28.03.2018 Patentblatt 2018/13**

(21) Anmeldenummer: **14704817.7**

(22) Anmeldetag: **18.02.2014**

(51) Int Cl.:
***C07J 43/00*** *(2006.01)* ***A61K 31/58*** *(2006.01)*
***A61P 5/24*** *(2006.01)* ***A61P 5/28*** *(2006.01)*

(86) Internationale Anmeldenummer:
**PCT/EP2014/053094**

(87) Internationale Veröffentlichungsnummer:
**WO 2014/128108 (28.08.2014 Gazette 2014/35)**

(54) **ESTRA-1,3,5(10),16-TETRAEN-3-CARBOXAMIDE ZUR INHIBIERUNG VON 17.BETA.-HYDROXYSTEROID DEHDROGENASE (AKR1C3)**

ESTRA-1,3,5(10),16-TETRAENE-3-CARBOXAMIDES FOR INHIBITION OF 17.BETA.-HYDROXYSTEROID DEHYDROGENASE (AKR1 C3)

ESTRA-1,3,5(10),16-TÉTRAÉNO-3-CARBOXAMIDE POUR INHIBER LA 17-BÊTA-HYDROXYSTÉROÏDE DÉSHYDROGÉNASE (AKR1C3)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **21.02.2013 EP 13156125**

(43) Veröffentlichungstag der Anmeldung:
**30.12.2015 Patentblatt 2015/53**

(73) Patentinhaber: **Bayer Pharma Aktiengesellschaft**
**13353 Berlin (DE)**

(72) Erfinder:
- **BOTHE, Ulrich**
  **13187 Berlin (DE)**
- **BUSEMANN, Matthias**
  **13467 Berlin (DE)**
- **BARAK, Naomi**
  **12049 Berlin (DE)**
- **ROTGERI, Andrea**
  **13503 Berlin (DE)**
- **FISCHER, Oliver Martin**
  **13127 Berlin (DE)**
- **MARQUARDT, Tobias**
  **42115 Wuppertal (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A1-2013/045407 WO-A1-2014/009274**
**WO-A2-00/07576 WO-A2-99/46279**

- **MOSTAGHEL E A ET AL: "Resistance to CYP17A1 inhibition with abiraterone in castration-resistant prostate cancer: induction of steroidogenesis and androgen receptor splice variants", CLINICAL CANCER RESEARCH, THE AMERICAN ASSOCIATION FOR CANCER RESEARCH, US, Bd. 17, Nr. 18, 15. September 2011 (2011-09-15), Seiten 5913-5925, XP002712865, ISSN: 1078-0432, DOI: 10.1158/1078-0432.CCR-11-0728 [gefunden am 2011-08-01]**
- **LI R ET AL: "Abiraterone inhibits 3[beta]-hydroxysteroid dehydrogenase: a rationale for increasing drug exposure in castration-resistant prostate cancer", CLINICAL CANCER RESEARCH, THE AMERICAN ASSOCIATION FOR CANCER RESEARCH, US, Bd. 18, Nr. 13, 1. Juli 2012 (2012-07-01), Seiten 3571-3579, XP002712888, ISSN: 1078-0432, DOI: 10.1158/1078-0432.CCR-12-0908**

EP 2 958 928 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft AKR1C3 Inhibitoren und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Blutungsbeschwerden und Endometriose.

Die Aldo-Keto-Reduktase 1C3 (AKR1C3; Synonyme: Typ 5 17β-Hydroxysteroid Dehydrogenase oder Prostaglandin F Synthase) ist ein multifunktionales Enzym und katalysiert unter anderem die Reduktion von 4-Androsten-3,17-dion (einem schwachen Androgen) zu Testosteron (einem potenten Androgen) und von Estron (einem schwachen Estrogen) zu 17β-Estradiol (einem starken Estrogen). Zusätzlich wird die Reduktion von Prostaglandin (PG) H2 zu PGF2α und PGD2 zu 9α,11β-PGF2 inhibiert (T. M. Penning et. al., 2006, 'Aldo-keto reductase (AKR) 1C3: Role in prostate disease and the development of specific inhibitors', Molecular and Cellular Endocrinology 248(1-2), 182 -191).

Die lokale Bildung von Estradiol (E2) spielt eine zentrale Rolle für die Initiierung und das Fortschreiten von Brustkrebserkrankungen und Endometriose. Die Reduktion der Gewebespiegel von Estrogenen und insbesondere von Estradiol wird erreicht durch die therapeutische Gabe von Aromataseinhibitoren (um die Bildung von Estrogenen aus Androgenen zu inhibieren) und von Sulfataseinhibitoren (um die Bildung von Estron aus Estronsulfat zu blockieren). Beide Therapieansätze haben aber den Nachteil, dass systemische Estrogenspiegel radikal reduziert werden (A. Oster et. al., J. Med. Chem. 2010, 53, 8176-8186). Kürzlich wurde experimentell nachgewiesen, dass endometriotische Läsionen in der Lage sind, lokal Estradiol zu synthetisieren (B. Delvoux et al., J Clin Endocrinol Metab. 2009, 94, 876-883). Für den Subtyp der ovariellen Endometriose wurde eine Überexpression der AKR1C3 mRNA beschrieben (T. Smuc et al., Mol Cell Endocrinol. 2009 Mar 25;301(1-2): 59-64).

An der Identifizierung von neuen Inhibitoren des Enzyms AKR1C3 besteht großer Bedarf, da Inhibitoren ein Potential für die Behandlung von hormonabhängigen Erkrankungen, wie zum Beispiel Endometriose, aber auch für die Behandlung von hormonunabhängigen Erkrankungen haben (M.C. Byrns, Y. Jin, T.M. Penning, Journal of Steroid Biochemistry and Molecular Biology (2010); A. L. Lovering et. al., Cancer Res 64(5), 1802-1810). Neben der Endometriose zählen dazu auch Prostatakrebs (K. M. Fung et al., Endocr Relat Cancer 13(1), 169-180), Prostatahyperplasie (R. O. Roberts et al., Prostate 66(4), 392-404), Endometriumskarzinom (T. L. Rizner et al., Mol Cell Endocrinol 2006 248(1-2), 126-135), polyzystisches ovarielles Syndrom (K. Qin et al., J Endocrinol Metab 2006, 91(1), 270-276), Lungenkarzinom (Q. Lan et al., Carcinogenesis 2004, 25(11), 2177-2181), non-Hodgkin-Lymphom (Q. Lan et al., Hum Genet 2007, 121(2), 161-168), Haarausfall (L. Colombe et al., Exp Dermatol 2007, 16(9), 762-769), Adipositas (P. A. Svensson et al., Cell Mol Biol Lett 2008, 13(4), 599-613), Blasenkarzinom (J. D. Figueroa, Carcinogenesis 2008, 29(10), 1955-1962), chronische myeloische Leukämie (J. Birtwistle, Mutat Res 2009, 662(1-2), 67-74), Nierenzellkarzinom (J. T. Azzarello, Int J Clin Exp Pathol 2009, 3(2), 147-155), Brustkrebs (M. C. Byrns, J Steroid Biochem Mol Biol 2010, 118(3), 177-187), die frühzeitige Geschlechtsreife (C. He, Hum Genet 2010, 128(5), 515-527) und die chronisch-obstruktive Lungenerkrankung (S. Pierrou, Am J Respir Crit Care 2007, 175(6), 577-586).

Einige Inhibitoren von AKR1C3 sind bekannt (Übersichtsartikel: Joanna M Day, Helena J Tutill, Atul Purohit und Michael J Reed, Endocrine-Related Cancer (2008) 15, 665-692, siehe auch Patentanmeldungen US20100190826, WO2007/100066 sowie P. Brožič et al., J. Med. Chem. 2012, 55, 7417-7424, A. O. Adenijii et al., J. Med. Chem. 2012, 55, 2311-2323 und S. M. F. Jamieson et al., J. Med. Chem. 2012, 55, 7746-7758). Als steroidale Substanz wurde zum Beispiel EM-1404 basierend auf dem Estratrien-Gerüst mit einer Spirolactoneinheit an Position 17 beschrieben (F. Labrie et al. US Patent 6,541,463; 2003).

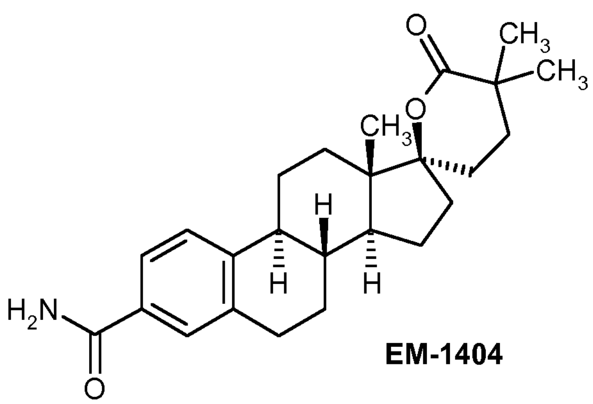

**EM-1404**

Weitere steroidale AKR1C3 Inhibitoren mit Lactoneinheit wurden in P. Bydal, Van Luu-The, F. Labrie, D. Poirier, European Journal of Medicinal Chemistry 2009, 44, 632-644 beschrieben. Fluorierte Estratrienderivate wurden beschrieben in D. Deluca, G. Moller, A. Rosinus, W. Elger, A. Hillisch, J. Adamski, Mol. Cell. Endocrinol. 2006, 248, 218 - 224.

In dem US Patent US 5,604,213 (S. E. Barrie et al.) wurde 17-(3-Pyridyl)estra-1,3,5(10),16-tetraen-3-ol, eine Struktur, die am Kohlenstoffatom 3 durch eine freie Hydroxygruppe substituiert ist, als 17$\alpha$-Hydroxylase/C17-20 Lyase (Cyp17A1) Inhibitor, aber nicht als AKR1C3 Inhibitor beschrieben.

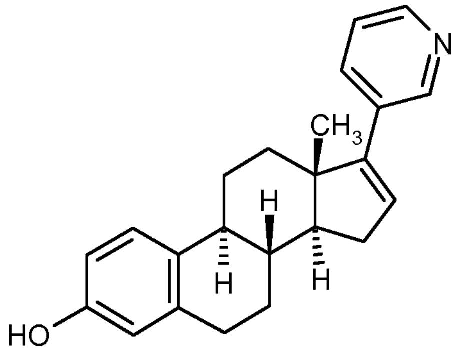

17-(3-Pyridyl)estra-1,3,5(10),16-tetraen-3-ol 17-(3-Pyridyl)estra-1,3,5(10),16-tetraen-Derivate, die an Position 3 mit einer Carboxamidgruppe substituiert sind, werden in dem US Patent US 5,604,213 nicht beschrieben.

In der Anmeldung US2005/0203075 werden Estra-1,3,5(10),16-tetraen-Derivate, die mit einer $-CONH_2$-Gruppe an 3-Position substituiert sind, als antiproliferativ und antiangiogenetisch wirksam ohne Bezug auf ein konkretes molekulares Target beschrieben. Diese Derivate sind jedoch nicht mit einem Heterocyclus an Position 17 des Estra-1,3,5(10),16-tetraen-Gerüstes substituiert.

Eine Übersicht über 17-Pyridyl- und 17-Pyrimidinylandrostan-Derivate, die als Cyp17A1 Inhibitoren beschrieben werden, ist in V. M. Moreira et al. Current Medicinal Chemistry, 2008 Vol. 15, No. 9 zu finden.

Obwohl zahlreiche AKR1C3 Inhibitoren beschrieben sind, besteht nach wie vor ein Bedarf an Substanzen mit verbesserten Eigenschaften wie beispielsweise verbesserter Löslichkeit.

Aufgabe der vorliegenden Erfindung ist es, als AKR1C3 Inhibitoren wirkende Stoffe mit verbesserten Eigenschaften wie beispielsweise mit verbesserter Löslichkeit zur Verfügung zu stellen.

Gegenstand der vorliegenden Erfindung sind Verbindungen der Formel (I)

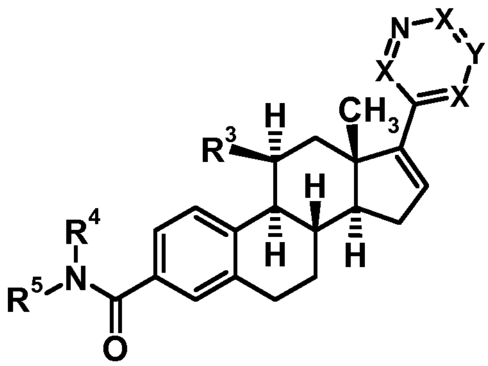

**(I)**

wobei

| | |
|---|---|
| X | unabhängig voneinander Kohlenstoff oder Stickstoff, wobei der Kohlenstoff mit $R^1$ substituiert sein kann, |
| Y | Kohlenstoff oder Stickstoff, wobei der Kohlenstoff mit $R^2$ substituiert sein kann, |
| $R^1$ und $R^2$ | unabhängig voneinander Wasserstoff, Halogen, $C_1$-$C_6$-Alkyl, $C_2$-$C_6$-Alkenyl, $C_2$-$C_4$-Alkinyl, $C_3$-$C_6$-Cycloalkyl, $C_1$-$C_6$-Alkoxy, $C_1$-$C_6$-Halogenalkyl, $C_1$-$C_6$-Halogenalkoxy, Nitril, Nitro, $-SO_2CH_3$, $-SO_2CH_2CH_3$, $-(C{=}O)CH_3$, Carboxyl, Hydroxyl, $-NH_2$, $-CH_2NH_2$, $-CH_2OH$, $-CH(OH)CH_3$, $-C(CH_3)_2OH$, $-(C{=}O)NH_2$, $-(C{=}O)NHCH_3$, $-(C{=}O)NHCH_2CH_3$, $-(C{=}O)N(CH_3)_2$, $-SO_2NH_2$, $-SO_2NHCH_3$ oder $-SO_2N(CH_3)_2$, |
| $R^3$ | Wasserstoff oder Halogen, |

$R^4$ Wasserstoff, $C_1$-$C_6$-Alkyl, $C_3$-$C_6$-Cycloalkyl oder $C_3$-$C_6$-Cycloalkyl-$C_1$-$C_6$-alkyl, welche gegebenenfalls mit bis zu 6 Halogenatomen substituiert sind und gegebenenfalls einfach oder zweifach substituiert sind mit Hydroxyl, $C_1$-$C_6$-Alkoxy, $C_1$-$C_6$-Halogenalkoxy,

$R^5$ Wasserstoff, $C_1$-$C_6$-Alkyl oder $C_3$-$C_6$-Cycloalkyl, wobei $C_1$-$C_6$-Alkyl und $C_3$-$C_6$-Cycloalkyl gegebenenfalls mit bis zu 6 Halogenatomen substituiert sind und gegebenenfalls einfach oder zweifach substituiert sind mit Hydroxyl, Hydroxy-$C_1$-$C_6$-alkyl, $C_1$-$C_6$-Alkyl, $C_1$-$C_6$-Alkoxy, Hydroxy-$C_2$-$C_6$-alkoxy, $C_1$-$C_6$-Halogenalkoxy, $C_3$-$C_6$-Cycloalkyl, Aryl, Heteroaryl, 3-10gliedriges Heterocycloalkyl, Aryl-$C_1$-$C_6$-alkyl, Heteroaryl-$C_1$-$C_6$-alkyl, -C(=O)R', -C(=O)$NH_2$, -C(=O)N(H)R', -C(=O)N(R')R", -$NH_2$, -NHR' -N(R')R", -N(H)C(=OR', -N(R')C(=O)R', -N(H)C(=O)OR', -N(R')C(=O)OR', -$NO_2$, -N(H)S(=O)R', -N(R')S(=O)R', -N(H)S(=O)$_2$R', -N(R')S(=O)$_2$R', -N=S(=O)(R')R", -S(=O)R', -S(=O)$_2$R', -S(=O)$_2$$NH_2$, -S(=O)$_2$NHR', -S(=O)$_2$N(R')R", -S(=O)(=NR')R", wobei Aryl, Heteroaryl, Aryl-$C_1$-$C_6$-alkyl und Heteroaryl-$C_1$-$C_6$-alkyl gegebenenfalls einfach oder mehrfach unabhängig voneinander substituiert sind mit $R^6$ und 3-10gliedriges Heterocycloalkyl gegebenenfalls einfach oder mehrfach unabhängig voneinander substituiert ist mit R',
oder

$R^4$ und $R^5$ gemeinsam mit dem direkt verknüpften Stickstoffatom einen 4-7gliedrigen Ring, welcher gegebenenfalls substituiert ist mit einem oder zwei Substituenten aus der Gruppe bestehend aus:
Halogen, Nitril, Hydroxyl, $C_1$-$C_6$-Alkyl, $C_1$-$C_6$-Halogenalkyl, $C_2$-$C_6$-Alkenyl, $C_2$-$C_4$-Alkinyl, $C_3$-$C_{10}$-Cycloalkyl, Aryl, Heteroaryl, -C(=O)$NH_2$, -C(=O)N(H)R', -C(=O)N(R')R", -C(=O)OH, -C(=O)OR', -$NH_2$, -NHR', -N(R')R", -N(H)C(=OR', -N(R')C(=O)R', -N(H)S(=O)R', -N(R')S(=O)R', -N(H)S(=O)$_2$R', -N(R')S(=O)$_2$R', -N=S(=O)(R')R", -OH, $C_1$-$C_6$-Alkoxy, $C_1$-$C_6$-Halogenalkoxy, -OC(=O)R', -OC(=O)$NH_2$, -OC(=O)NHR', -OC(=O)N(R')R", -SH, $C_1$-$C_6$-Alkyl-S-, -S(=O)R', -S(=O)$_2$R', -S(=O)$_2$$NH_2$, -S(=O)$_2$NHR', -S(=O)$_2$N(R')R", wobei Aryl und Heteroaryl gegebenenfalls einfach oder mehrfach unabhängig voneinander substituiert sind mit $R^6$ und in welchem 5-, 6- oder 7-gliedrigen Ring gegebenenfalls eine oder mehrere Methylengruppen durch NH, NR', O oder S ersetzt sind,

$R^6$ Halogen, Nitril, $C_1$-$C_6$-Alkyl, $C_1$-$C_6$-Halogenalkyl, $C_2$-$C_6$-Alkenyl, $C_2$-$C_4$-Alkinyl, $C_3$-$C_{10}$-Cycloalkyl, 3-10gliedriges Heterocycloalkyl, Aryl, Heteroaryl, -C(=O)R', -C(=O)$NH_2$,-C(=O)N(H)R',-C(=O)N(R')R", -C(=O)OR', -$NH_2$, -NHR', -N(R')R", -N(H)C(=OR', -N(R')C(=O)R', -N(H)C(=O)$NH_2$, -N(H)C(=O)NHR', -N(H)C(=O)N(R')R", -N(R')C(=O)$NH_2$, -N(R')C(=O)NHR', -N(R')C(=O)N(R')R", -N(H)C(=O)OR', -N(R')C(=O)OR', -$NO_2$, -N(H)S(=O)R',-N(R')S(=O)R', -N(H)S(=O)$_2$R', -N(R')S(=O)$_2$R, -N=S(=O)(R')R", -OH, $C_1$-$C_6$-Alkoxy, $C_1$-$C_6$-Halogenalkoxy, -OC(=O)R', -OC(=O)$NH_2$, -OC(=O)NHR', -OC(=O)N(R')R", -SH, $C_1$-$C_6$-Alkyl-S-, -S(=O)R', -S(=O)$_2$R', -S(=O)$_2$$NH_2$, -S(=O)$_2$NHR', -S(=O)$_2$N(R')R", - S(=O)(=NR')R"

R' und R" unabhängig voneinander $C_1$-$C_6$-Alkyl, $C_3$-$C_{10}$-Cycloalkyl oder $C_1$-$C_6$-Halogenalkyl bedeuten oder deren Tautomere, N-Oxide, Hydrate, Solvate oder Salze oder eine Mischung bestehend aus den Vorgenannten.

Erfindungsgemäße Verbindungen sind die Verbindungen der Formel (I) und deren Tautomere, N-Oxide, Hydrate, Salze, Solvate und Solvate der Salze, sowie die von Formel (I) umfassten, nachfolgend als Ausführungsbeispiele genannten Verbindungen und deren Tautomere, N-Oxide, Hydrate, Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen

der vorliegenden Erfindung Hydrate bevorzugt.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein, beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Im Rahmen der vorliegenden Erfindung haben die Substituenten, soweit nicht anders spezifiziert, die folgende Bedeutung:

<u>Alkyl</u> steht im Rahmen der Erfindung für einen linearen oder verzweigten Alkylrest mit der jeweils angegebenen Anzahl an Kohlenstoffatomen. Beispielhaft seien genannt: Methyl, Ethyl, n-Propyl, Isopropyl, n-Butyl, iso-Butyl, 1-Methylpropyl, tert.-Butyl, n-Pentyl, Neopentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 1-Ethylbutyl und 2-Ethylbutyl. Bevorzugt sind: Methyl, Ethyl, n-Propyl, Isopropyl, n-Butyl, 2-Methylbutyl und Neopentyl.

<u>Alkenyl</u> steht im Rahmen der Erfindung für einen linearen oder verzweigten Alkenylrest mit 2 bis 6 Kohlenstoffatomen und einer Doppelbindung. Bevorzugt ist ein geradkettiger oder verzweigter Alkenylrest mit 2 bis 4 Kohlenstoffatomen und einer Doppelbindung. Beispielhaft seien genannt: Vinyl, Allyl, Isopropenyl und n-But-2-en-1-yl.

<u>Alkinyl</u> steht im Rahmen der Erfindung für einen linearen oder verzweigten Alkinylrest mit 2 bis 4 Kohlenstoffatomen und einer Dreifachbindung. Beispielhaft seien genannt: Ethinyl, n-Prop-1-in-1-yl, n-Prop-2-in-1-yl, n-But-2-in-1-yl und n-But-3-in-1-yl.

<u>Cycloalkyl</u> steht in Rahmen der Erfindung für einen monocyclischen, gesättigten Alkylrest mit der jeweils angegebenen Anzahl an Kohlenstoffatomen. Beispielhaft seien genannt: Cyclopropyl, Cyclobutyl, Cyclopentyl und Cyclohexyl. Bevorzugt ist Cyclopentyl.

<u>Hydroxy-$C_1$-$C_6$-alkyl</u> steht im Rahmen der Erfindung für einen linearen oder verzweigten Alkylrest mit 1 bis 6 Kohlenstoffatomen, der in der Kette oder endständig eine Hydroxy-Gruppe als Substituenten trägt. Beispielhaft seien genannt: Hydroxymethyl, 1-Hydroxyethyl, 2-Hydroxyethyl, 1-Hydroxy-1-methylethyl, 1,1-Dimethyl-2-hydroxyethyl, 1-Hydroxypropyl, 2-Hydroxypropyl, 3-Hydroxypropyl, 1-Hydroxy-2-methylpropyl, 2-Hydroxy-1-methylpropyl, 2-Hydroxy-2-methylpropyl, 1-Hydroxybutyl, 2-Hydroxybutyl, 3- Hydroxybutyl und 4-Hydroxybutyl. Bevorzugt sind: Hydroxymethyl, 2-Hydroxyethyl und 3-Hydroxypropyl.

<u>Alkoxy</u> steht im Rahmen der Erfindung für einen linearen oder verzweigten Alkoxyrest mit 1 bis 6 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft seien genannt: Methoxy, Ethoxy, n-Propoxy, 1-Methylpropoxy, n-Butoxy und iso-Butoxy. Bevorzugt ist Methoxy.

<u>Hydroxy-$C_2$-$C_6$-alkoxy</u> steht im Rahmen der Erfindung für einen linearen oder verzweigten Alkoxyrest mit 2 bis 6 Kohlenstoffatomen, der in der Kette oder endständig eine Hydroxy-Gruppe als Substituenten trägt. Beispielhaft

seien genannt: 2-Hydroxyethoxy und 2-Hydroxypropoxy. Bevorzugt ist 2-Hydroxyethoxy.

Cycloalkoxy steht im Rahmen der Erfindung für einen monocyclischen, gesättigten Carbocyclus mit 3 bis 7 Kohlenstoffatomen, der über ein Sauerstoffatom gebunden ist. Beispielhaft seien genannt: Cyclopropyloxy, Cyclobutyloxy, Cyclopentyloxy, Cyclohexyloxy und Cycloheptyloxy.

Heterocycloalkyl, bzw. Heterocyclus steht im Rahmen der Erfindung für einen gesättigten Heterocyclus mit insgesamt 3 bis 10 Ringatomen, der ein oder zwei Ring-Heteroatome aus der Reihe N, O, S, SO und/oder $SO_2$ enthält. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Dioxidothiomorpholinyl, Dihydroindolyl und Dihydroisoindolyl. Bevorzugt sind: Azetidinyl, Oxetanyl, Pyrrolidinyl, Piperidinyl, Piperazinyl und Morpholinyl.

Aryl steht im Rahmen der Erfindung für einen mono- bis tricyclischen aromatischen, carbocyclischen Rest mit in der Regel 6 bis 14 Kohlenstoffatomen. Beispielhaft seien genannt: Phenyl, Naphthyl und Phenanthrenyl. Bevorzugt ist Phenyl.

Heteroaryl steht im Rahmen der Erfindung für einen monocyclischen aromatischen Heterocyclus (Heteroaromaten) mit insgesamt 5 oder 6 Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Tetrazolyl, Oxadiazolyl, Thiadiazolyl, Oxathiadiazolyl, Pyridyl, Pyrimidinyl, Pyridazinyl, Pyrazinyl und Triazinyl. Bevorzugt sind: Pyrazolyl, 1H-Tetrazol-5-yl, Oxadiazolyl, Thiadiazolyl, Pyridyl und Pyrimidinyl.
Der oben genannte monocyclische aromatische Heterocyclus ist gegebenenfalls mit Hydroxyl oder -SH substituiert, wobei Heteroaryl für alle möglichen tautomeren Formen steht. Beispielhaft seien genannt: 5-Oxo-4,5-dihydro-1,2,4-thiadiazol-3-yl, 5-Oxo-4,5-dihydro-1H-1,2,4-triazol-3-yl, 5-Thioxo-4,5-dihydro-1,2,4-oxadiazol-3-yl, 5-Oxo-4,5-dihydro-1,2,4-oxadiazol-3-yl, 2-Oxido-3H-1,2,3,5-oxathiadiazol-4-yl, 3-Oxo-2,3-dihydro-1,2,4-oxadiazol-5-yl.

Aryl-$C_1$-$C_6$-alkyl steht im Rahmen der Erfindung für einen mono- bis tricyclischen aromatischen, carbocyclischen Rest mit in der Regel 6 bis 14 Kohlenstoffatomen, der über einen linearen oder verzweigten Alkylrest mit der jeweils angegebenen Anzahl an Kohlenstoffatomen gebunden ist.

Heteroaryl-$C_1$-$C_6$-alkyl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist und der über einen linearen oder verzweigten Alkylrest mit der jeweils angegebenen Anzahl an Kohlenstoffatomen gebunden ist.

$C_3$-$C_6$-Cycloalkyl-$C_1$-$C_6$-alkyl steht in Rahmen der Erfindung für einen monocyclischen, gesättigten Alkylrest mit der jeweils angegebenen Anzahl an Kohlenstoffatomen, der über einen linearen oder verzweigten Alkylrest mit der jeweils angegebenen Anzahl an Kohlenstoffatomen gebunden ist.

Halogen steht im Rahmen der Erfindung für Fluor, Chlor, Brom und Iod. Bevorzugt sind Fluor und Chlor.

Hydroxyl steht im Rahmen der Erfindung für eine Hydroxy-Gruppe.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Ebenfalls Gegenstand der Erfindung sind Verbindungen der Formel (I), wobei

X Kohlenstoff substituiert mit Wasserstoff,

Y Kohlenstoff oder Stickstoff, wobei der Kohlenstoff mit $R^2$ substituiert sein kann,

$R^2$ Wasserstoff, Fluor, Chlor, Nitril, Methoxy, Ethoxy, Trifluormethoxy, Methyl, Ethyl, Trifluormethyl, $-(C=O)CH_3$,

$R^3$ Wasserstoff oder Fluor,

$R^4$ Wasserstoff, Methyl, Ethyl, Isopropyl, Propyl, Butyl, Cyclopropyl oder 2,2,2-Trifluorethyl,

$R^5$ Wasserstoff, Methyl, Ethyl, Propyl, 2,2,2-Trifluorethyl, 3,3,3-Trifluorpropyl, 2-Fluorethyl, 2-Sulfamoylethyl, 3-Sulfamoylpropyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1*H*-Tetrazol-5-yl)ethyl, 1*H*-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, 4-Hydroxybutyl, (2*RS*)-2,3-Dihydroxypropyl, (2*R*)-2,3-Dihydroxypropyl, 2,3-Dihydroxybutyl, 2-(Methylsulfinyl)ethyl, 3-(Methylsulfinyl)propyl, 2-(Methylsulfonyl)ethyl, 3-(Methylsulfonyl)propyl, 2-(*S*-Methylsulfonimidoyl)ethyl, (2*R*)-2-Hydroxypropyl, (2*S*)-1-Hydroxypropan-2-yl, 2-Methoxyethyl, 3-Methoxypropyl, 2-(Isopropylsulfonyl)ethyl, (3-Methyloxetan-3-yl)methyl, (2*S*)-2-Hydroxypropyl, 2-(2-Hydroxyethoxy)ethyl oder

$R^4$ und $R^5$ gemeinsam mit dem direkt verknüpften Stickstoffatom Piperidinyl, Pyrrolidinyl, Morpholinyl, N-Methylpiperazinyl, 1-Oxidothiomorpholinyl, 1,1-Dioxidothiomorpholin-4-yl, 4-Hydroxypiperidinyl, 4-(Trifluormethyl)piperidin-4-yl, (3*R*)-3-Hydroxypiperidinyl, (2*S*)-2-(1*H*-Tetrazol-5-yl)pyrrolidinyl, *N*-Methyl-L-prolinamidyl und L-Prolinamidyl

bedeuten oder deren Tautomere, N-Oxide, Hydrate, Solvate oder Salze oder eine Mischung bestehend aus den Vorgenannten.

Auch Gegenstand der Erfindung sind Verbindungen der Formel (I) wobei

X Kohlenstoff substituiert mit Wasserstoff,

Y Kohlenstoff oder Stickstoff, wobei der Kohlenstoff mit $R^2$ substituiert sein kann,

$R^2$ Wasserstoff, Fluor, Chlor, Methyl, Nitril, Methoxy, Trifluormethyl,

$R^3$ Wasserstoff oder Fluor,

$R^4$ Wasserstoff, Methyl, Ethyl, Isopropyl, Propyl oder Cyclopropyl,

$R^5$ Wasserstoff, Methyl, Ethyl, 2-Sulfamoylethyl, 3-Sulfamoylpropyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1*H*-Tetrazol-5-yl)ethyl, 1*H*-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, (2*RS*)-2,3-Dihydroxypropyl, (2*R*)-2,3-Dihydroxypropyl, 2-(Methylsulfinyl)ethyl, (2*R*)-2-Hydroxypropyl, (2*S*)-1-Hydroxypropan-2-yl, 2-Methoxyethyl, 2-(Isopropylsulfonyl)ethyl, (3-Methyloxetan-3-yl)methyl, (2*S*)-2-Hydroxypropyl oder 2-(2-Hydroxyethoxy)ethyloder

$R^4$ und $R^5$ gemeinsam mit dem direkt verknüpften Stickstoffatom, Piperidinyl, Pyrrolidinyl, Morpholinyl, 4-Hydroxypiperidinyl, (3*R*)-3-Hydroxypiperidinyl, (2*S*)-2-(1*H*-Tetrazol-5-yl)pyrrolidinyl, *N*-Methyl-L-prolinamidyl oder L-Prolinamidyl

bedeuten oder deren Tautomere, N-Oxide, Hydrate, Solvate oder Salze oder eine Mischung bestehend aus den Vorgenannten.

Gegenstand der Erfindung sind außerdem Verbindungen der Formel (I)
wobei

X Kohlenstoff substituiert mit Wasserstoff,

Y Kohlenstoff oder Stickstoff, wobei der Kohlenstoff mit $R^2$ substituiert sein kann,

$R^2$ Wasserstoff, Fluor, Nitril, Methoxy oder Trifluormethyl,

$R^3$ Wasserstoff oder Fluor,

$R^4$ Wasserstoff, Methyl, Ethyl oder Isopropyl,

$R^5$ Wasserstoff, Ethyl, 2-Sulfamoylethyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1H-Tetrazol-5-yl)ethyl, 1H-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, (2*RS*)-2,3-Dihydroxypropyl, (2*R*)-2,3-Dihydroxypropyl, 2-(Methylsulfinyl)ethyl, (2*R*)-2-Hydroxypropyl, (2*S*)-1-Hydroxypropan-2-yl, 2-Methoxyethyl, 2-(Isopropylsulfonyl)ethyl, (3-Methyloxetan-3-yl)methyl, (2*S*)-2-Hydroxypropyl oder 2-(2-Hydroxyethoxy)ethyl
oder

$R^4$ und $R^5$ gemeinsam mit dem direkt verknüpften Stickstoffatom 4-Hydroxypiperidinyl, (3*R*)-3-Hydroxypiperidinyl, (2*S*)-2-(1*H*-Tetrazol-5-yl)pyrrolidinyl, *N*-Methyl-L-prolinamidyl oder L-Prolinamidyl

bedeuten oder deren Tautomere, N-Oxide, Hydrate, Solvate oder Salze oder eine Mischung bestehend aus den Vorgenannten.

Weiterhin sind Gegenstand der Erfindung die Verbindungen:

1. 17-(3-Pyridyl)estra-1,3,5(10),16-tetraen-3-carboxamid
2. 17-(5-Methoxypyridin-3-yl)estra-1,3,5(10),16-tetraen-3-carboxamid
3. 17-[5-(Trifluormethyl)pyridin-3-yl]estra-1,3,5(10),16-tetraen-3-carboxamid
4. 17-(5-Fluorpyridin-3-yl)estra-1,3,5(10),16-tetraen-3-carboxamid
5. 17-(Pyrimidin-5-yl)estra-1,3,5(10),16-tetraen-3-carboxamid
6. 17-(5-Cyanpyridin-3-yl)estra-1,3,5(10),16-tetraen-3-carboxamid
7. 11$\beta$-Fluor-17-(5-fluorpyridin-3-yl)estra-1,3,5(10),16-tetraen-3-carboxamid
8. 11$\beta$-Fluor-17-(5-fluorpyridin-3-yl)-*N*-(2-sulfamoylethyl)estra-1,3,5(10),16-tetraen-3-carboxamid
9. 17-(5-Fluorpyridin-3-yl)-*N*-[(1*S*,2*R*)-2-hydroxycyclopentyl]estra-1,3,5(10),16-tetraen-3-carboxamid
10. 17-(5-Fluorpyridin-3-yl)-*N*-[2-(hydroxymethyl)-2-methylpropyl]estra-1,3,5(10),16-tetraen-3-carboxamid
11. 17-(5-Fluorpyridin-3-yl)-*N*-[(1*S*,2*S*)-2-hydroxycyclopentyl]estra-1,3,5(10),16-tetraen-3-carboxamid
12. 17-(5-Fluorpyridin-3-yl)-*N*-[(*R*)-3-(hydroxymethyl)butyl]estra-1,3,5(10),16-tetraen-3-carboxamid
13. 17-(5-Fluorpyridin-3-yl)-*N*-[1-(hydroxymethyl)cyclopentyl]estra-1,3,5(10),16-tetraen-3-carboxamid
14. [17-(5-Fluorpyridin-3-yl)estra-1,3,5(10),16-tetraen-3-yl](4-hydroxypiperidin-1-yl)keton
15. 17-(5-Fluorpyridin-3-yl)-*N*-[(*S*)-1-(hydroxymethyl)propyl]estra-1,3,5(10),16-tetraen-3-carboxamid
16. 17-(5-Fluorpyridin-3-yl)-*N*-[(*R*)-1-(hydroxymethyl)-2-methylpropyl]estra-1,3,5(10),16-tetraen-3-carboxamid
17. [17-(5-Fluorpyridin-3-yl)estra-1,3,5(10),16-tetraen-3-yl][(*R*)-3-hydroxypiperidin-1-yl]keton
18. rel-17-(5-Fluorpyridin-3-yl)-*N*-[(1*R*,2*R*)-2-hydroxy-1-methylpropyl]estra-1,3,5(10),16-tetraen-3-carboxamid
19. 17-(5-Fluorpyridin-3-yl)-*N*-(2-hydroxyethyl)-*N*-isopropylestra-1,3,5(10),16-tetraen-3-carboxamid
20. 17-(5-Fluorpyridin-3-yl)-*N*-[(*RS*)-3,3,3-trifluor-2-hydroxypropyl]estra-1,3,5(10),16-tetraen-3-carboxamid
21. 17-(5-Fluorpyridin-3-yl)-*N*-[2-(1*H*-tetrazol-5-yl)ethyl]estra-1,3,5(10),16-tetraen-3-carboxamid
22. 17-(5-Fluorpyridin-3-yl)-*N*-(1*H*-tetrazol-5-ylmethyl)estra-1,3,5(10),16-tetraen-3-carboxamid

23. 17-(3-Pyridyl)-*N*-(2-sulfamoylethyl)estra-1,3,5(10),16-tetraen-3-carboxamid
24. *N*-(2-Sulfamoylethyl)-17-[5-(trifluormethyl)pyridin-3-yl]estra-1,3,5(10),16-tetraen-3-carboxamid
25. *N*-[2-(*N*-Methylsulfamoyl)ethyl]-17-[5-(trifluormethyl)pyridin-3-yl]estra-1,3,5(10),16-tetraen-3-carboxamid
26. *N*-(3-Amino-3-oxopropyl)-17-(5-fluorpyridin-3-yl)estra-1,3,5(10),16-tetraen-3-carboxamid
27. 17-(5-Fluorpyridin-3-yl)-*N*-[3-(methylamino)-3-oxopropyl]estra-1,3,5(10),16-tetraen-3-carboxamid
28. [17-(5-Fluorpyridin-3-yl)estra-1,3,5(10),16-tetraen-3-yl][(*S*)-2-(1*H*-tetrazol-5-yl)pyrrolidin-1-yl]keton
29. 1-{[17-(5-Fluorpyridin-3-yl)estra-1,3,5(10),16-tetraen-3-yl]carbonyl}-*N*-methyl-L-prolinamid
30. 1-{[17-(5-Fluorpyridin-3-yl)estra-1,3,5(10),16-tetraen-3-yl]carbonyl}-L-prolinamid
31. 17-(5-Fluorpyridin-3-yl)-*N*-{2-methyl-2-[(methylsulfonyl)amino]propyl}estra-1,3,5(10),16-tetraen-3-carboxamid
32. *N*-Ethyl-17-(5-fluorpyridin-3-yl)-*N*-(2-hydroxyethyl)estra-1,3,5(10),16-tetraen-3-carboxamid
33. *N*-[(*S*)-2,3-Dihydroxypropyl]-17-(5-fluorpyridin-3-yl)estra-1,3,5(10),16-tetraen-3-carboxamid
34. 17-(5-Fluorpyridin-3-yl)-*N*-(3-hydroxypropyl)-*N*-methylestra-1,3,5(10),16-tetraen-3-carboxamid
35. *N*-[(*RS*)-2,3-Dihydroxypropyl]-17-(5-fluorpyridin-3-yl)-*N*-methylestra-1,3,5(10),16-tetraen-3-carboxamid
36. *N*-[(*R*)-2,3-Dihydroxypropyl]-17-(5-fluorpyridin-3-yl)estra-1,3,5(10),16-tetraen-3-carboxamid
37. 17-(5-Fluorpyridin-3-yl)-*N*-[2-(methylsulfinyl)ethyl]estra-1,3,5(10),16-tetraen-3-carboxamid
38. 17-(5-Fluorpyridin-3-yl)-*N*-[(*R*)-2-hydroxypropyl]estra-1,3,5(10),16-tetraen-3-carboxamid
39. *N*-Ethyl-17-(5-fluorpyridin-3-yl)estra-1,3,5(10),16-tetraen-3-carboxamid
40. 17-(5-Fluorpyridin-3-yl)-*N*-[(*S*)-1-(hydroxymethyl)ethyl]estra-1,3,5(10),16-tetraen-3-carboxamid
41. 17-(5-Fluorpyridin-3-yl)-*N*-(2-methoxyethyl)estra-1,3,5(10),16-tetraen-3-carboxamid
42. 17-(5-Fluorpyridin-3-yl)-*N*-[2-(isopropylsulfonyl)ethyl]estra-1,3,5(10),16-tetraen-3-carboxamid
43. 17-(5-Fluorpyridin-3-yl)-*N*-[(3-methyloxetan-3-yl)methyl]estra-1,3,5(10),16-tetraen-3-carboxamid
44. 17-(5-Fluorpyridin-3-yl)-*N*-(2-hydroxyethyl)-*N*-methylestra-1,3,5(10),16-tetraen-3-carboxamid
45. 17-(5-Fluorpyridin-3-yl)-*N*-[(*S*)-2-hydroxypropyl]estra-1,3,5(10),16-tetraen-3-carboxamid
46. 17-(5-Fluorpyridin-3-yl)-*N*-[2-(2-hydroxyethoxy)ethyl]estra-1,3,5(10),16-tetraen-3-carboxamid
47. 17-(Pyrimidin-5-yl)-*N*-(2-sulfamoylethyl)estra-1,3,5(10),16-tetraen-3-carboxamid

und deren Tautomere, N-Oxide, Hydrate, Solvate oder Salze oder eine Mischung bestehend aus den Vorgenannten.

Bei den erfindungsgemäßen Verbindungen handelt es sich um Substanzen basierend auf einem Estra-1,3,5(10),16-tetraen-Gerüst substituiert mit einem aromatischen Heterocyclus an Position 17.

Die erfindungsgemäßen Verbindungen sind potente Inhibitoren von AKR1C3. Im Gegensatz zu Abirateron, ein klinisch eingesetzter, steroidaler Cyp17A1 (Lyase) Inhibitor, der einen Pyridinring aufweist, wird Cyp17A1 von den erfindungsgemäßen Verbindungen bis zu einer Substanzkonzentration von 20 μM nicht inhibiert. Die beanspruchten Verbindungen zeigen eine Inhibition von AKR1C3 *in vitro* ($IC_{50}$-Werte < 500 nM) und überwiegend sogar $IC_{50}$-Werte < 100 nM (siehe Tabelle 1 und 2).

Darüber hinaus werden die mit AKR1C3 verwandten Enzyme AKR1C1, AKR1C2 und AKR1C4 durch die erfindungsgemäßen Verbindungen nicht inhibiert.

Die hier beanspruchten, neuen AKR1C3 Inhibitoren zeigen im Vergleich zum bekannten AKR1C3 Inhibitor EM-1404 eine verbesserte wässrige Löslichkeit. Dadurch ist die Formulierbarkeit der erfindungsgemäßen Verbindungen in wässrigen Applikationsmedien verbessert.

Gegenstand der vorliegenden Erfindung sind Verbindungen der Formel (I) zur Behandlung und/oder Prophylaxe von Krankheiten.

Die erfindungsgemäßen Verbindungen zeigen unvorhersehbar ein wertvolles pharmakologisches und pharmakokinetisches Wirkspektrum. Sie eignen sich daher zur Verwendung als Arzneimittel zur Behandlung und/oder Prophylaxe von Krankheiten bei Menschen und Tieren. Der Begriff "Behandlung" im Rahmen der vorliegenden Erfindung schließt die Prophylaxe ein. Die pharmazeutische Wirksamkeit der erfindungsgemäßen Verbindungen lässt sich durch ihre Wirkung als AKR1C3 Inhibitor erklären. Wie die Tabellen 1 (Beispiel 48, Inhibition von AKR1C3 in einem biochemischen Assay) sowie 2 (Beispiel 49, Inhibition von AKR1C3 in einem zellbasierten System) zeigen, sind die erfindungsgemäßen Verbindungen potente Inhibitoren des AKR1C3 Enzyms, wodurch sie in der Lage sind, die lokale Östradiolproduktion in endometrischen Läsionen zu blockieren. Zur Behandlung der Endometriose ist eine nebenwirkungsarme Therapie insbesondere bevorzugt, weswegen eine Selektivität der AKR1C3 Inhibitoren gegenüber dem steroidmetabolisierenden Enzym CYP17A1 (Lyase) wichtig ist. Die Daten zu ausgewählten erfindungsgemäßen Verbindungen in Tabelle 3 (Beispiel 50, Inhibierung humaner CYP17) zeigen, dass bis zu einer sehr hohen Konzentration von 20 μM keine Inhibition der CYP17A1 aufgetreten ist, die Verbindungen somit selektiv gegenüber der CYP17A1 sind. Neben der Selektivität gegenüber CYP17A1 ist die Selektivität gegenüber anderen Enzymen der AKR1C Familie wichtig, um ein günstiges Nebenwirkungsprofil zu erhalten, da die Enzyme AKR1C1, -2 und -4 ebenfalls an der Steroidsynthese beteiligt sind. Eine Inhibierung dieser Enzyme würde den Vorteil der selektiven lokalen Inhibition der Östradiolsynthese wieder aufheben und könnte zu systemischen Veränderungen verschiedener Steroidhormone führen. Tabelle 4 (Beispiel 51, Inhibition von AKR1C1, -2 bzw. -4) zeigt für die erfindungsgemäße Verbindung 1, dass keine Inhibition dieser Enzyme

vorliegt. Phenolphthalein, das als Positivkontrolle verwendet wurde, zeigt hingegen eine Inhibition der Enzyme. Die erfindungsgemäßen Verbindungen sind daher besonders zur Behandlung und/oder Prophylaxe der Endometriose geeignet.

Desweiteren eignen sich die erfindungsgemäßen Verbindungen zur Behandlung und/oder Prophylaxe von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes geeignet.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: selektive Estrogen Rezeptor Modulatoren (SERMs), Estrogenrezeptor (ER) Antagonisten, Aromataseinhibitoren, 17β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen (Mkk3/6, Mek1/2, Erk1/2), Inhibitoren der Proteinkinasen B (PKBα/β/γ; Akt1/2/3), Inhibitoren der Phosphoinositid-3-Kinasen (PI3K), Inhibitoren der Cyclinabhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten, und Nicht-steroidale Enzündungshemmer (NSAIDs).

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Darüber hinaus können die erfindungsgemäßen Verbindungen auch in Kombination mit einer Strahlentherapie und/oder einer chirurgischen Intervention eingesetzt werden. Als geeignete Kombinationswirkstoffe seien beispielhaft genannt:

131I-chTNT, Abarelix, Abiraterone, Aclarubicin, Aldesleukin, Alemtuzumab, Alitretinoin, Altretamin, Aminoglutethimid, Amrubicin, Amsacrin, Anastrozol, Arglabin, Arsentrioxidas, Asparaginase, Azacitidin, Basiliximab, RDEA 119, Belotecan, Bendamustin, Bevacizumab, Bexarotene, Bicalutamid, Bisantrene, Bleomycin, Bortezomib, Buserelin, Busulfan, Cabazitaxel, Kalciumfolinat, Kalciumlevofolinat, Capecitabine, Carboplatin, Carmofur, Carmustin, Catumaxomab, Celecoxib, Celmoleukin, Cetuximab, Chlorambucil, Chlormadinon, Chlormethine, Cisplatin, Cladribin, Clodronsäure, Clofarabine, Crisantaspase, Cyclophosphamid, Cyproteron, Cytarabine, Dacarbazine, Dactinomycin, Darbepoetin alfa, Dasatinib, Daunorubicin, Decitabine, Degarelix, Denileukin diftitox, Denosumab, Deslorelin, Dibrospidiumchlorid, Docetaxel, Doxifluridin, Doxorubicin, Doxorubicin + Estron, Eculizumab, Edrecolomab, Elliptiniumazetat, Eltrombopag, Endostatin, Enocitabine, Epirubicin, Epitiostanol, Epoetin alfa, Epoetin beta, Eptaplatin, Eribulin, Erlotinib, Estradiol, Estramustine, Etoposide, Everolimus, Exemestan, Fadrozole, Filgrastim, Fludarabin, Fluorouracil, Flutamid, Formestane, Fotemustine, Fulvestrant, Galliumnitrat, Ganirelix, gefitinib, Gemcitabine, Gemtuzumab, Glutoxim, Goserelin, Histamin Dihydrochlorid, Histrelin, Hydroxycarbamid, I-125 Pellets, Ibandronicsaüre, Ibritumomab Tiuxetan, Idarubicin, Ifosfamid, Imatinib, Imiquimod, Improsulfan, Interferon alfa, Interferon beta, Interferon gamma, Ipilimumab, Irinotecan, Ixabepilon, Lanreotid, lapatinib, Lenalidomid, Lenograstim, Lentinan, Letrozol, Leuprorelin, Levamisol, Lisurid, Lobaplatin, Lomustine, Lonidamin, Masoprocol, Medroxyprogesterone, Megestrol, Melphalan, Mepitiostan, Mercaptopurin, Methotrexat, Methoxsalen, Methyl minolevulinat, Methyltestosteron, Mifamurtid, Miltefosin, Miriplatin, Mitobronitol, Mitoguazone, Mitolactol, Mitomycin, Mitotane, Mitoxantron, Nedaplatin, Nelarabine, Nilotinib, Nilutamid, Nimotuzumab, Nimustine, Nitracrine, Ofatumumab, Omeprazol, Oprelvekin, Oxaliplatin, p53 Gentherapie, Paclitaxel, Palifermin, Palladium-103 Pellets, Pamidronicsäure, Panitumumab, Pazopanib, Pegaspargase, PEG-Epoetin beta (methoxy PEG-Epoetin beta), Pegfilgrastim, Peginterferon alfa-2b, Pemetrexed, Pentazocin, Pentostatin, Peplomycin, Perfosfamid, Picibanil, Pirarubicin, Plerixafor, Plicamycin, Poliglusam, Polyestradiolphosphat, Polysaccharide-K, Porfimer Sodium, Pralatrexat, Prednimustine, Procarbazine, Quinagolid, Radium-223 Chlorid, Raloxifen, Raltitrexed, Ranimustine, Razoxane, Regorafenib, Risedronicsäure, Rituximab, Romidepsin, Romiplostim, Sargramostim, Sipuleucel-T, Sizofiran, Sobuzoxan, Sodium Glycididazol, Sorafenib, Streptozocin, Sunitinib, Talaporfin, Tamibaroten, Tamoxifen, Tasonermin, Teceleukin, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Tioguanin, Tocilizumab, Topotecan, Toremifen, Tositumomab, Trabectedin, Trastuzumab,

Treosulfan, Tretinoin, Trilostan, Triptorelin, Trofosfamid, Tryptophan, Ubenimex, Valrubicin, Vandetanib, Vapreotid, Vemurafenib, Vinblastin, Vincristin, Vindesin, Vinflunin, Vinorelbin, Vorinostat, Vorozol, Yttrium-90 Mikroglaskugeln, Zinostatin, Zinostatin Stimalamer, Zoledronicsäure, Zorubicin.

Vorzugsweise betrifft die vorliegende Erfindung Arzneimittel, enthaltend mindestens eine erfindungsgemäße Verbindung und einen oder mehrere der folgenden Wirkstoffe, insbesondere zur Behandlung und/oder Prophylaxe Androgenrezeptor-abhängiger proliferativer Erkrankungen:

LHRH (luteinizing hormone-releasing hormone) Agonisten,

LHRH (luteinizing hormone-releasing hormone) Antagonisten,

C(17,20)-Lyase-Inhibitoren,

5-$\alpha$-Reduktase-Inhibitoren Typ I,

5-$\alpha$-Reduktase-Inhibitoren Typ II,

gemischte 5-$\alpha$-Reduktase-Inhibitoren Typ I/II,

$\alpha$-Strahlung emittierende Radiopharmazeutika zur Behandlung von Knochenmetastasen, wie z.B. Radium-223 Chlorid,

Zytostatika,

VEGF (Vascular Endothelial Growth Factor) -Kinase-Inhibitoren,

Antigestagene,

Antiestrogene,

EGF-Antikörper,

Estrogene oder

andere Androgenrezeptor Antagonisten,

Poly (ADP-ribose) Polymerase I Inhibitoren, oder

Bi-specific T-cell engagers (BiTE) gekoppelt an einem Zelloberflächenprotein wie zum Beispiel prostate-specific membrane antigen (PSMA).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z. B. intravenös, intraarteriell, intrakardial, intraspinal oder intralumbal) oder unter Einschaltung einer Resorption (z. B. intramuskulär, subcutan, intracutan, percutan oder intraperitoneal). Für die parenterale Applikation eignen sich als Applikationsformen u. a. Injektions- und Infusionszubereitungen in Form von Lösungen, Suspensionen, Emulsionen, Lyophilisaten oder sterilen Pulvern.

Für die sonstigen Applikationswege eignen sich z. B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate, intrauterine Spiralen, Vaginalringe oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u. a. Trägerstoffe (beispielsweise mikrokristalline Cellulose, Laktose, Mannitol), Lösungsmittel (z. B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht je Tag betragen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Weiterer Gegenstand der vorliegenden Erfindung sind Arzneimittel zur Behandlung und Prophylaxe von Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

Weiterer Gegenstand der vorliegenden Erfindung sind die Verbindungen der allgemeinen Formel (I), zur Verwendung in Form eines pharmazeutischen Präparates für die enterale, parenterale, vaginale, intrauterine und orale Applikation.

Eine Teilmenge der erfindungsgemäßen Verbindungen lässt sich ausgehend vom literaturbekannten Methyl-17-oxoestra-1,3,5(10)-trien-3-carboxylat (Steroids, 1995, 60, 3, 299 - 306) herstellen (Syntheseschema 1):

Die Umsetzung zum Intermediat 1 erfolgt durch die Verwendung von Trifluormethansulfonsäureanhydrid oder *N,N*-Bis[(trifluormethyl)sulfonyl]anilin in Gegenwart einer Base wie Pyridin, 2,6-Dimethylpyridin oder 2,6-Di-*tert*-butylpyridin oder in Gegenwart eines tertiären Amins wie Triethylamin oder Diisopropylethylamin oder durch Verwendung von Alkalimetallhexamethylsilazanen oder Lithium-diisopropylamid (LDA) (J. Med. Chem., 1995, 38, 2463 - 2471, J. Org. Chem., 1985 , 50, 1990 - 1992, JACS, 2009 , 131, 9014 - 9019, Archiv der Pharmazie (Weinheim, Germany), 2001, 334, 12, 373 - 374). Bevorzugt ist die Umsetzung mit Trifluormethansulfonsäureanhydrid in Gegenwart von 2,6-Di-*tert*-butylpyridin in Dichlormethan.

Die Herstellung der Intermediate 2 erfolgt mit Hilfe der dem Fachmann bekannten *Suzuki*-Reaktion. Hierfür wird das Intermediat 1 mit einer stickstoffhaltigen aromatischen Boronsäure, einem Boronsäureester wie z. B. einem Boronsäurepinakolester, einem *MIDA* Boronat (D. M. Knapp et al. J. Am. Chem. Soc. 2009, 131, 6961) oder mit einem Trifluorboratsalz (G. A. Molander et al., J. Org. Chem. 2009, 74, 973) umgesetzt. Als Katalysatoren kommen eine Vielzahl

an palladiumhaltigen Katalysatoren in Betracht, wie zum Beispiel Tetrakis(triphenylphosphin)palladium(0), Bis(triphenylphosphin)palladium(II)-dichlorid oder [1,3-Bis(2,6-diisopropylphenyl)imidazol-2-yliden](3-chlorpyridyl)-palladium(II) dichlorid (CAS 905459-27-0). Alternativ kann eine Palladiumhaltige Quelle wie zum Beispiel Palladium(II)-acetat, Palladium(II)-chlorid oder $Pd(dba)_2$ in Kombination mit einem Phosphorhaltigen Liganden wie zum Beispiel Triphenylphosphin, SPhos (D. M. Knapp et. al., J. Am. Chem. Soc. 2009, 131, 6961) oder RuPhos (G. A. Molander, J. Org. Chem. 2009, 74, 973) eingesetzt werden. Bevorzugt ist die Umsetzung mit Boronsäuren in Gegenwart von Tetrakis(triphenylphosphin)palladium(0) oder [1,3-Bis(2,6-diisopropylphenyl)imidazol-2-yliden](3-chlorpyridyl)palladium(II) dichlorid.
Die Herstellung der Intermediate 3 erfolgt durch Verseifung des Methylesters nach dem Fachmann bekannten Methoden. Hierfür wird das Intermediat 2 in einem Lösemittel wie Tetrahydrofuran (THF), Methanol oder Dimethylsulfoxid (DMSO) oder in einer Mischung aus Methanol und THF mit Natronlauge oder einer wässrigen Lithiumhydroxidlösung versetzt. Gegebenenfalls wird erwärmt. Bevorzugt ist die Umsetzung in THF und Methanol in Gegenwart von Natronlauge oder wässriger Lithiumhydroxidlösung bei 40°C.

Die Herstellung der Beispielverbindungen erfolgt ausgehend von Intermediaten 3 durch eine Amidkupplung mit einem Amin. Für die Amidkupplung (Stufe A) kommen dem Fachmann bekannte Reagenzien wie zum Beispiel *N*,*N'*-Dicyclohexylcarbodiimid (DCC), *N*-[3-(Dimethylamino)propyl]-*N*'-ethylcarbodiimid Hydrochlorid (EDC) [CAS 25952-53-8] oder HATU (O-(7-Azabenzotriazol-1-yl)-*N*,*N*,*N'*,N'-tetramethyluronium hexafluorophosphonat in Frage. Zusätzlich können als Additive noch Reagenzien wie 1*H*-Benzotriazol-1-ol Hydrat (HOBt Hydrat [CAS 123333-53-9]) oder *N*,*N*-Dimethylpyridin-4-amin (DMAP) benutzt werden. Als Basen können zum Beispiel Pyridin, Triethylamin oder Diisopropylethylamin verwendet werden. Bevorzugt ist die Umsetzung mittels EDC, HOBt Hydrat und Triethylamin. Für die Umwandlung des Carbonsäureesters in die Carbonsäure (Stufe B) können - falls es sich zum Beispiel um einen Methyl, Ethyl oder Benzylester handelt - Verseifungsmethoden verwendet werden, wie bei der Herstellung der Intermediate 3 beschrieben. Handelt es sich um einen Carbonsäure-*tert*-butylester, so kann dieser nach dem Fachmann bekannten Methoden zur Carbonsäure umgewandelt werden wie zum Beispiel durch die Reaktion mit Trifluoressigsäure in Dichlormethan oder Chloroform oder durch die Reaktion mit Chlorwasserstoff in 1,4-Dioxan. Die Reaktion mit Trifluoressigsäure in Dichlormethan ist bevorzugt.

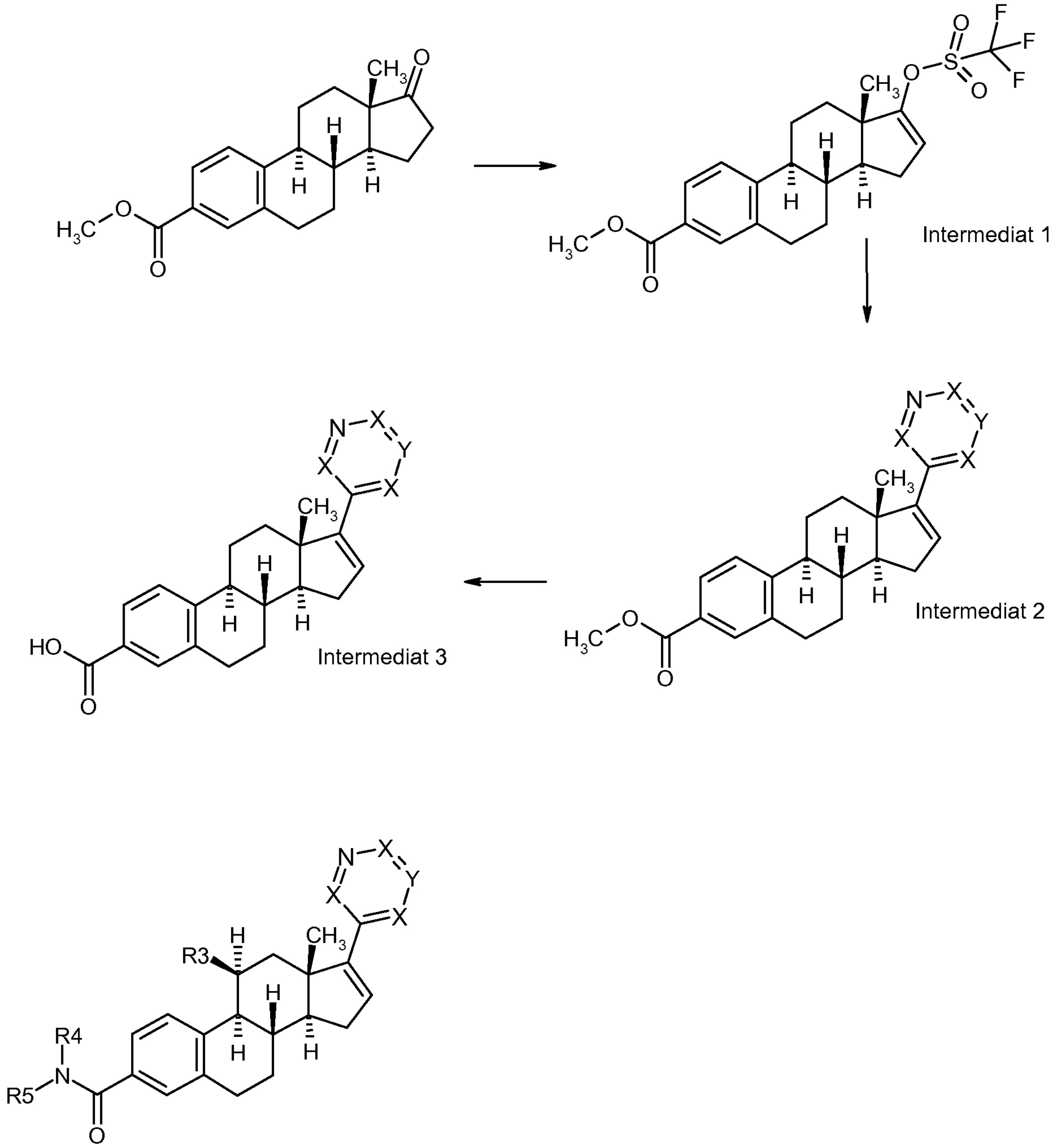


**Syntheseschema 1 (Herstellung einer Teilmenge der Beispielverbindungen mit R3 = H)**

Eine Teilmenge der erfindungsgemäßen Verbindungen der Formel (I) mit R5 = F und R6 = H lässt sich wie in Syntheseschema 2 beschrieben herstellen:

3,11$\alpha$-Dihydroxyestra-1,3,5(10)-trien-17-on (CAS 5210-15-1) wird mit Essigsäureanhydrid und Pyridin in Gegenwart von 4-Dimethylaminopyridin (DMAP) in Dichlormethan zu Intermediat 4 umgesetzt. Die Umwandlung in das Intermediat 5 erfolgt mit Natriumhydrogencarbonat in Methanol. Die Umsetzung von Intermediat 5 mit 1,1,2,2,3,3,4,4,4-Nonafluorbutan-1-sulfonylfluorid und Kaliumcarbonat führt zu Intermediat 6, welches mit Palladium(II)acetat, 1,3-Bis(diphenylphosphino)propan, Triethylamin in Methanol und DMSO in einem Autoklaven in einer Kohlenmonoxidatmosphäre zu Intermediat 7 umgewandelt wird. Die Umsetzung zu Intermediat 8 erfolgt mit Methoden wie bei der Herstellung von Intermediat 1 beschrieben. Die Transformation von Intermediat 8 zu Intermediat 9 wird mit Methoden, wie bei der Herstellung von Intermediat 2 beschrieben, durchgeführt. Intermediat 9 wird mit Kaliumcarbonat in Methanol zu Intermediat 10 verseift. Die Transformation zum Intermediat 11 wird mit 1,8-Diazabicyclo[5.4.0]undec-7-en und 1,1,2,2,3,3,4,4,4-Nonafluorbutan-1-sulfonylfluorid in THF durchgeführt. Die Verseifung von Intermediat 11 zu Intermediat 12 erfolgt mit Bedingungen wie bei der Herstellung von Intermediat 3 beschrieben. Bevorzugt ist die Umsetzung in THF und Methanol in Gegenwart von wässriger Lithiumhydroxidlösung. Die Herstellung einer Teilmenge der Beispielverbindungen ausgehend von Intermediat 12 erfolgt analog zur Herstellung der Beispielverbindungen ausgehend von Intermediat 3 wie in Syntheseschema 1 beschrieben.

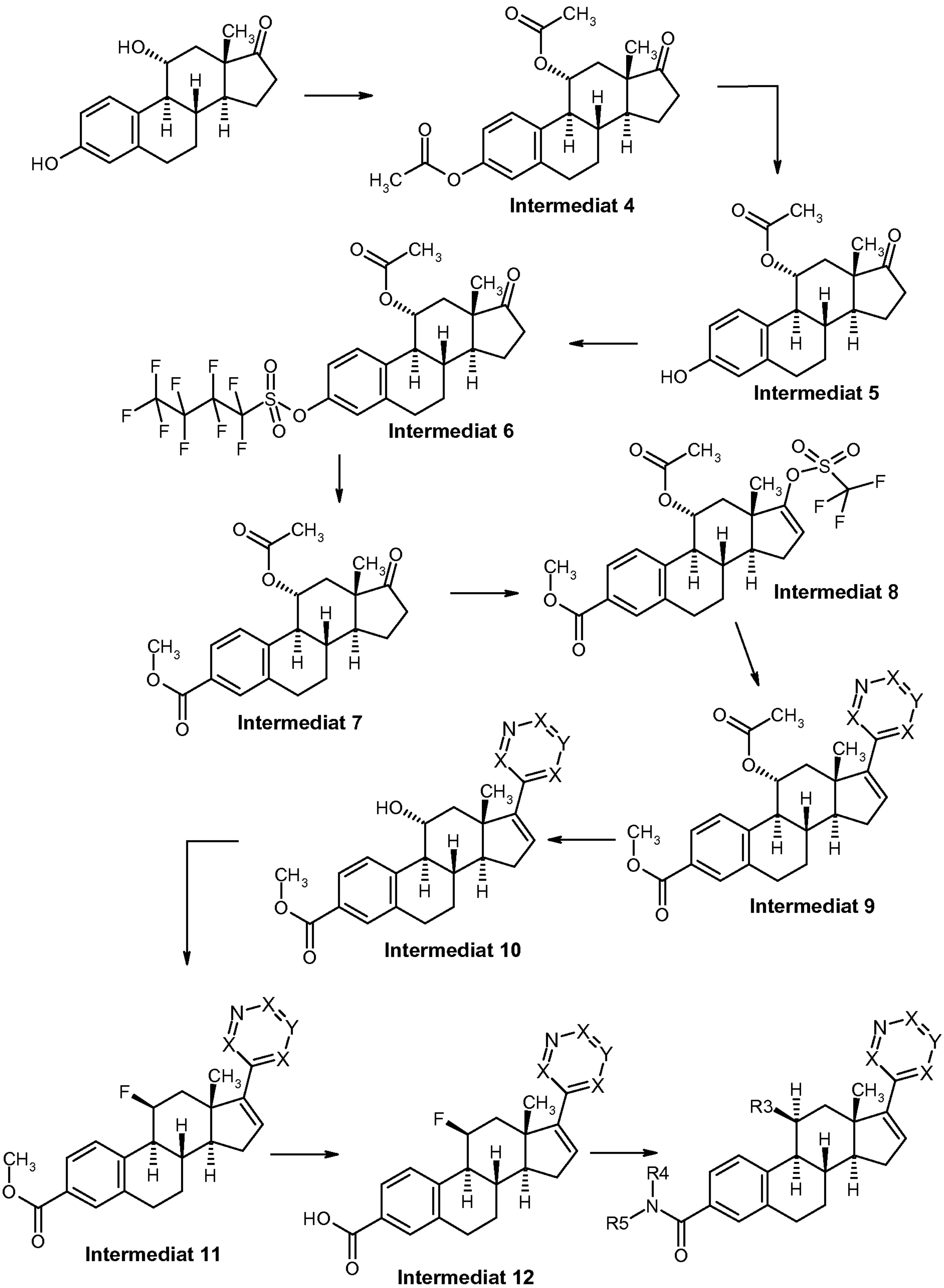

Intermediat 4

Intermediat 5

Intermediat 6

Intermediat 7

Intermediat 8

Intermediat 9

Intermediat 10

Intermediat 11

Intermediat 12

**Syntheseschema 2 (Herstellung einer Teilmenge der Beispielverbindungen mit R3 = F)**

Eine weitere Teilmenge der erfindungsgemäßen Verbindungen der Formel (I) lässt sich wie in Syntheseschema

3 verdeutlicht herstellen. Ausgehend von 17-Oxoestra-1,3,5(10)-trien-3-carboxamid (E. Morera, G. Ortar, THL, 1998, 39, 2835 - 2838) erhält man Intermediat 13 durch Umsetzung mit Hydrazinhydrat in Gegenwart von Hydrazinsulfat. Aus Intermediat 13 erhält man Intermediat 14 durch Umsetzung mit Iod in Dioxan in Gegenwart von Triethylamin. Intermediat 14 lässt sich dann mit Hilfe der Suzuki-Reaktion wie bei Syntheseschema 1 beschrieben zu den Titelverbindungen umsetzen.

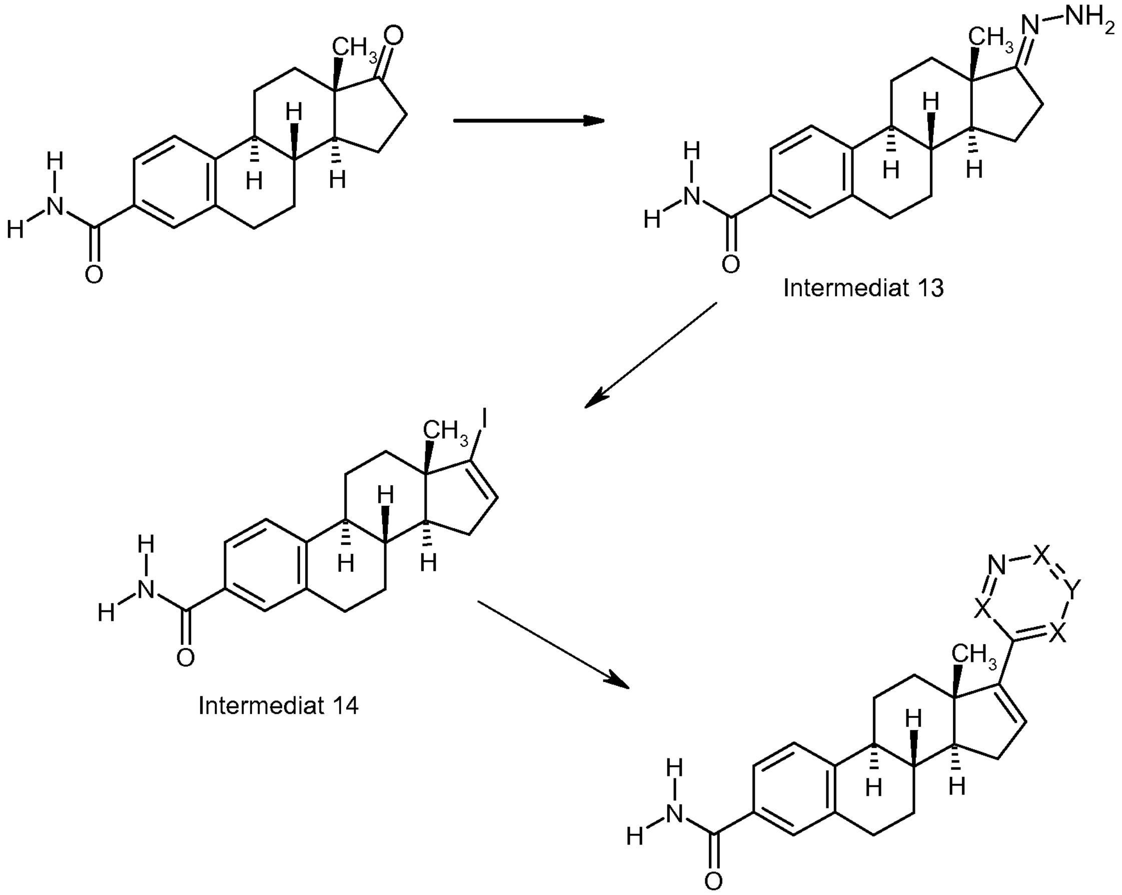


**Syntheseschema 3 (Herstellung einer Teilmenge der Beispielverbindungen der Formel (I))**

Eine weitere Teilmenge der erfindungsgemäßen Verbindungen der Formel (I) lässt sich wie in Syntheseschema 4 verdeutlicht herstellen. Ausgehend von 17-Oxoestra-1,3,5(10)-trien-3-carbonitril (Journal of the Chemical Society, 1964, 5889) erhält man Intermediat 15 wie bei der Herstellung von Intermediat 1 beschrieben. Intermediat 16 lässt sich dann mit Hilfe der Suzuki-Reaktion wie bei Syntheseschema 1 beschrieben herstellen. Ausgehend von Intermediat 16 erhält man eine Teilmenge der erfindungsgemäßen Verbindungen durch Umsetzung mit Natriumperborat Tetrahydrat in einem alkoholischen Lösemittel wie Methanol oder Ethanol (A. McKillop, D. Kemp, Tetrahedron, 1989, 45, 11, 3299 - 3306. Die Umsetzung in Ethanol in der Mikrowelle ist bevorzugt.

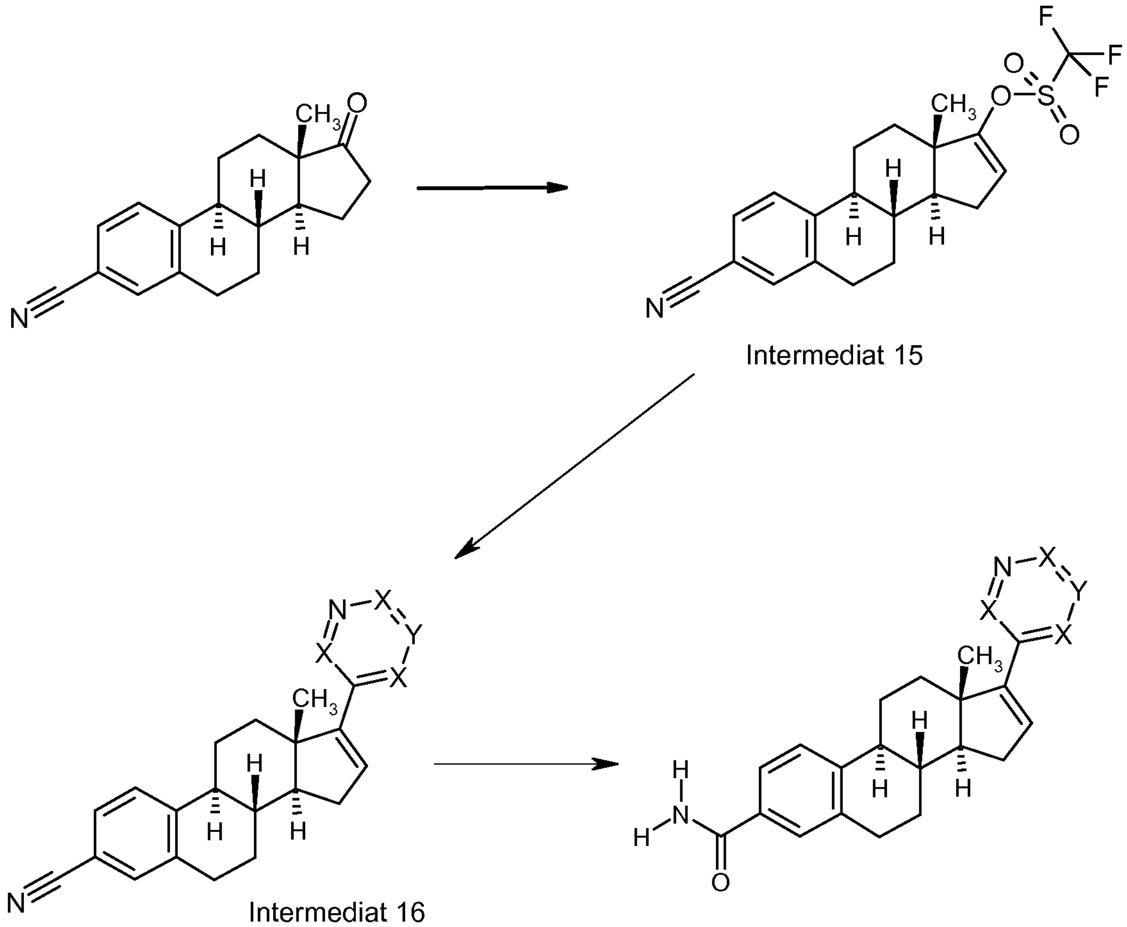

Intermediat 15

Intermediat 16

**Syntheseschema 4 (Herstellung einer Teilmenge der Beispielverbindungen der Formel (I))**

## Abkürzungsverzeichnis Chemie

Abkürzungen und Akronyme:

| | |
|---|---|
| DMF | *N,N*-Dimethylformamid |
| DMSO | Dimethylsulfoxid |
| NMP | 1-Methylpyrrolidin-2-on |
| THF | Tetrahydrofuran |
| d. Th. | der Theorie (bei Ausbeute) |
| h | Stunde(n) |
| HPLC | Hochdruck-, Hochleistungsflüssigkeitschromatographie |
| LC-MS | Flüssigkeitschromatographie-gekoppelte Massenspektroskopie |
| ES-MS | Elektrospray Massenspektrometrie |
| min | Minute(n) |
| MS | Massenspektrometrie |
| NMR | Kernresonanzspektroskopie |
| Rt | Retentionszeit |

(fortgesetzt)

| | |
|---|---|
| TFA | Trifluoressigsäure |
| Raumtemp. | Raumtemperatur |

## Reinigung der erfindungsgemäßen Verbindungen

In einigen Fällen wurden die erfindungsgemäßen Verbindungen durch präparative HPLC mit Hilfe eines Autopurifier-Gerätes der Firma Waters (Detektion der Verbindungen durch UV-Detektion sowie Elektrospray-Ionisation) in Kombination mit kommerziell erhältlichen, vorgepackten HPLC Säulen (zum Beispiel Säule XBridge (Firma Waters), C18, 5μm, 30 x 100mm) gereinigt. Als Lösemittelsystem wurde Acetonitril/Wasser mit Zusatz von Ameisensäure verwendet.

In einigen Fällen wurde folgende Methode für die präparative HPLC Trennung verwendet:

| | |
|---|---|
| *System:* | Waters Autopurificationsystem: Pump 2545, Sample Manager 2767, CFO, DAD 2996, ELSD 2424, SQD |
| *Säule:* | XBrigde C18 5μm 100x30 mm |
| *Lösemittel:* | A = $H_2O$ + 0.1% Vol. Ameisensäure (99%) |
| | B = Acetonitril |
| *Gradient:* | 0-1 min 1% B, 1-8 min 1-99% B, 8-10 min 99% B |
| *Fluss:* | 50 ml/min |
| *Temperatur:* | Raumtemp. |
| *Injektion:* | 1 x 2.5 ml |
| *Detektion:* | DAD scan range 210-400 nm |
| | MS ESI+, ESI-, scan range 160-1000 m/z |

Zum Entfernen des HPLC-Lösemittelgemisches wurde eine Gefriertrocknung oder eine Vakuumzentrifugation verwendet.

In einigen Fällen wurden die erfindungsgemäßen Verbindungen durch Chromatographie an Kieselgel gereinigt. Hierbei wurden vorgepackte Silica Gel Cartridges (zum Beispiel von der Firma Separtis, *Isolute® Flash silica gel*) in Kombination mit dem Flashmaster II Chromatographiegerät (Argonaut/Biotage) und Chromatographielösemittel bzw. -gemische wie zum Beispiel Hexan, Ethylacetat sowie Dichlormethan und Methanol verwendet.

## Strukturanalytik der erfindungsgemäßen Verbindungen:

In einigen Fällen wurden die erfindungsgemäßen Verbindungen durch LC-MS analysiert.

In einigen Fällen wurde folgende Analytikmethode (Methode 1) verwandt:
Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 μl; DAD scan: 210-400 nm Bei den NMR-Daten der erfindungsgemäßen Verbindungen gelten folgende Bedeutungen:

| | |
|---|---|
| s | Singulett |
| d | Dublett |
| t | Triplett |
| q | Quartett |
| quin | Quintett |
| m | Multiplett |
| br | breit |

(fortgesetzt)

| | |
|---|---|
| mc | Zentriertes Multiplett |

**Synthese der erfindungsgemäßen Verbindungen:**

**Intermediat 1**

**Methyl-17-{[(trifluormethyl)sulfonyl]oxy}estra-1,3,5(10),16-tetraen-3-carboxylat**

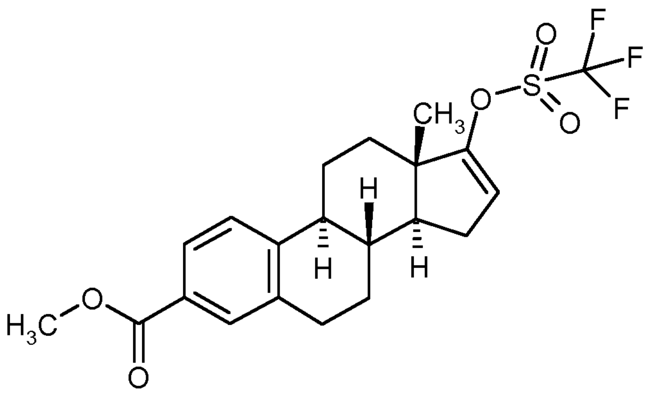

Zu einer Mischung aus 5.00 g (16.0 mmol) Methyl-17-oxoestra-1,3,5(10)-trien-3-carboxylat (Steroids, 1995, 60, 3, 299 - 306) in 100 ml Dichlormethan und 5.3 ml 2,6-Di-*tert*-butylpyridin wurden 3.2 ml Trifluormethansulfonsäureanhydrid getropft und 20 h bei Raumtemp. gerührt. Danach wurde die Mischung vorsichtig auf 250 ml gesättigte wässrige Natriumhydrogencarbonatlösung gegossen, 40 min gerührt und die Phasen getrennt, gefolgt von einer zweimaligen Extraktion mit Dichlormethan. Anschließend wurden die vereinigten organischen Phasen mit gesättigter Natriumhydrogencarbonatlösung und Natriumchloridlösung gewaschen, über Natriumsulfat getrocknet und eingeengt. Nach Ausrühren mit Hexan betrug die Ausbeute 4.55 g der Titelverbindung als Feststoff.
$^{1}$H-NMR (300MHz, CHLOROFORM-d): $\delta$ [ppm]= 1.01 (s, 3H), 1.37 - 1.74 (m, 5H), 1.81 (td, 1H), 1.88 - 2.02 (m, 2H), 2.05 - 2.19 (m, 1H), 2.27 - 2.55 (m, 3H), 2.83 - 3.11 (m, 2H), 3.90 (s, 3H), 5.63 (dd, 1H), 7.32 (d, 1H), 7.68 - 7.90 (m, 2H).

**Intermediat 2-a**

**Methyl-17-(5-fluorpyridin-3-yl)estra-1,3,5(10),16-tetraen-3-carboxylat**

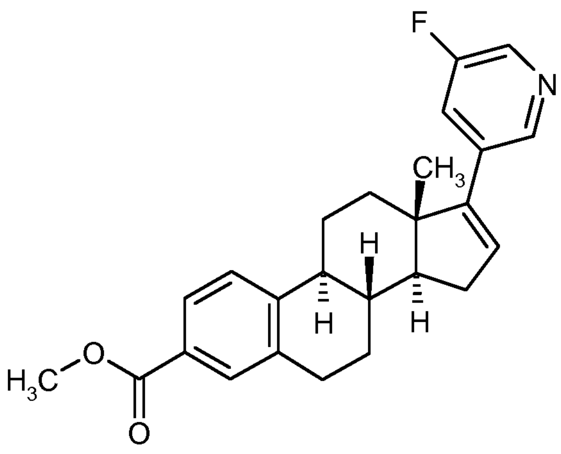

8.00 g (2.25 mmol) Methyl-17-{[(trifluormethyl)sulfonyl]oxy}estra-1,3,5(10),16-tetraen-3-carboxylat und 3.55 g (1.4 Äquiv.) 5-Fluorpyridin-3-boronsäure wurden in 60 ml Toluol und 40 ml Ethanol vorgelegt. Dann wurden 1.53 g (2.0 Äquiv.) Lithiumchlorid, 24 ml 2M wässrige Natriumcarbonatlösung und 1.04 g (5 mol%) Tetrakis(triphenylphosphin)palladium(0) zugegeben und 3.5 h auf 100°C erwärmt. Danach wurde Wasser addiert, dreimal mit Ethylacetat extrahiert, mit gesättigter Natriumhydrogencarbonatlösung, Kochsalzlösung gewaschen und eingeengt. Nach säulenchromatographischer Reinigung an Kieselgel (Hexan/Ethylacetat) betrug die Ausbeute 5.5 g (78% d. Th.) der Titelverbindung.
$^1$H-NMR (400MHz, CHLOROFORM-d): $\delta$ [ppm]= 1.06 (s, 3H), 1.47 - 1.63 (m, 1H), 1.63 - 1.78 (m, 3H), 1.84 (td, 1H), 1.98 - 2.06 (m, 1H), 2.13 - 2.26 (m, 2H), 2.35 - 2.51 (m, 3H), 2.98 (dd, 2H), 3.90 (s, 3H), 6.10 (dd, 1H), 7.32 - 7.44 (m, 2H), 7.76 - 7.86 (m, 2H), 8.36 (br. s., 1H), 8.48 (s, 1H).

**Intermediat 2-b**

**Methyl-17-(5-methoxypyridin-3-yl)estra-1,3,5(10),16-tetraen-3-carboxylat**

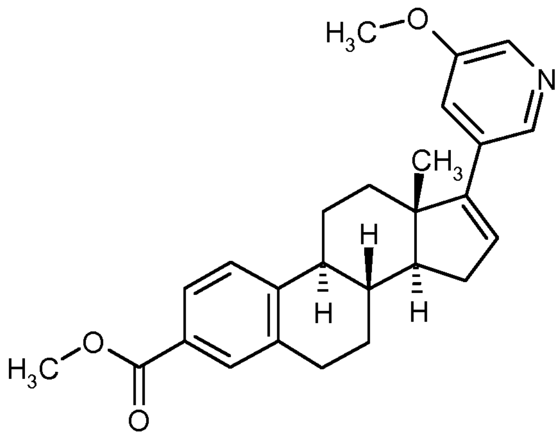

Analog zur Herstellung von Intermediat 2-a wurden 2.00 g (4.50 mmol) Intermediat 1 mit 0.96 g (1.4 Äquiv.) (5-Methoxypyridin-3-yl)boronsäure in Gegenwart von 260 mg Tetrakis(triphenylphosphin)palladium(0) bei 100°C über Nacht zu 1.4 g (76% d. Th.) der Titelverbindung umgesetzt.
$^1$H-NMR (300MHz, CHLOROFORM-d): $\delta$ [ppm]= 1.05 (s, 3H), 1.43 - 1.60 (m, 1H), 1.62 - 1.89 (m, 4H), 1.95 - 2.08 (m, 1H), 2.10 - 2.25 (m, 2H), 2.30 - 2.53 (m, 3H), 2.98 (dd, 2H), 3.88 (s, 3H), 3.90 (s, 3H), 6.00 - 6.08 (m, 1H), 7.16 - 7.22 (m, 1H), 7.35 (d, 1H), 7.75 - 7.83 (m, 2H), 8.20 (d, 1H), 8.28 (d, 1H).

**Intermediat 2-c**

**Methyl-17-(pyrimidin-5-yl)estra-1,3,5(10),16-tetraen-3-carboxylat**

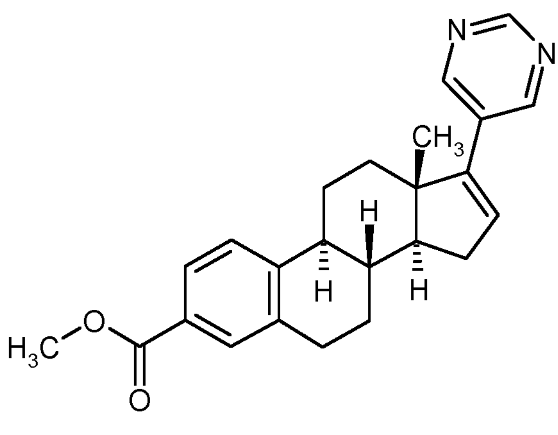

Analog zur Herstellung von Intermediat 2-a wurden 3.00 g (6.75 mmol) Intermediat 1 mit 1.17 g (1.4 Äquiv.) Pyrimidin-5-ylboronsäure in Gegenwart von 390 mg Tetrakis(triphenylphosphin)palladium(0) bei 100°C über Nacht zu 1.70 g (64% d. Th.) der Titelverbindung umgesetzt.
$^1$H-NMR (400MHz, CHLOROFORM-d): δ [ppm]= 1.06 (s, 3H), 1.47 - 1.59 (m, 1H), 1.65 - 1.80 (m, 3H), 1.85 (td, 1H), 1.98 - 2.06 (m, 1H), 2.12 - 2.25 (m, 2H), 2.36 - 2.53 (m, 3H), 2.98 (dd, 2H), 3.90 (s, 3H), 6.14 (dd, 1H), 7.35 (d, 1H), 7.76 - 7.85 (m, 2H), 8.76 (s, 2H), 9.09 (s, 1H).

**Intermediat 2-d**

**Methyl-17-[5-(trifluormethyl)pyridin-3-yl]estra-1,3,5(10),16-tetraen-3-carboxylat**

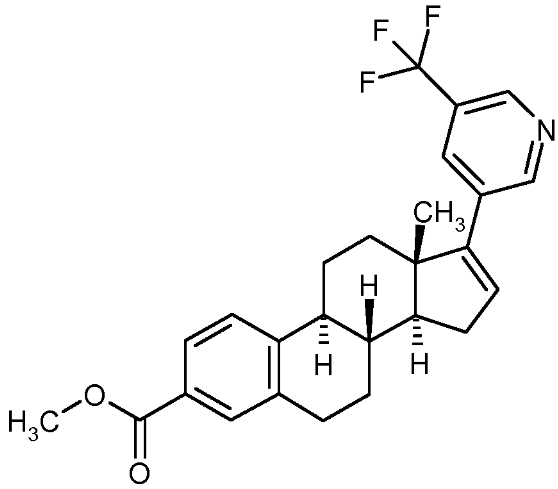

Analog zur Herstellung von Intermediat 2-a wurden 1.66 g (3.74 mmol) Intermediat 1 mit 1.00 g (1.4 Äquiv.) [5-(Trifluormethyl)pyridin-3-yl]boronsäure in Gegenwart von 216 mg Tetrakis(triphenylphosphin)palladium(0) bei 100°C über Nacht zu 1.20 g (73% d. Th.) der Titelverbindung umgesetzt.
$^1$H-NMR (300MHz, CHLOROFORM-d): δ [ppm]= 1.08 (s, 3H), 1.49 - 1.89 (m, 6H), 1.97 - 2.09 (m, 1H), 2.09 - 2.28 (m, 2H), 2.35 - 2.54 (m, 3H), 2.98 (dd, 2H), 3.90 (s, 3H), 6.15 (dd, 1H), 7.36 (s, 1H), 7.77 - 7.85 (m, 2H), 7.88 (s, 1H), 8.83 (s, 2H).

**Intermediat 2-e**

**Methyl-17-(5-cyanpyridin-3-yl)estra-1,3,5(10),16-tetraen-3-carboxylat**

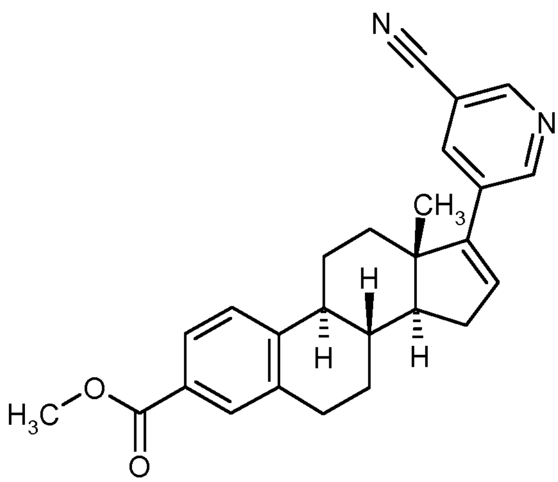

Analog zur Herstellung von Intermediat 2-a wurden 650 mg (1.46 mmol) Intermediat 1 mit 216 mg (1.0 Äquiv.) (5-Cyanpyridin-3-yl)boronsäure in Gegenwart von 84 mg Tetrakis(triphenylphosphin)palladium(0) bei 120°C in der Mikrowelle (100 Watt) innerhalb von 90 min umgesetzt. Nach säulenchromatographischer Reinigung an Kieselgel (Hexan/Ethylacetat) betrug die Ausbeute 109 mg Rohprodukt. $C_{26}H_{26}N_2O_2$ MS (ESIpos) gefundene Masse: 398.00. $^1$H-NMR (300MHz, DMSO-$d_6$): δ [ppm]= 1.00 (s, 3H), 2.82 - 2.96 (m, 2H), 3.79 (s, 3H), 6.31 - 6.36 (m, 1H), 7.40 (d, 1H), 7.61 - 7.71 (m, 2H), 8.27 (t, 1H), 8.85 - 8.89 (m, 2H).

**Intermediat 2-f**

**Methyl-17-(3-pyridyl)estra-1,3,5(10),16-tetraen-3-carboxylat**

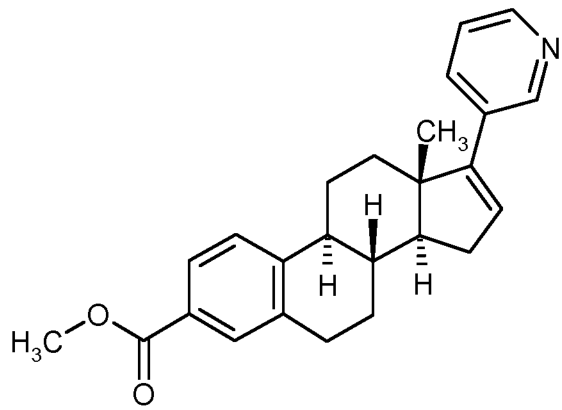

Analog zur Herstellung von Intermediat 2-a wurden 500 mg (1.13 mmol) Intermediat 1 mit 194 mg (1.4 Äquiv.) (5-Cyanpyridin-3-yl)boronsäure in Gegenwart von 39 mg Bis(triphenylphosphin)palladium(II)chlorid bei 100°C innerhalb von 18 h umgesetzt. Die Reaktionsmischung wurde dreimal mit Ethylacetat extrahiert und die vereinigten organischen Phasen eingeengt. Die Ausbeute betrug 462 mg eines Rohproduktes. $C_{25}H_{27}NO_2$ MS (ESIpos) gefundene Masse: 373.00

**Intermediat 3-a**

**17-(5-Fluorpyridin-3-yl)estra-1,3,5(10),16-tetraen-3-carbonsäure**

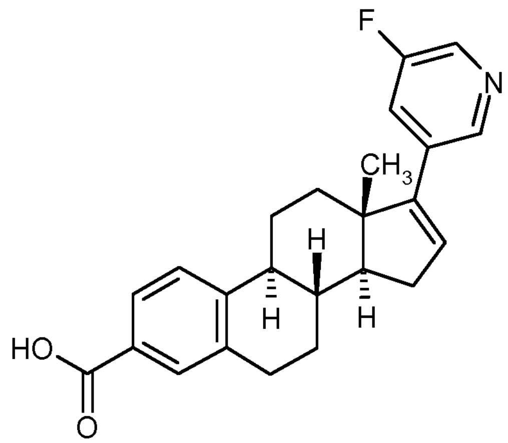

372 mg (0.95 mmol) Intermediat 2-a wurden in 50 ml THF und 3 ml Methanol vorgelegt. Anschließend wurde eine Lösung aus 120 mg Lithiumhydroxid in 3 ml Wasser addiert und 18 h bei Raumtemp. gerührt. Anschließend wurden erneut 5 Äquiv. Lithiumhydroxid addiert, 24 h bei Raumtemp. gerührt und 18 h 40°C gerührt. Danach wurde mit Wasser verdünnt, mit 10%iger wässriger Zitronensäurelösung auf pH 4 angesäuert, Ethylacetat wurde zugesetzt, Feststoff abfiltriert. Nach Waschen des Feststoffes mit Ethylacetat und Wasser und Trocknen betrug die Ausbeute 153 mg (43%

d. Th.) der Titelverbindung. Die organische Phase des Filtrats wurde abgetrennt und die wässrige Phase zweimal mit Ethylacetat extrahiert. Nach Waschen der vereinigten organischen Phasen mit Natriumchloridlösung, Trocknen über Natriumsulfat und Einengen wurde ein einen Rückstand erhalten, der aus Diethylether ausgerührt wurde. Nach Trocknen wurden weitere 143 mg (40% d. Th.) der Titelverbindung erhalten.
$^1$H-NMR (300MHz, DMSO-$d_6$): δ [ppm]= 0.99 (s, 3H), 1.38 - 1.78 (m, 5H), 1.83 - 1.97 (m, 1H), 2.05 - 2.21 (m, 2H), 2.25 - 2.43 (m, 3H), 2.89 (dd, 2H), 6.27 (dd, 1H), 7.36 (d, 1H), 7.58 - 7.72 (m, 3H), 8.43 (d, 1H), 8.49 (t, 1H).

**Intermediat 3-b**

**17-(5-Methoxypyridin-3-yl)estra-1,3,5(10),16-tetraen-3-carbonsäure**

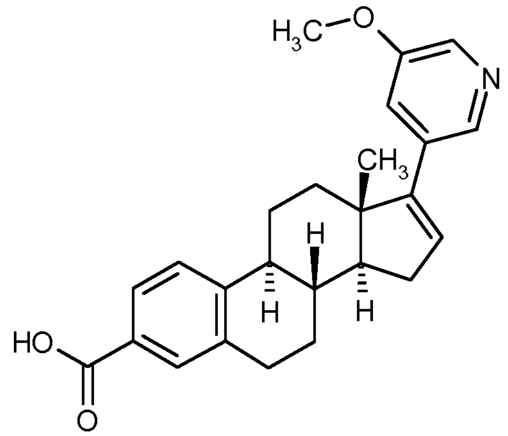

Eine Lösung aus 1.4 g (3.47 mmol) Methyl-17-(5-methoxypyridin-3-yl)estra-1,3,5(10),16-tetraen-3-carboxylat in 30 ml THF, 4 ml Methanol und 8.7 ml 2M Natronlauge wurde über Nacht bei Raumtemp. gerührt und dann für 8.5 h auf 40°C erwärmt. Anschließend wurde mit Wasser verdünnt, angesäuert mit 10%iger Zitronensäurelösung auf pH = 4, dreimal mit Ethylacetat extrahiert, mit Kochsalzlösung gewaschen und eingeengt. Nach Ausrühren des Rohproduktes mit Ether betrug die Ausbeute 1.2 g (89% d. Th.) der Titelverbindung.
$^1$H-NMR (300MHz, DMSO-$d_6$): δ [ppm]= 0.98 (s, 3H), 1.34 - 1.81 (m, 5H), 1.84 - 1.97 (m, 1H), 2.03 - 2.19 (m, 2H), 2.21 - 2.43 (m, 3H), 2.89 (dd, 2H), 3.81 (s, 3H), 6.12 - 6.20 (m, 1H), 7.20 - 7.29 (m, 1H), 7.36 (d, 1H), 7.59 - 7.70 (m, 2H), 8.15 (d, 1H), 8.20 (d, 1H).

**Intermediat 3-c**

**17-(Pyrimidin-5-yl)estra-1,3,5(10),16-tetraen-3-carbonsäure**

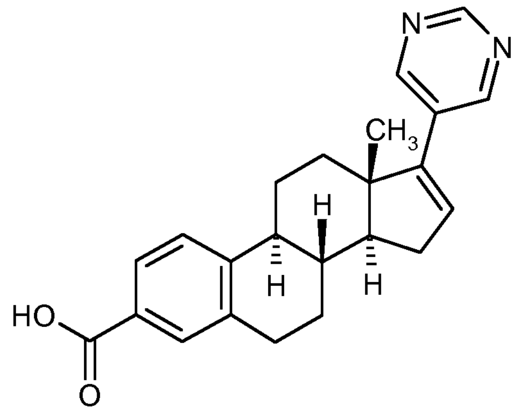

Eine Mischung aus 1.70 g (4.54 mmol) Methyl-17-(pyrimidin-5-yl)estra-1,3,5(10),16-tetraen-3-carboxylat, 40 ml THF, 11.3 ml 2M Natronlauge und 5 ml Methanol wurde bei Raumtemp. über Nacht, dann 8.5 h bei 40°C und dann über Nacht bei Raumtemp. gerührt. Danach wurde mit Wasser verdünnt, angesäuert mit 10%iger Zitronensäurelösung auf pH = 4 und Ethylacetat zugegeben. Der unlösliche Feststoff wurde abfiltriert und getrocknet. Die Ausbeute betrug 1.3 g (79% d. Th.) der Titelverbindung.
$^1$H-NMR (300MHz, DMSO-$d_6$): δ [ppm]= 0.99 (s, 3H), 1.39 - 1.79 (m, 5H), 1.84 - 1.97 (m, 1H), 2.06 - 2.21 (m, 2H), 2.26 - 2.44 (m, 3H), 2.89 (dd, 2H), 6.28 - 6.33 (m, 1H), 7.36 (d, 1H), 7.59 - 7.69 (m, 2H), 8.83 (s, 2H), 9.04 (s, 1H), 12.7 (br. s., 1H).

**Intermediat 3-d**

**17-[5-(Trifluormethyl)pyridin-3-yl]estra-1,3,5(10),16-tetraen-3-carbonsäure**

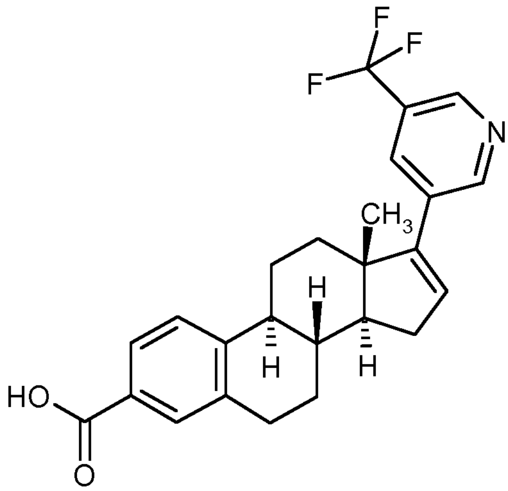

1.2 g Methyl-17-[5-(trifluormethyl)pyridin-3-yl]estra-1,3,5(10),16-tetraen-3-carboxylat wurden in 12 ml THF vorgelegt, und eine Lösung aus 0.23 g Lithiumhydroxid in 12 ml Wasser addiert und bei 40°C über Nacht gerührt. Anschließend wurde mit Wasser verdünnt, angesäuert mit 10%iger Zitronensäurelösung auf pH = 4 und dreimal mit Ethylacetat extrahiert. Danach wurde mit Kochsalzlösung gewaschen, eingeengt und mit Diethylether ausgerührt. Ausbeute: 850 mg der Titelverbindung als Feststoff.
$^1$H-NMR (400MHz, DMSO-$d_6$): δ [ppm]= 1.01 (s, 3H), 1.37 - 1.50 (m, 1H), 1.50 - 1.69 (m, 3H), 1.76 (td, 1H), 1.86 - 1.95 (m, 1H), 2.08 - 2.19 (m, 2H), 2.27 - 2.44 (m, 3H), 2.90 (dd, 2H), 6.36 (dd, 1H), 7.36 (d, 1H), 7.61 - 7.68 (m, 2H), 8.04 (s, 1H), 8.82 - 8.86 (m, 1H), 8.90 (d, 1H), 12.7 (br. s., 1H).

**Intermediat 3-e**

**17-(5-Cyanpyridin-3-yl)estra-1,3,5(10),16-tetraen-3-carbonsäure**

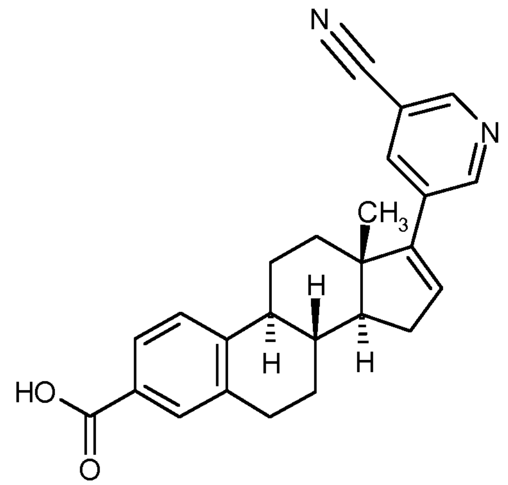

Analog wurden 105 mg Methyl-17-(5-cyanpyridin-3-yl)estra-1,3,5(10),16-tetraen-3-carboxylat mit Lithiumhydroxid in THF und Methanol umgesetzt. Nach Reinigung durch präparative HPLC (Acetonitril/Wasser/Ameisensäure) betrug die Ausbeute 27 mg der Titelverbindung.
$^1$H-NMR (300MHz, DMSO-$d_6$): δ [ppm]= 1.00 (s, 3H), 1.35 - 1.81 (m, 6H), 1.84 - 1.96 (m, 1H), 2.06 - 2.21 (m, 2H), 2.25 - 2.50 (m, zum Teil verdeckt durch DMSO Signal), 2.83 - 2.95 (m, 2H), 6.34 (br. s., 1H), 7.36 (d, 1H), 7.58 - 7.70 (m, 2H), 8.27 (t, 1H), 8.83 - 8.90 (m, 2H), 12.7 (s).

**Intermediat 3-f**

**17-(3-Pyridyl)estra-1,3,5(10),16-tetraen-3-carbonsäure**

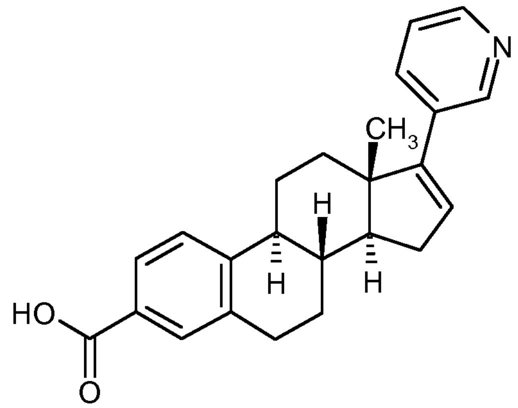

Eine Mischung aus 462 mg Methyl-17-(3-pyridyl)estra-1,3,5(10),16-tetraen-3-carboxylat, 3.1 ml 2M Natronlauge, 4 ml THF und 1 ml Methanol wurde bei 40°C 18 h gerührt. Anschließend wurde mit Wasser verdünnt, angesäuert mit zehnprozentiger Zitronensäurelösung auf pH 4 und mit Ethylacetat versetzt. Danach wurde der unlösliche Feststoff abfiltriert, mit Wasser und Ethylacetat gewaschen und getrocknet. Ausbeute: 375 mg (84% d. Th.) eines Feststoffes.
$C_{24}H_{25}NO_2$
MS (ESIpos) gefundene Masse: 359.00.
$^1$H-NMR (300MHz, DMSO-$d_6$): δ [ppm]= 0.99 (s, 3H), 1.38 - 1.78 (m, 5H), 1.81 - 1.97 (m, 1H), 2.02 - 2.19 (m, 2H), 2.19 - 2.44 (m, 4H), 2.83 - 2.96 (m, 2H), 6.09 - 6.15 (m, 1H), 6.03 - 6.24 (m, 1H), 7.29 - 7.40 (m, 2H), 7.57 - 7.68 (m, 2H), 7.77 (dt, 1H), 8.42 (dd, 1H), 8.59 (d, 1H).

**Intermediat 4**

**17-Oxoestra-1,3,5(10)-trien-3,11$\alpha$-diyl-diacetat**

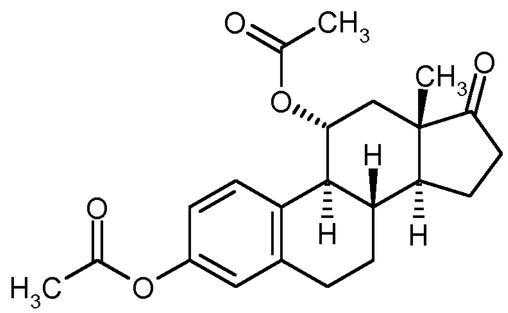

Zu einer Lösung aus 10.0 g (34.9 mmol) 3,11$\alpha$-Dihydroxyestra-1,3,5(10)-trien-17-on in 100 ml Dichlormethan wurden bei Raumtemp. 13.2 ml (4.0 Äquiv.) Essigsäureanhydrid zugetropft und das Reaktionsgemisch auf 5°C abgekühlt. Danach wurden 14.1 ml Pyridin zugetropft und 10 min auf Raumtemp. abgekühlt und 4 h gerührt. Danach wurde eine Spatelspitze DMAP addiert und 72 h bei Raumtemp. gerührt. Dann wurde auf 500 ml Wasser aufgegossen, die Phasen getrennt, mit Dichlormethan extrahiert, mit 1M Salzsäure, Wasser, Natriumchloridlösung gewaschen, über Natriumsulfat getrocknet und eingeengt. Ausbeute: 12.9 g (99% d. Th.) eines weißen Feststoffes. $^1$H-NMR (400MHz, DMSO-$d_6$): $\delta$ [ppm]= 0.81 (s, 3H), 1.29 (t, 1H), 1.43 - 1.72 (m, 4H), 1.79 - 2.00 (m, 2H), 2.00 - 2.06 (m, 3H), 2.06 - 2.19 (m, 2H), 2.19 - 2.25 (m, 3H), 2.42 - 2.57 (m, überlagert durch DMSO Signal), 2.76 (t, 2H), 5.26 (td, 1H), 6.82 - 6.89 (m, 2H), 6.97 (d, 1H).

**Intermediat 5**

**3-Hydroxy-17-oxoestra-1,3,5(10)-trien-11$\alpha$-yl-acetat**

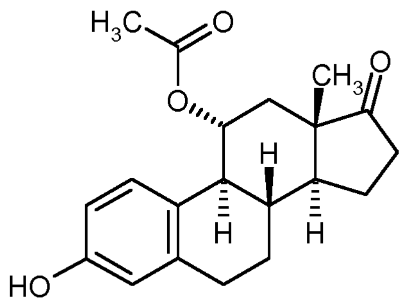

12.9 g (34.7 mmol) 17-Oxoestra-1,3,5(10)-trien-3,11$\alpha$-diyl-diacetat in 100 ml Methanol wurden mit 14.6 g (5 Äquiv.) Natriumhydrogencarbonat versetzt und die Mischung wurde über Nacht bei Raumtemp. gerührt. Danach wurde mit 100 ml Wasser und 1 ml 1M Salzsäure versetzt und 30 min gerührt. Danach wurde viermal mit Ethylacetat extrahiert. Dabei sammelte sich in der organischen Phase ein Feststoff, der abgesaugt und getrocknet wurde. Ausbeute: 3.74 g (33% d. Th.) der Titelverbindung. Zusätzlich wurden 6.39 g (56% d. Th.) der Titelverbindung isoliert durch Waschen der organischen Phase mit gesättigter Natriumchloridlösung, Trockenen über Natriumsulfat, Einengen, Ausrühren des Rückstandes aus Ethylacetat, Absaugen und Trocknen im Vakuum.
$^1$H-NMR (300MHz, DMSO-$d_6$): $\delta$ [ppm]= 0.79 (s, 3H), 1.25 (t, 1H), 1.36 - 1.69 (m, 4H), 1.75 - 1.98 (m, 2H), 1.98 - 2.18 (m, 5H), 2.34 - 2.43 (m), 2.68 (t, 2H), 5.16 (td, 1H), 6.43 - 6.55 (m, 2H), 6.76 (d, 1H), 9.07 (s, 1H).

**Intermediat 6**

**3-{[(1,1,2,2,3,3,4,4,4-Nonafluorbutyl)sulfonyl]oxy}-17-oxoestra-1,3,5(10)-trien-11$\alpha$-yl-acetat**

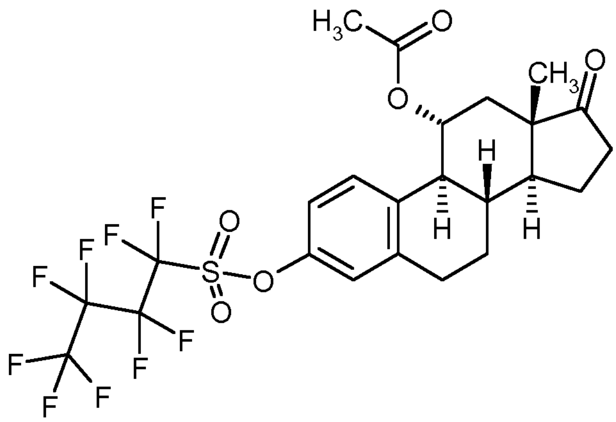

Eine Lösung aus 10.1 g (31 mmol) 3-Hydroxy-17-oxoestra-1,3,5(10)-trien-11$\alpha$-yl-acetat in 20 ml THF wurde mit 12.8 g (3 Äquiv.) Kaliumcarbonat und 6.5 ml (1.2 Äquiv.) 1,1,2,2,3,3,4,4,4-Nonafluorbutan-1-sulfonylfluorid versetzt und 4 h unter Rückfluss erhitzt und 18 h bei Raumtemp. gerührt. Danach wurden erneut 1 ml 1,1,2,2,3,3,4,4,4-Nonafluorbutan-1-sulfonylfluorid zugesetzt und 3 h unter Rückfluss erhitzt. Danach wurde mit Wasser und gesättigter Kochsalzlösung versetzt, 20 min gerührt, die Phasen getrennt und die wässrige Phase wurde dreimal mit je 50 ml Ethylacetat extrahiert. Die vereinigten organischen Phasen wurden zweimal mit je 50 ml Wasser und zweimal mit 50 ml gesättigter Kochsalzlösung gewaschen, über Natriumsulfat getrocknet, filtriert und eingeengt. Nach säulenchromatographischer Reinigung an Kieselgel (Hexan/Ethylacetat) betrug die Ausbeute 18.1 g (96% d. Th.) 3-{[(1,1,2,2,3,3,4,4,4-Nonafluorbutyl)sulfonyl]oxy}-17-oxoestra-1,3,5(10)-trien-11$\alpha$-yl-acetat.

$^1$H-NMR (400MHz, DMSO-$d_6$): $\delta$ [ppm]= 0.85 (s, 3H), 1.26 - 1.37 (m, 1H), 1.47 - 1.76 (m, 4H), 1.83 - 2.02 (m, 2H), 2.03 - 2.25 (m, 5H, enthält s bei 2.06 ppm), 2.41 - 2.47 (m), 2.59 (t, 1H), 2.77 - 2.95 (m, 2H), 5.29 (td, 1H), 7.15 (d, 1H), 7.23 - 7.29 (m, 2H).

**Intermediat 7**

**Methyl-11$\alpha$-acetoxy-17-oxoestra-1,3,5(10)-trien-3-carboxylat**

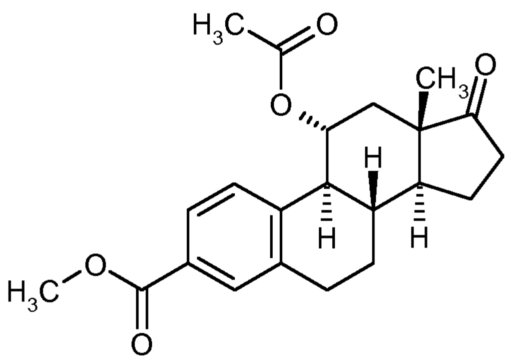

10.0 g (16.4 mmol) 3-{[(1,1,2,2,3,3,4,4,4-Nonafluorbutyl)sulfonyl]oxy}-17-oxoestra-1,3,5(10)-trien-11$\alpha$-yl-acetat, 230 mg (6 mol%) Palladium(II)acetat und 440 mg (6 mol%) 1,3-Bis(diphenylphosphino)propan wurden unter Argon in einem Autoklaven vorgelegt und mit 36 ml Methanol, 54 ml DMSO und 6 ml Triethylamin versetzt. Das Reaktionsgemisch wurde dreimal mit Kohlenmonoxid gespült und bei 7.5 bar Kohlenmonoxiddruck 30 min bei Raumtemp. gerührt. Danach wurde der Autoklav entspannt, evakuiert und bei 6.8 bar Kohlenmonoxiddruck bei 70°C 3.5 h gerührt. Danach wurde eingeengt, und der Rückstand wurde in Wasser und Ethylacetat aufgenommen. Die Phasen wurden getrennt und die wässrige Phase wurde zweimal mit Ethylacetat extrahiert. Die vereinigten organischen Phasen wurden mit 1M Salzsäure und gesättigter Natriumhydrogencarbonatlösung gewaschen, über Natriumsulfat getrocknet und eingeengt. Nach Reinigung des Rückstandes durch Säulenchromatographie an Kieselgel (Hexan/Ethylacetat) betrug die Ausbeute 5.96 g (98% d. Th.) der Titelverbindung als Feststoff.

$^1$H-NMR (300MHz, DMSO-$d_6$): $\delta$ [ppm]= 0.81 (s, 3H), 1.29 (t, 1H), 1.40 - 1.76 (m, 4H), 1.78 - 2.00 (m, 2H), 2.00 - 2.21

(m, 5H, enthält s bei 2.03 ppm), 2.37 - 2.52 (m, verdeckt durch DMSO Signal), 2.59 (t, 1H), 2.72 - 2.93 (m, 2H), 3.79 (s, 3H), 5.29 (td, 1H), 5.23 - 5.38 (m, 1H), 7.08 (d, 1H), 7.68 - 7.75 (m, 2H).

**Intermediat 8**

**Methyl-11$\alpha$-acetoxy-17-{[(trifluormethyl)sulfonyl]oxy}estra-1,3,5(10),16-tetraen-3-carboxylat**

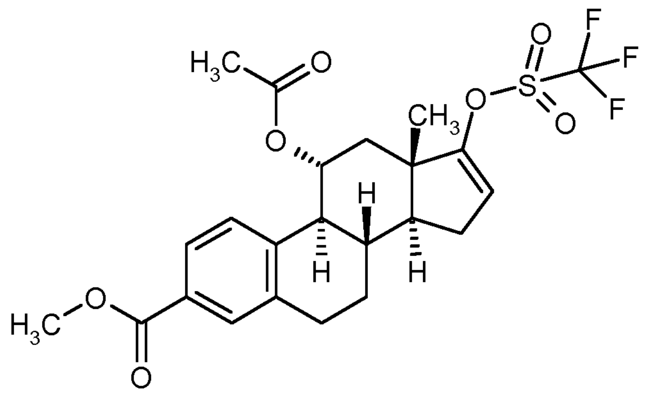

2.96 g (7.99 mmol) Methyl-11$\alpha$-acetoxy-17-oxoestra-1,3,5(10)-trien-3-carboxylat wurden analog zur Herstellung von Intermediat 1 zu 5.13 g der Titelverbindung als Rohprodukt (enthielt noch 2,6-Di-tert-butylpyridin) umgesetzt. $^1$H-NMR (300MHz, DMSO-$d_6$): $\delta$ [ppm]= 0.93 (s, 3H), 1.41 - 1.71 (m, 3H), 1.71 - 1.87 (m, 1H), 1.87 - 2.16 (m, 5H, enhält s bei 2.03 ppm), 2.16 - 2.40 (m, 2H), 2.67 (t, 1H), 2.74 - 2.93 (m, 2H), 3.79 (s, 3H), 5.34 (td, 1H), 5.75 - 5.82 (m, 1H), 7.03 (d, 1H), 7.67 - 7.75 (m, 2H).

**Intermediat 9**

**Methyl-11$\alpha$-acetoxy-17-(5-fluorpyridin-3-yl)estra-1,3,5(10),16-tetraen-3-carboxylat**

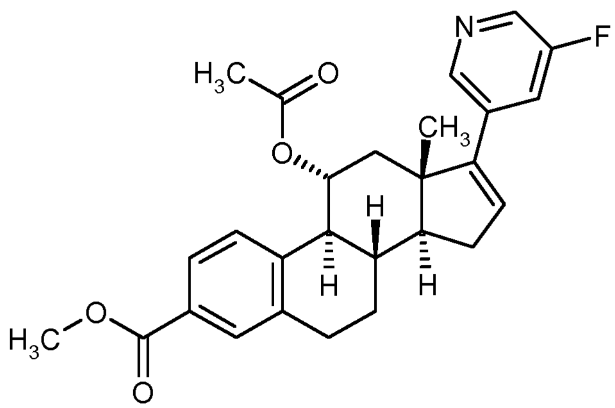

2.50 g (4.98 mmol) Methyl-11$\alpha$-acetoxy-17-{[(trifluormethyl)sulfonyl]oxy}estra-1,3,5(10),16-tetraen-3-carboxylat wurden mit 981 mg (1.4 Äquiv.) 5-Fluorpyridin-3-boronsäure in Gegenwart von 170 mg (5 mol%) [1,3-Bis(2,6-Diisopropylphenyl)imidazol-2-yliden](3-chlorpyridyl)palladium(II)dichlorid (PEPPSI™-IPr, CAS 905459-27-0) analog zu Intermediat 2-a innerhalb von 5 h bei Rückflusstemperatur umgesetzt. Ausbeute: 2.62 g der Titelverbindung als Rohprodukt.

**Intermediat 10**

**Methyl-17-(5-fluorpyridin-3-yl)-11$\alpha$-hydroxyestra-1,3,5(10),16-tetraen-3-carboxylat**

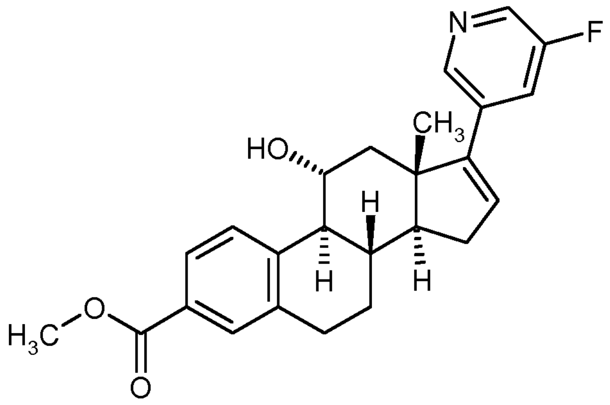

Zu 2.62 g (5.83 mmol) Methyl-11$\alpha$-acetoxy-17-(5-fluorpyridin-3-yl)estra-1,3,5(10),16-tetraen-3-carboxylat in 40 ml Methanol wurden 4.0 g (5 Äquiv.) Kaliumcarbonat gegeben und die Mischung wurde 3 h bei Raumtemp. gerührt. Anschließend wurde mit Wasser und 1M Salzsäure verdünnt und dreimal mit Ethylacetat extrahiert. Die vereinigten organischen Phasen wurden mit Wasser und gesättigter Natriumchloridlösung gewaschen, über Natriumsulfat getrocknet und eingeengt. Nach Säulenchromatographie an Kieselgel (Hexan/Ethylacetat) betrug die Ausbeute 1.19 g (50% d. Th.) der Titelverbindung.
$^1$H-NMR (300MHz, DMSO-$d_6$, ausgewählte Signale): $\delta$ [ppm]= 0.95 (s, 3H), 1.40 - 1.61 (m, 3H), 2.78 - 2.97 (m, 2H), 3.79 (s, 3H), 4.06 - 4.21 (m, 1H), 4.79 - 4.92 (m, 1H), 6.26 (br. s., 1H), 7.59 - 7.74 (m, 3H), 8.07 (d, 1H), 8.39 - 8.54 (m, 2H).

**Intermediat 11**

**Methyl-11$\beta$-fluor-17-(5-fluorpyridin-3-yl)estra-1,3,5(10),16-tetraen-3-carboxylat**

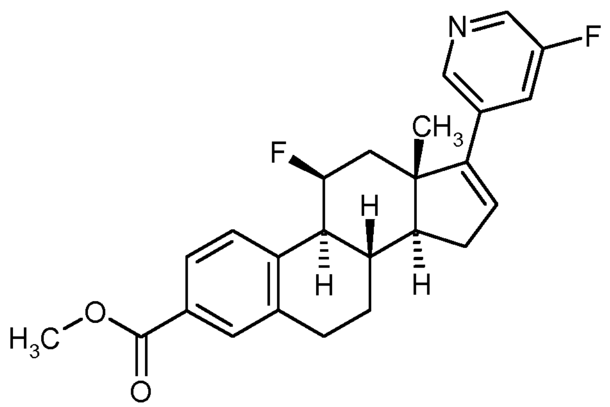

Zu einer eiskalten Lösung aus 531 mg (3.49 mmol) Methyl-17-(5-fluorpyridin-3-yl)-11$\alpha$-hydroxyestra-1,3,5(10),16-tetraen-3-carboxylat in 15 ml THF wurden 0.52 ml (1.65 Äquiv.) 1,8-Diazabicyclo[5.4.0]undec-7-en und 0.58 ml (1.5 Äquiv.) 1,1,2,2,3,3,4,4,4-Nonafluorbutan-1-sulfonylfluorid getropft und es wurde unter Eisbadkühlung 3 h gerührt. Danach wurde eingeengt und durch Säulenchromatographie an Kieselgel (Hexan/Ethylacetat) gereinigt. Die Ausbeute betrug 747 mg (84% d. Th.) der Titelverbindung als Rohprodukt.
$^1$H-NMR (300MHz, DMSO-$d_6$, ausgewählte Signale): $\delta$ [ppm]= 2.86 - 2.97 (m, 2H), 5.57 - 5.83 (m, 1H), 6.26 - 6.32 (m, 1H), 7.45 - 7.53 (m, 1H), 7.65 - 7.78 (m, 3H), 8.39 - 8.53 (m, 2H).

**Intermediat 12**

**11β-Fluor-17-(5-fluorpyridin-3-yl)estra-1,3,5(10),16-tetraen-3-carbonsäure**

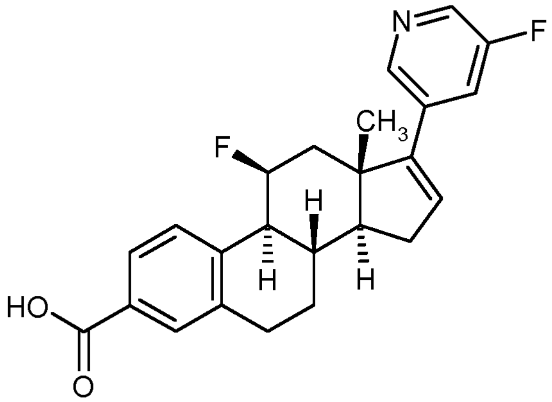

Eine Mischung aus 862 mg (2.11 mmol) Methyl-11β-fluor-17-(5-fluorpyridin-3-yl)estra-1,3,5(10),16-tetraen-3-carboxylat in 10 ml THF wurde mit 5 ml Methanol und 442 mg Lithiumhydroxid Monohydrat in 5 ml Wasser versetzt und bei Raumtemp. über Nacht gerührt. Das Reaktionsgemisch wurde mit Wasser versetzt und mit 10%iger wässriger Zitronensäurelösung auf pH = 4 eingestellt. Der ausgefallene Niederschlag wurde abgesaugt und mit Ethylacetat gewaschen und getrocknet. Ausbeute: 498 mg (60% d. Th.) eines weißen Feststoffes.
$^1$H-NMR (500MHz, DMSO-$d_6$): δ [ppm]= 1.19 (s, 3H), 1.44 - 1.59 (m, 1H), 1.80 - 1.96 (m, 2H), 1.96 - 2.08 (m, 2H), 2.18 - 2.29 (m, 1H), 2.32 - 2.42 (m, 1H), 2.59 (td, 1H), 2.74 (dd, 1H), 2.77 (br. s., 1H), 2.86 - 3.00 (m, 2H), 5.66 - 5.80 (m, 1H), 6.32 (dd, 1H), 7.48 (d, 1H), 7.65 - 7.78 (m, 3H), 8.47 (d, 1H), 8.54 (t, 1H).

**Intermediat 13**

**17-Hydrazonoestra-1,3,5(10)-trien-3-carboxamid**

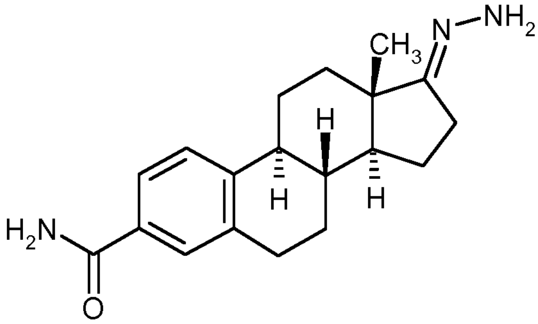

Zu 3.34 g (11.2 mmol) 17-Oxoestra-1,3,5(10)-trien-3-carboxamid (E. Morera, G. Ortar, THL, 1998, 39, 2835 - 2838) in 70 ml Ethanol gab man 3.65 g Hydrazinhydrat und 9 mg Hydrazinsulfat gelöst in 3 ml Wasser und rührte 4 Tage bei Raumtemp.. Danach wurde auf 200 ml Eiswasser aufgegossen und 40 min gerührt. Anschließend wurde der Niederschlag abgesaugt. Ausbeute: nach Trocknen im Vakuum 3.07 g (88% d. Th.) der Titelverbindung.
$^1$H-NMR (300MHz, DMSO-d6): δ [ppm]= 0.76 (s, 3H), 1.15 - 1.55 (m, 6H), 1.75 - 1.99 (m, 3H), 2.01 - 2.41 (m, 4H), 2.75 - 2.96 (m, 2H), 5.32 (br. s., 2H), 7.16 (br. s., 1H), 7.30 (d, 1H), 7.49 - 7.64 (m, 2H), 7.80 (br. s., 1H).

**Intermediat 14**

**17-Iodestra-1,3,5(10),16-tetraen-3-carboxamid**

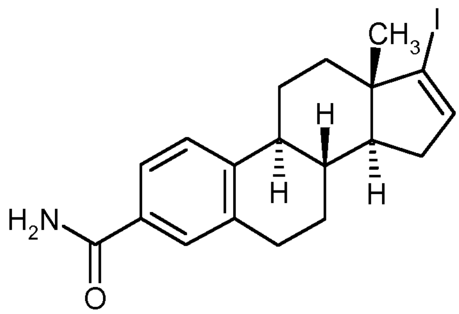

3.07 g (9.56 mmol) 17-Hydrazonoestra-1,3,5(10)-trien-3-carboxamid wurden in 65 ml Dioxan vorgelegt und 11 ml Triethylamin zugegeben. Innerhalb von 30 min wurden 10.0 g (4.0 Äquiv.) Iod addiert und 3 h bei Raumtemp. gerührt. Dann wurde auf 10%ige Natriumsulfit-Lösung aufgegossen, 40 min gerührt und der ausgefallene Feststoff abgesaugt und mit Wasser nachgewaschen. Es wurden 3.23 g eines braunen Feststoffs (Rohprodukt) erhalten.
1H-NMR (300MHz, DMSO-d6, ausgewählte Signale): δ [ppm]= 0.67 (s, 3H), 1.81 - 1.91 (m, 1H), 1.94 - 2.09 (m, 1H), 2.12 - 2.35 (m), 2.79 - 2.95 (m, 2H), 6.18 (s, 1H), 7.18 (br. s., 1H), 7.29 (d, 1H), 7.48 - 7.63 (m, 2H), 7.81 (br. s., 1H).

**Intermediat 15**

**3-Cyanestra-1,3,5(10),16-tetraen-17-yl-trifluormethansulfonat**

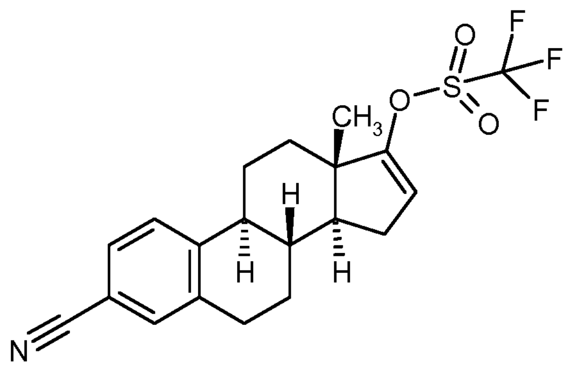

Zu einer Lösung aus 1.50 g (5.37 mmol) 17-Oxoestra-1,3,5(10)-trien-3-carbonitril (Journal of the Chemical Society, 1964, 5889) in 34 ml Dichlormethan wurden 2.4 ml 2,6-Di-*tert*-butylpyridin (2 Äquiv.) und 1.1 ml Trifluormethansulfonsäureanhydrid zugetropft. Danach wurde 22 h bei Raumtemp. gerührt, vorsichtig auf 100 ml gesättigte Natriumhydrogencarbonatlösung aufgegossen, 45 min gerührt und die organischen Phasen abgetrennt. Danach wurde zweimal mit Dichlormethan extrahiert, die vereinigten organischen Phasen mit gesättigter Natriumhydrogencarbonatlösung, gesättigter Kochsalzlösung gewaschen, über Natriumsulfat getrocknet und eingeengt. Der verbleibende Feststoff wurde mit Hexan ausgerührt. Ausbeute: 1.51 g der Titelverbindung als Rohprodukt.
$^{1}$H-NMR (300MHz, CHLOROFORM-d): δ [ppm]= 1.01 (s, 3H), 5.61 - 5.67 (m, 1H), 7.32 - 7.47 (m, 3H).

**Intermediat 16-a**

**17-(5-Fluorpyridin-3-yl)estra-1,3,5(10),16-tetraen-3-carbonitril**

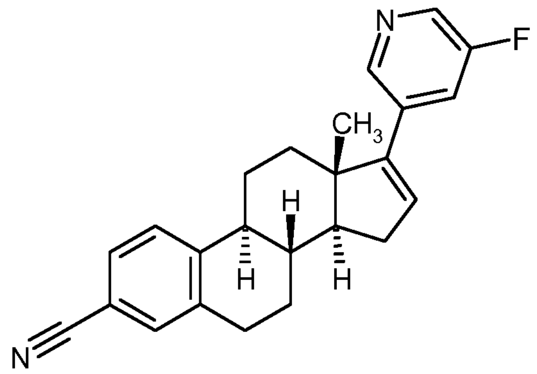

Eine Mischung aus 400 mg (0.97 mmol) 3-Cyanestra-1,3,5(10),16-tetraen-17-yl-trifluormethansulfonat, 192 mg (1.4 Äquiv.) 5-Fluorpyridin-3-boronsäure, 4 ml Toluol, 3 ml Ethanol, 82 mg Lithiumchlorid und 1.3 ml 2M wässriger Natriumcarbonatlösung wurde mit 56 mg $Pd(PPh_3)_4$ versetzt und 60 min bei 120°C in der Mikrowelle erhitzt. Danach wurde über Celite filtriert, die organische Phase abgetrennt, die wässrige Phase dreimal mit Ethylacetat extrahiert und die vereinigten organischen Phasen mit gesättigter Natriumhydrogencarbonatlösung und gesättigter Kochsalzlösung gewaschen, über Natriumsulfat getrocknet und eingeengt. Nach säulenchromatographischer Reinigung an Kieselgel betrug die Ausbeute 162 mg (47% d. Th.) eines Feststoffes. $C_{24}H_{23}FN_2$ MS (ESIpos) gefundene Masse: 358.00.
$^1$H-NMR (300MHz, CHLOROFORM-d): δ [ppm]= 1.06 (s, 3H), 1.43 - 1.90 (m), 1.96 - 2.10 (m, 1H), 2.11 - 2.28 (m, 2H), 2.31 - 2.53 (m, 3H), 2.95 (dd, 2H), 6.10 (br. s., 1H), 7.32 - 7.52 (m, 4H), 8.35 (d, 1H), 8.47 (s, 1H).

**Intermediat 16-b**

**17-(Pyrimidin-5-yl)estra-1,3,5(10),16-tetraen-3-carbonitril**

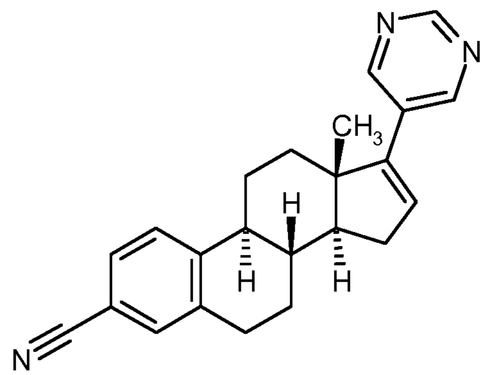

Analog zur Herstellung von Intermediat 16-a wurden 400 mg (0.97 mmol) 3-Cyanestra-1,3,5(10),16-tetraen-17-yl-trifluormethansulfonat mit 169 mg Pyrimidin-5-boronsäure umgesetzt. Ausbeute: 101 mg der Titelverbindung als Rohprodukt. MS (ESIpos) gefundene Masse: 341.00.
$^1$H-NMR (400MHz, CHLOROFORM-d): δ [ppm]= 6.11 - 6.20 (m, 1H), 8.72 - 8.80 (2H), 9.07 - 9.14 (1H).

**Beispiel 1**

**17-(Pyridin-3-yl)estra-1,3,5(10),16-tetraen-3-carboxamid**

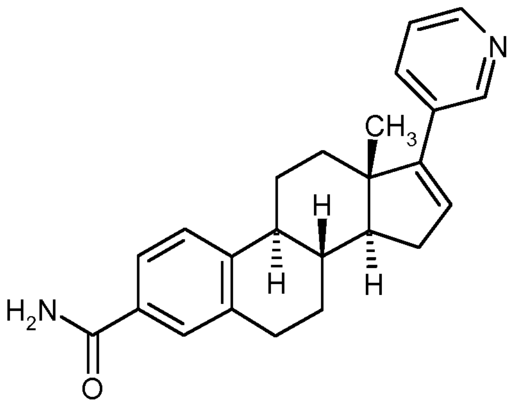

Eine Mischung aus 150 mg (0.37 mmol) 17-Iodestra-1,3,5(10),16-tetraen-3-carboxamid, 63 mg (0.52 mmol) Pyridin-3-boronsäure, 31 mg Lithiumchlorid, 1.5 ml Toluol, 493 Microliter 2M Natriumcarbonatlösung und 1 ml Ethanol wurde mit 13 mg Bis(triphenylphosphin)palladium(II)chlorid versetzt und in der Mikrowelle 90 min bei 120°C / 100 Watt erhitzt. Danach wurde filtriert, die Phasen getrennt und dreimal mit Ethylacetat extrahiert. Die vereinigten organischen Phasen wurden mit gesättigter Natriumhydrogencarbonatlösung und Natriumchloridlösung gewaschen, über Natriumsulfat getrocknet und eingeengt. Nach Reinigung des Rückstandes durch präparative HPLC (Acetonitril/Wasser/Ameisensäure) wurden 9 mg eines Feststoffes erhalten. UPLC Analytik (Methode 1) Rt = 0.94 min, gefundene Masse ESI(+) 358.20.
1H-NMR (300MHz, DMSO-d6, ausgewählte Signale): δ [ppm]= 0.99 (s, 3H), 1.38 - 1.78 (m, 6H), 1.91 (d, 1H), 2.02 - 2.20 (m, 2H), 2.87 (d, 2H), 6.12 (s., 1H), 7.19 (s., 1H), 7.25 - 7.38 (m, 2H), 7.50 - 7.64 (m, 2H), 7.70 - 7.97 (m, 2H), 8.42 (d, 1H), 8.59 (s, 1H).

**Beispiel 2**

**17-(5-Methoxypyridin-3-yl)estra-1,3,5(10),16-tetraen-3-carboxamid**

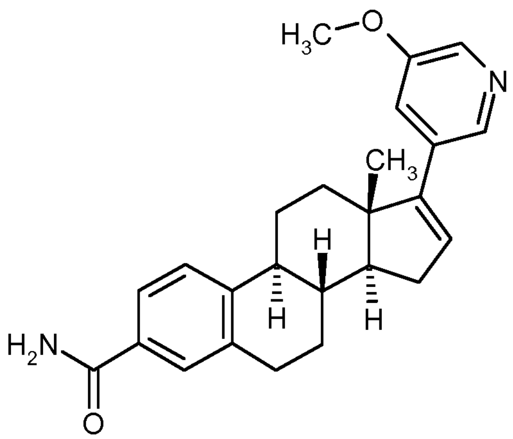

Analog wurden 100 mg (0.24 mmol) 17-Iodestra-1,3,5(10),16-tetraen-3-carboxamid und 53 mg (1.4 Äquiv.) 5-Methoxypyridin-3-boronsäure mit 14 mg Tetrakis(triphenylphosphin)palladium(0) als Katalysator zu 7 mg der Titelverbindung umgesetzt. UPLC Analytik (Methode 1) Rt = 1.24 min, gefundene Masse ESI(+) 388.22.
1H-NMR (400MHz, DMSO-d6): δ [ppm]= 0.99 (s, 3H), 1.37 - 1.67 (m, 4H), 1.74 (td, 1H), 1.85 - 1.97 (m, 1H), 2.04 - 2.18 (m, 2H), 2.20 - 2.37 (m, 2H), 2.79 - 2.97 (m, 2H), 3.82 (s, 3H), 6.16 (s., 1H), 7.16 (br. s., 1H), 7.20 - 7.33 (m), 7.52 - 7.62 (m, 2H), 7.80 (br. s., 1H), 8.16 (d, 1H), 8.20 (d, 1H).

**Beispiel 3**

**17-[5-(Trifluormethyl)pyridin-3-yl]estra-1,3,5(10),16-tetraen-3-carboxamid**

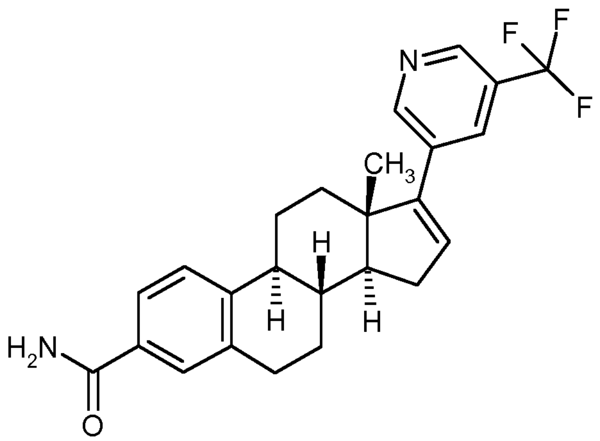

Analog wurden 150 mg (0.37 mmol) 17-Iodestra-1,3,5(10),16-tetraen-3-carboxamid und 141 mg (1.4 Äquiv.) 3-(4,4,5,5-Tetramethyl-1,3,2-dioxaborolan-2-yl)-5-(trifluormethyl)pyridin mit 21 mg Tetrakis(triphenylphosphin)palladium(0) als Katalysator zu 31 mg (20% d. Th.) der Titelverbindung umgesetzt. UPLC Analytik (Methode 1) Rt = 1.42 min, gefundene Masse ESI(+) 426.19. $^1$H-NMR (300MHz, DMSO-$d_6$): $\delta$ [ppm]= 1.01 (s, 3H), 1.38 - 1.68 (m), 1.75 (td, 1H), 1.84 - 1.96 (m, 1H), 2.07 - 2.20 (m, 2H), 2.22 - 2.44 (m), 2.79 - 3.01 (m, 2H), 6.36 (br. s., 1H), 7.19 (br. s., 1H), 7.27 - 7.34 (m, 1H), 7.52 - 7.66 (m, 2H), 7.82 (br. s., 1H), 8.04 (s, 1H), 8.83 (s, 1H), 8.87 - 8.95 (m, 1H).

**Beispiel 4**

**17-(5-Fluorpyridin-3-yl)estra-1,3,5(10),16-tetraen-3-carboxamid Herstellungsmethode A**

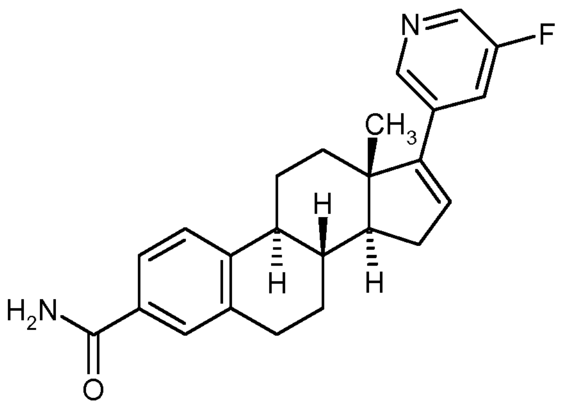

700 mg (1.85 mmol) 17-(5-Fluorpyridin-3-yl)estra-1,3,5(10),16-tetraen-3-carbonsäure wurden in 15 ml 2-Methyltetrahydrofuran und 2 ml NMP vorgelegt, 1,1'-Carbonyldiimidazol und Imidazol Hydrochlorid wurden zuaddiert und 18 h bei Raumtemp. gerührt. Dann wurden 4.4 ml 25%ige wässrige Ammoniaklösung addiert und 72 h bei Raumtemp. gerührt. Danach wurde mit 1M Salzsäurelösung, Wasser und Ethylacetat versetzt und 10 min gerührt. Der Feststoff wurde abgesaugt und getrocknet. Ausbeute: 406 mg (58% d. Th.) der Titelverbindung. $^1$H-NMR (300MHz, DMSO-$d_6$): $\delta$ [ppm]= 0.99 (s, 3H), 1.38 - 1.78 (m, 5H), 1.85 - 1.96 (m, 1H), 2.04 - 2.20 (m, 2H), 2.20 - 2.44 (m, 3H), 2.81 - 2.93 (m, 2H), 6.23 - 6.30 (m, 1H), 7.18 (br. s., 1H), 7.30 (d, 1H), 7.54 - 7.72 (m, 3H), 7.82 (br. s., 1H), 8.41 - 8.52 (m, 2H).

**Herstellungsmethode B**

Eine Mischung aus 50 mg 17-(5-Fluorpyridin-3-yl)estra-1,3,5(10),16-tetraen-3-carbonitril in 3 ml Ethanol und 2 ml Wasser wurde mit 86 mg (4 Äquiv.) Natriumperborat Tetrahydrat versetzt und 30 min bei 130°C bei 300 Watt in der Mikrowelle erhitzt. 21 mg Natriumperborat Tetrahydrat wurden addiert und 15 min bei 130°C bei 300 Watt erwärmt.

Weitere 98 mg 17-(5-Fluorpyridin-3-yl)estra-1,3,5(10),16-tetraen-3-carbonitril wurden in 5 ml Ethanol und 3 ml Wasser mit 210 mg (5 Äquiv.) Natriumperborat Tetrahydrat 30 min bei 130°C in der Mikrowelle erhitzt. Die Reaktionsgemische wurden vereinigt und dreimal mit tert-Butylmethylether extrahiert, mit Kochsalzlösung gewaschen, über Natriumsulfat getrocknet, eingeengt und der Rückstand durch präparative HPLC gereinigt. Ausbeute: 75 mg der Titelverbindung.

**Beispiel 5**

**17-(Pyrimidin-5-yl)estra-1,3,5(10),16-tetraen-3-carboxamid**

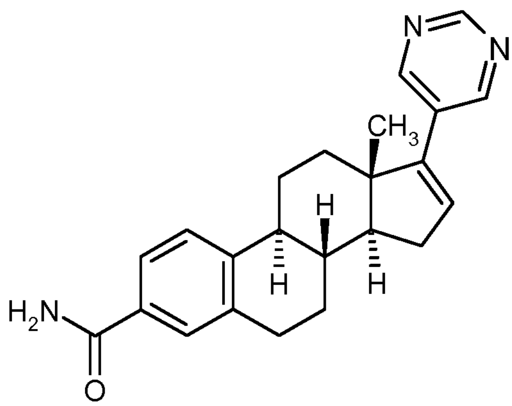

Analog zur Herstellung von Beispiel 6 (Herstellungsmethode B) wurden 88 mg 17-(Pyrimidin-5-yl)estra-1,3,5(10),16-tetraen-3-carbonitril mit Natriumperborat bei 140°C und 300 Watt in der Mikrowelle umgesetzt. Nach präparativer HPLC erhielt man 11 mg der Titelverbindung. $C_{23}H_{25}N_3O$ UPLC Analytik (Methode 1) Rt = 1.14 min, gefundene Masse ESI(+) 359.20 $^1$H-NMR (300MHz, DMSO-$d_6$): δ [ppm]= 0.99 (s, 3H), 1.35 - 1.80 (m, 5H), 1.85 - 1.96 (m, 1H), 2.07 - 2.20 (m, 2H), 2.26 - 2.44 (m, 3H), 2.82 - 2.93 (m, 2H), 6.28 - 6.33 (m, 1H), 7.18 (br. s., 1H), 7.30 (d, 1H), 7.55 - 7.62 (m, 2H), 7.82 (br. s., 1H), 8.83 (s, 2H), 9.04 (s, 1H).

**Beispiel 6**

**17-(5-Cyanpyridin-3-yl)estra-1,3,5(10),16-tetraen-3-carboxamid**

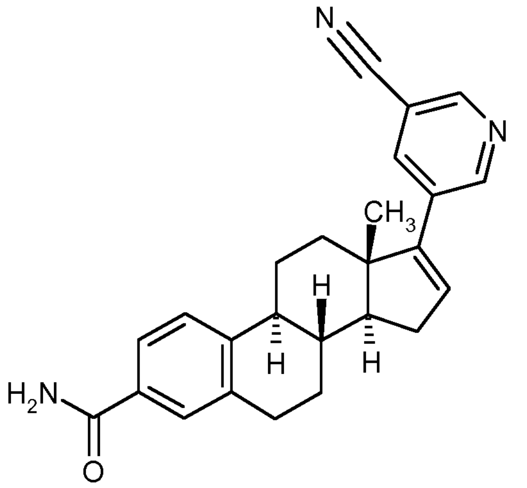

17 mg (0.044 mmol) 17-(5-Cyanpyridin-3-yl)estra-1,3,5(10),16-tetraen-3-carbonsäure wurden in 0.4 ml 2-Methyltetrahydrofuran vorgelegt. Dann wurden 11 mg 1,1'-Carbonyldiimidazol und 2 mg 1*H*-Imidazol Hydrochlorid dazugegeben und bei Raumtemp. 18 h gerührt. Dann wurden 79 µl 33%ige Ammoniak-Lösung addiert und 72 h bei Raumtemp. gerührt, mit 10 ml 1M Salzsäurelösung versetzt, mit Ethylacetat extrahiert, eingeengt und das Rohprodukt durch präparative HPLC (Acetonitril/Wasser/Ameisensäure) gereinigt. Ausbeute: 9 mg der Titelverbindung.
$^1$H-NMR (400MHz, DMSO-$d_6$): δ [ppm]= 1.00 (s, 3H), 1.35 - 1.50 (m, 1H), 1.50 - 1.68 (m, 3H), 1.73 (td, 1H), 1.85 - 1.98 (m, 1H), 2.07 - 2.23 (m, 2H), 2.25 - 2.36 (m, 2H), 2.36 - 2.44(m, zum Teil verdeckt durch DMSO Signal), 2.83 - 2.95 (m, 2H), 6.33 - 6.36 (m, 1H), 7.19 (br. s., 1H), 7.31 (d, 1H), 7.55 - 7.61 (m, 2H), 7.82 (br. s., 1H), 8.28 (t, 1H), 8.87 (dd, 2H).

**Beispiel 7**

**11*β*-Fluor-17-(5-fluorpyridin-3-yl)estra-1,3,5(10),16-tetraen-3-carboxamid**

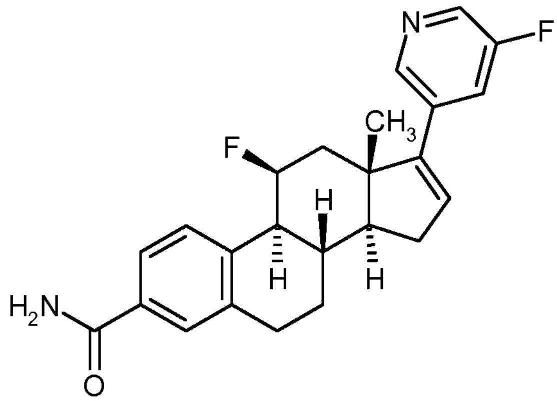

100 mg (11beta)-11-Fluor-17-(5-fluorpyridin-3-yl)estra-1,3,5(10),16-tetraen-3-carbonsäure wurden in 3 ml 2-Methyltetrahydrofuran vorgelegt, dann wurden 62 mg (1.5 Äquiv.) 1,1'-Carbonyldiimidazol und 13 mg Imidazol Hydrochlorid addiert und 18 h bei Raumtemp.gerührt. Dann wurden 597 µl 25%ige wässrige Ammoniaklösung addiert und 3 h bei Raumtemp. gerührt, mit 10 ml 1M Salzsäurelösung versetzt, dreimal mit Ethylacetat extrahiert, eingeengt und durch präparative HPLC gereinigt. Ausbeute: 51 mg der Titelverbindung. $C_{24}H_{24}F_2N_2O$ UPLC Analytik (Methode 1) Rt = 1.22 gefundene Masse ESI(+) 394.19.
1H-NMR (400MHz, DMSO-d6): δ [ppm]= 1.14 (s, 3H), 1.40 - 1.55 (m, 1H), 1.73 - 2.03 (m, 4H), 2.13 - 2.25 (m, 1H), 2.27 - 2.38 (m, 1H), 2.50 - 2.60 (m, 1H), 2.67 (dd, 1H), 2.80 - 2.97 (m, 2H), 5.60 - 5.81 (1H), 6.30 (br. s., 1H), 7.22 (br. s., 1H), 7.40 (d, 1H), 7.55 - 7.63 (m, 2H), 7.71 (dt, 1H), 7.85 (s, 1H), 8.45 (d, 1H), 8.48 - 8.55 (m, 1H).

**Beispiel 8**

**11β-Fluor-17-(5-fluorpyridin-3-yl)-*N*-(2-sulfamoylethyl)estra-1,3,5(10),16-tetraen-3-carboxamid**

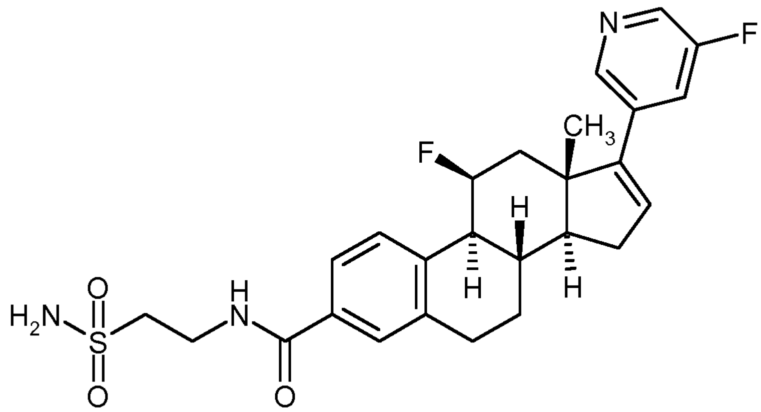

Zu einer Mischung aus 100 mg 11β-Fluor-17-(5-fluorpyridin-3-yl)estra-1,3,5(10),16-tetraen-3-carbonsäure und 81 mg (2 Äquiv.) 2-Aminoethan-1-sulfonamid Hydrochlorid in 0.5 ml DMF und 3 ml THF wurden 39 mg 1-Hydroxy-1*H*-benzotriazol Hydrat, 97 mg (2 Äquiv.) *N*-[3-(Dimethylamino)propyl]-*N'*-ethylcarbodiimid Hydrochlorid (EDC) [CAS 25952-53-8] und 0.11 ml Triethylamin zugegeben und es wurde 18 h bei Raumtemp. gerührt. Nach Zugabe von Wasser wurde dreimal mit Ethylacetat extrahiert, die vereinigten organischen Phasen wurden eingeengt und durch präparative HPLC gereinigt. Ausbeute: 24 mg der Titelverbindung. $C_{26}H_{29}F_2N_3O_3S$ UPLC Analytik (Methode 1) Rt = 1.19 min, gefundene Masse ESI(+) 501.19.
$^1$H-NMR (400MHz, DMSO-$d_6$): δ [ppm]= 1.14 (s, 3H), 1.48 (qd, 1H), 1.75 - 2.01 (m, 4H), 2.14 - 2.24 (m, 1H), 2.28 - 2.37 (m, 1H), 2.48 - 2.66 (m, 2H), 2.71 - 2.77 (m, 0.5H), 2.82 - 2.97 (m, 2H), 3.19 (dd, 2H), 3.56 - 3.63 (m, 2H), 5.64 (br. s, 0.5H), 5.76 (br. s., 0.5H), 6.28 - 6.32 (m, 1H), 6.91 (s, 2H), 7.43 (d, 1H), 7.53 - 7.59 (m, 2H), 7.69 - 7.74 (m, 1H), 8.43 - 8.53 (m, 3H).

**Beispiel 9**

**17-(5-Fluorpyridin-3-yl)-*N*-[(1*S*,2*R*)-2-hydroxycyclopentyl]estra-1,3,5(10),16-tetraen-3-carboxamid**

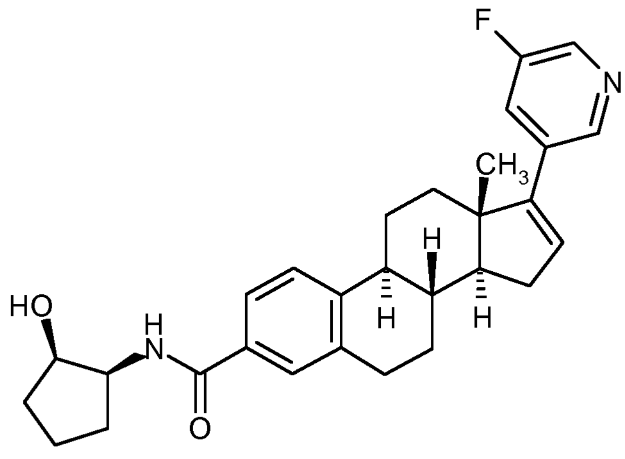

Analog zu Beispiel 8 wurden 100 mg 17-(5-Fluorpyridin-3-yl)estra-1,3,5(10),16-tetraen-3-carbonsäure mit 73 mg (1*R*,2*S*)-2-Aminocyclopentan-1-ol Hydrochlorid (1:1) zu 75 mg der Titelverbindung umgesetzt.
UPLC Analytik (Methode 1) Rt = 1.40 min, gefundene Masse ESI(+) 460.25.

$^1$H-NMR (300MHz, DMSO-$d_6$): δ [ppm]= 1.00 (s, 3H), 1.42 - 1.97 (m, 12H), 2.03 - 2.21 (m, 2H), 2.21 - 2.44 (m, 3H), 2.84 - 2.94 (m, 2H), 3.94 - 4.05 (m, 2H), 4.68 (d, 1H), 6.24 - 6.29 (m, 1H), 7.31 (d, 1H), 7.54 - 7.71 (m, 4H), 8.41 - 8.51 (m, 2H).

**Beispiel 10**

**17-(5-Fluorpyridin-3-yl)-*N*-[2-(hydroxymethyl)-2-methylpropyl]estra-1,3,5(10),16-tetraen-3-carboxamid**

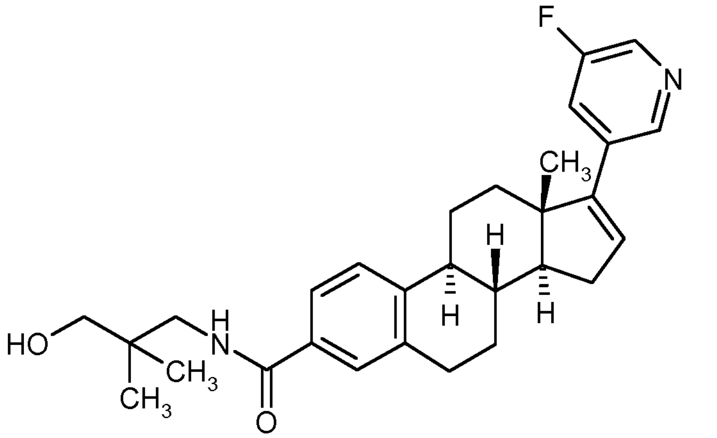

Analog zu Beispiel 8 wurden 100 mg 17-(5-Fluorpyridin-3-yl)estra-1,3,5(10),16-tetraen-3-carbonsäure mit 55 mg 3-Amino-2,2-dimethylpropan-1-ol zu 53 mg der Titelverbindung umgesetzt. Nach präparativer Reinigung durch HPLC wurde das Rohprodukt mit 1 ml DMSO versetzt, der verbleibende Feststoff wurde abgesaugt und dreimal mit je 0.5 ml DMSO gespült. Das Filtrat wurde mit Wasser und gesättigter Natriumhydrogencarbonatlösung versetzt und im Anschluss mit Ethylacetat extrahiert. Die vereinigten organischen Phasen wurden mit über Natriumsulfat getrocknet und eingeengt. Ausbeute: 54 mg der Titelverbindung.
$^1$H-NMR (400MHz, DMSO-$d_6$): δ [ppm]= 0.79 (s, 6H), 1.00 (s, 3H), 1.37 - 1.66 (m, 4H), 1.74 (td, 1H), 1.87 - 1.96 (m, 1H), 2.07 - 2.20 (m, 2H), 2.24 - 2.44 (m, 3H), 2.84 - 2.96 (m, 2H), 3.09 (dd, 4H), 4.59 (t, 1H), 6.27 (br. s., 1H), 7.32 (d, 1H), 7.52 - 7.59 (m, 2H), 7.68 (dt, 1H), 8.25 (t, 1H), 8.43 (d, 1H), 8.49 (s, 1H).

**Beispiel 11**

**17-(5-Fluorpyridin-3-yl)-*N*-[(1*S*,2*S*)-2-hydroxycyclopentyl]estra-1,3,5(10),16-tetraen-3-carboxamid**

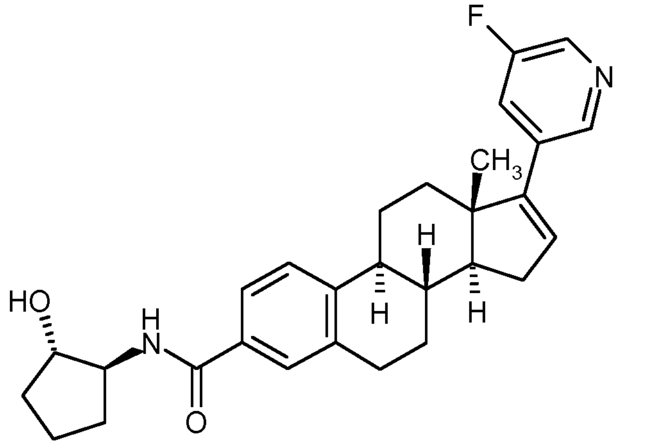

Analog zu Beispiel 8 wurden 100 mg 17-(5-Fluorpyridin-3-yl)estra-1,3,5(10),16-tetraen-3-carbonsäure mit 72 mg (1*S*,2*S*)-2-Aminocyclopentan-1-ol Hydrochlorid (1:1) zu 79 mg der Titelverbindung umgesetzt. $C_{29}H_{33}FN_2O_2$ UPLC

Analytik (Methode 1) Rt = 1.39 min, gefundene Masse ESI(+) 460.25.
$^1$H-NMR (300MHz, DMSO-$d_6$): δ [ppm]= 0.99 (s, 3H), 1.32 - 2.02 (m, 13H), 2.05 - 2.20 (m, 2H), 2.24 - 2.43 (m, 3H), 2.50 (br. s., 1H), 2.83 - 2.93 (m, 2H), 3.25 (s, 1H), 3.89 - 3.99 (m, 2H), 4.70 (d, 1H), 6.24 - 6.29 (m, 1H), 7.30 (d, 1H), 7.51 - 7.60 (m, 2H), 7.67 (dt, 1H), 8.05 (d, 1H), 8.41 - 8.52 (m, 2H).

**Beispiel 12**

**17-(5-Fluorpyridin-3-yl)-*N*-[(*R*)-3-(hydroxymethyl)butyl]estra-1,3,5(10),16-tetraen-3-carboxamid**

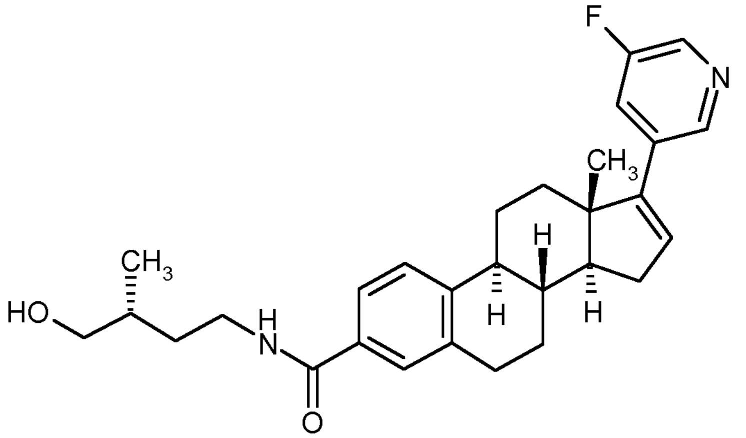

Analog zu Beispiel 8 wurden 100 mg 17-(5-Fluorpyridin-3-yl)estra-1,3,5(10),16-tetraen-3-carbonsäure mit 55 mg (2*R*)-4-Amino-2-methylbutan-1-ol zu 70 mg der Titelverbindung umgesetzt.
$C_{29}H_{35}FN_2O_2$ UPLC Analytik (Methode 1) Rt = 1.38 min, gefundene Masse ESI(+) 462.27.
$^1$H-NMR (300MHz, DMSO-$d_6$): δ [ppm]= 0.84 (d, 3H), 0.99 (s, 3H), 1.16 - 1.29 (m, 1H), 1.35 - 1.79 (m, 7H), 1.91 (d, 1H), 2.04 - 2.21 (m, 2H), 2.25 - 2.44 (m, 3H), 2.82 - 2.93 (m, 2H), 3.14 - 3.25 (m, 4H), 4.39 (t, 1H), 6.26 (br. s., 1H), 7.30 (d, 1H), 7.50 - 7.59 (m, 2H), 7.64 - 7.71 (dd, 1H), 8.24 (t, 1H), 8.41 - 8.52 (m, 2H).

**Beispiel 13**

**17-(5-Fluorpyridin-3-yl)-*N*-[1-(hydroxymethyl)cyclopentyl]estra-1,3,5(10),16-tetraen-3-carboxamid**

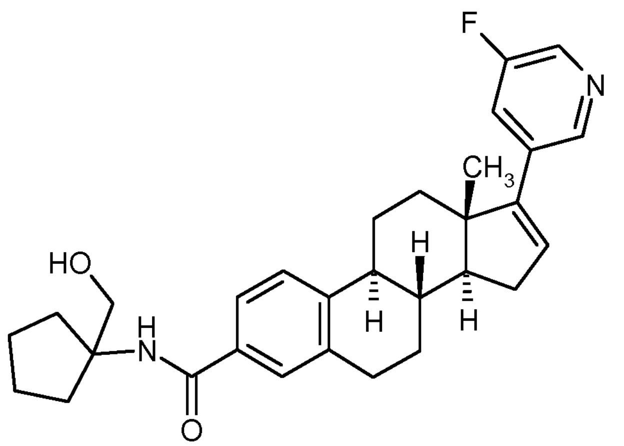

Analog zu Beispiel 8 wurden 100 mg 17-(5-Fluorpyridin-3-yl)estra-1,3,5(10),16-tetraen-3-carbonsäure mit 61 mg (1-Aminocyclopentyl)methanol zu 34 mg der Titelverbindung umgesetzt.
$C_{30}H_{35}FN_2O_2$ UPLC Analytik (Methode 1) Rt = 1.50 min, gefundene Masse ESI(+) 474.27.
$^1$H-NMR (300MHz, DMSO-$d_6$): δ [ppm]= 0.99 (s, 3H), 1.37 - 1.78 (m, 11H), 1.86 - 2.02 (m, 3H), 2.05 - 2.20 (m, 2H), 2.25

- 2.41 (m, 3H), 2.83 - 2.92 (m, 2H), 3.52 (d, 2H), 4.80 (t, 1H), 6.26 (br. s., 1H), 7.28 (d, 1H), 7.48 - 7.57 (d, 3H), 7.64 - 7.71 (m, 1H), 8.41 - 8.51 (m, 2H).

**Beispiel 14**

**[17-(5-Fluorpyridin-3-yl)estra-1,3,5(10),16-tetraen-3-yl](4-hydroxypiperidin-1-yl)keton**

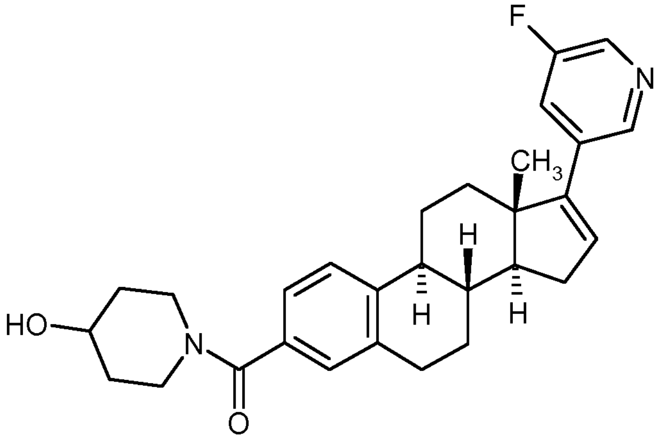

Analog zu Beispiel 8 wurden 100 mg 17-(5-Fluorpyridin-3-yl)estra-1,3,5(10),16-tetraen-3-carbonsäure mit 54 mg Piperidin-4-ol zu 74 mg der Titelverbindung umgesetzt.
UPLC Analytik (Methode 1) Rt = 1.33 min, gefundene Masse ESI(+) 460.25.
$^1$H-NMR (300MHz, DMSO-$d_6$): δ [ppm]= 1.00 (s, 3H), 1.18 - 1.97 (m, 10H), 2.03 - 2.43 (m, 5H), 2.79 - 2.92 (m, 2H), 3.03 - 3.19 (m, 2H), 3.53 (br. s., 1H), 3.62 - 3.75 (m, 1H), 3.94 (br. s., 1H), 4.72 (d, 1H), 6.26 (s, 1H), 6.99 - 7.13 (m, 2H), 7.29 (d, 1H), 7.63 - 7.71 (m, 1H), 8.39 - 8.53 (m, 2H).

**Beispiel 15**

**17-(5-Fluorpyridin-3-yl)-*N*-[(*S*)-1-(hydroxymethyl)propyl]estra-1,3,5(10),16-tetraen-3-carboxamid**

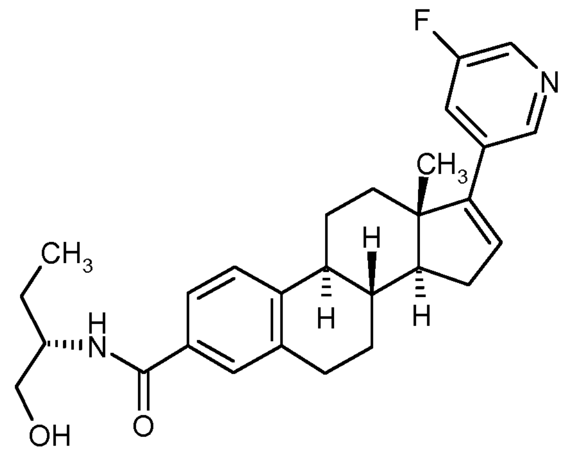

Analog zu Beispiel 8 wurden 100 mg 17-(5-Fluorpyridin-3-yl)estra-1,3,5(10),16-tetraen-3-carbonsäure mit 47 mg (2*S*)-2-Aminobutan-1-ol zu 74 mg der Titelverbindung umgesetzt.
$C_{28}H_{33}FN_2O_2$ UPLC Analytik (Methode 1) Rt = 1.38 min, gefundene Masse ESI(+) 448.25.
$^1$H-NMR (300MHz, DMSO-$d_6$): δ [ppm]= 0.82 (t, 3H), 0.99 (s, 3H), 1.31 - 1.80 (m, 7H), 1.85 - 1.97 (m, 1H), 2.05 - 2.43 (m, 5H), 2.82 - 2.94 (m, 2H), 3.31 - 3.46 (m, 1H), 3.74 - 3.89 (m, 1H), 4.59 (t, 1H), 6.27 (s, 1H), 7.30 (d, 1H), 7.53 - 7.61

(m, 2H), 7.64 - 7.71 (m, 1H), 7.81 (d, 1H), 8.39 - 8.53 (m, 2H).

**Beispiel 16**

**17-(5-Fluorpyridin-3-yl)-*N*-[(*R*)-1-(hydroxymethyl)-2-methylpropyl]estra-1,3,5(10),16-tetraen-3-carboxamid**

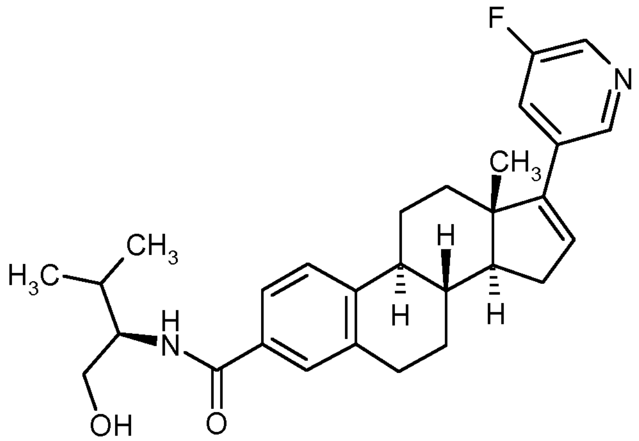

Analog zu Beispiel 8 wurden 100 mg 17-(5-Fluorpyridin-3-yl)estra-1,3,5(10),16-tetraen-3-carbonsäure mit 55 mg (2*R*)-2-Amino-3-methylbutan-1-ol zu 78 mg der Titelverbindung umgesetzt.
$C_{29}H_{35}FN_2O_2$ UPLC Analytik (Methode 1) Rt = 1.43 min, gefundene Masse ESI(+) 462.27.
$^1$H-NMR (300MHz, DMSO-$d_6$): δ [ppm]= 0.83 (d, 3H), 0.86 (d, 3H), 0.99 (s, 3H), 1.39 - 1.67 (m, 4H), 1.68 - 1.79 (m, 1H), 1.81 - 1.96 (m, 2H), 2.03 - 2.22 (m, 2H), 2.22 - 2.41 (m, 3H), 2.82 - 2.95 (m, 2H), 3.47 (t, 2H), 3.70 - 3.82 (m, 1H), 4.49 (t, 1H), 6.27 (br. s., 1H), 7.30 (d, 1H), 7.53 - 7.61 (m, 2H), 7.63 - 7.72 (m, 1H), 7.76 (d, 1H), 8.43 (d, 1H), 8.49 (s, 1H).

**Beispiel 17**

**[17-(5-Fluorpyridin-3-yl)estra-1,3,5(10),16-tetraen-3-yl][(*R*)-3-hydroxypiperidin-1-yl]keton**

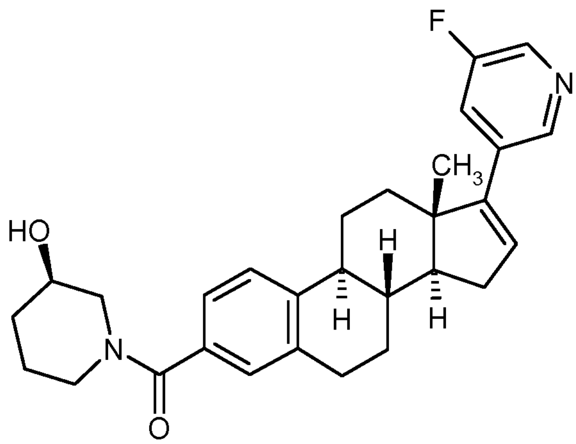

Analog zu Beispiel 8 wurden 100 mg 17-(5-Fluorpyridin-3-yl)estra-1,3,5(10),16-tetraen-3-carbonsäure mit 73 mg (3*R*)-Piperidin-3-ol zu 75 mg der Titelverbindung umgesetzt.
$C_{29}H_{33}FN_2O_2$ UPLC Analytik (Methode 1) Rt = 1.37 min, gefundene Masse ESI(+) 460.25.
$^1$H-NMR (300MHz, DMSO-$d_6$): δ [ppm]= 1.00 (s, 3H), 1.22 - 1.99 (m, 11H), 2.03 - 2.43 (m, 5H), 2.72 - 3.14 (4H, enthält breites Singulett bei 3.02 ppm), 3.44 (breites Singuelett, 2H), 3.83 (breites Singulett, 0.6H), 4.13 (breites Singulett, 0.4H), 4.70 - 4.96 (breites Signal, 0.9H), 6.26 (s., 1H), 7.01 - 7.13 (m, 2H), 7.29 (d, 1H), 7.63 - 7.72 (m, 1H), 8.39 - 8.53 (m, 2H).

**Beispiel 18**

**rel-17-(5-Fluorpyridin-3-yl)-*N*-[(1*R*,2*R*)-2-hydroxy-1-methylpropyl]estra-1,3,5(10),16-tetraen-3-carboxamid**

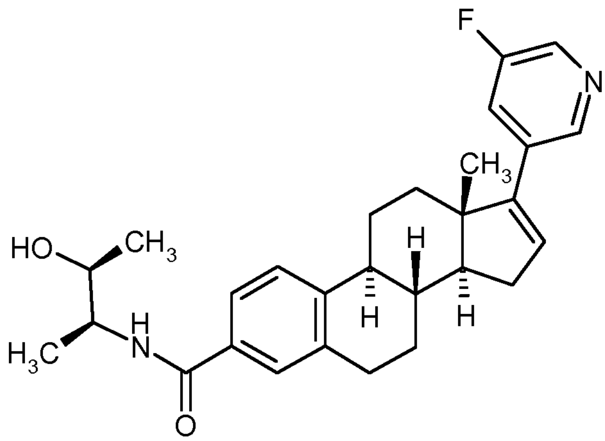

Analog zu Beispiel 8 wurden 100 mg 17-(5-Fluorpyridin-3-yl)estra-1,3,5(10),16-tetraen-3-carbonsäure mit 67 mg *rel*-(2*S*,3*S*)-2-Amino-1-methylpropan-1-ol Hydrochlorid (1:1) zu 80 mg der Titelverbindung umgesetzt.
$^{1}$H-NMR (300MHz, DMSO-$d_6$): δ [ppm]= 0.91 - 1.03 (m, 6H), 1.06 (d, 3H), 1.35 - 1.80 (m, 5H), 1.85 - 1.97 (m, 1H), 2.03 - 2.42 (m, 5H), 2.81 2.94 (m, 2H), 3.59 - 3.69 (m, 1H), 3.85 3.99 (m, 1H), 4.56 (d, 1H), 6.27 (br. s., 1H), 7.30 (d, 1H), 7.50 - 7.61 (m, 2H), 7.64 - 7.80 (m, 2H), 8.39 - 8.53 (m, 2H).

**Beispiel 19**

**17-(5-Fluorpyridin-3-yl)-*N*-(2-hydroxyethyl)-*N*-isopropylestra-1,3,5(10),16-tetraen-3-carboxamid**

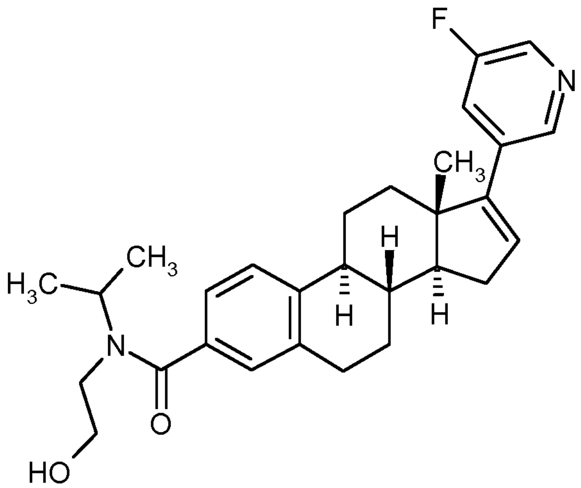

Analog zu Beispiel 8 wurden 100 mg 17-(5-Fluorpyridin-3-yl)estra-1,3,5(10),16-tetraen-3-carbonsäure mit 55 mg 2-(Isopropylamino)ethan-1-ol zu 27 mg der Titelverbindung umgesetzt.
$^{1}$H-NMR (500MHz, DMSO-d6): δ [ppm]= 1.01 - 1.14 (m, 12H), 1.41 - 1.52 (m, 1H), 1.54 - 1.71 (m, 4H), 1.77 (td, 1H), 1.90 - 1.98 (m, 1H), 2.10 - 2.24 (m, 2H), 2.30 - 2.39 (m, 2H), 2.40 - 2.46 (m, 1H), 2.83 - 2.95 (m, 2H), 3.54 (br. s., 2H), 3.87 (br. s., 1H), 4.72 (br. s., 1H), 6.30 (dd, 1H), 7.03 (s, 1H), 7.07 (d, 1H), 7.32 (d, 1H), 7.70 (dt, 1H), 8.46 (d, 1H), 8.52 (t, 1H).

**Beispiel 20**

**17-(5-Fluorpyridin-3-yl)-*N*-[(*RS*)-3,3,3-trifluor-2-hydroxypropyl]estra-1,3,5(10),16-tetraen-3-carboxamid**

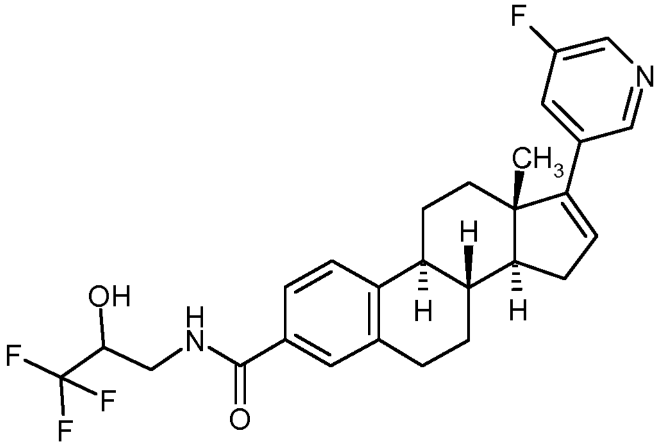

Analog zu Beispiel 8 wurden 250 mg 17-(5-Fluorpyridin-3-yl)estra-1,3,5(10),16-tetraen-3-carbonsäure mit 171 mg 2-Amino-1-(trifluormethyl)ethan-1-ol zu 161 mg der Titelverbindung umgesetzt. $C_{27}H_{28}F_4N_2O_2$ UPLC Analytik (Methode 1) Rt = 1.45 min, gefundene Masse ESI(+) 488.21.
$^1$H-NMR (300MHz, DMSO-$d_6$): δ [ppm]= 0.99 (s, 3H), 1.35 - 1.80 (m, 5H), 1.85 - 1.97 (m, 1H), 2.05 - 2.21 (m, 2H), 2.25 - 2.43 (m, 3H), 2.81 - 2.95 (m, 2H), 3.18 - 3.26 (m, 1H), 3.49 - 3.62 (m, 1H), 4.07 - 4.22 (m, 1H), 6.40 - 6.30 (m, 1H), 6.45 (d, 1H), 7.33 (d, 1H), 7.52 - 7.62 (m, 2H), 7.64 - 7.73 (m, 1H), 8.43 (d, 1H), 8.47 - 8.51 (m, 1H), 8.55 (t, 1H).

**Beispiel 21**

**17-(5-Fluorpyridin-3-yl)-*N*-[2-(1*H*-tetrazol-5-yl)ethyl]estra-1,3,5(10),16-tetraen-3-carboxamid**

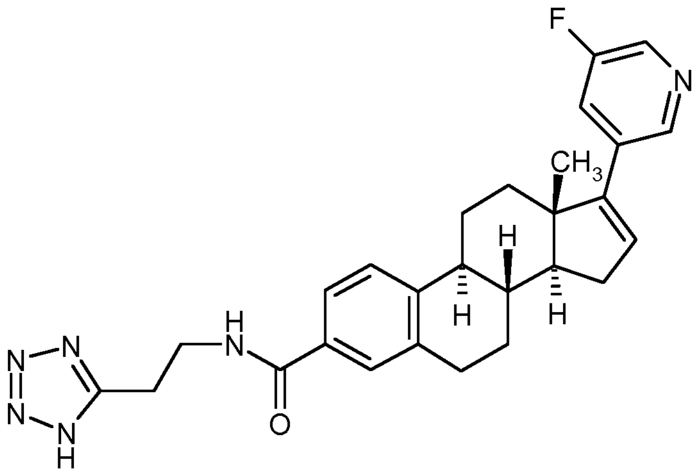

Analog zu Beispiel 8 wurden 100 mg 17-(5-Fluorpyridin-3-yl)estra-1,3,5(10),16-tetraen-3-carbonsäure mit 60 mg 2-(1*H*-Tetrazol-5-yl)ethan-1-amin zu 4 mg der Titelverbindung umgesetzt.
UPLC Analytik (Methode 1) Rt = 1.27 min, gefundene Masse ESI(+) 472.24.
$^1$H-NMR (300MHz, DMSO-$d_6$): δ [ppm]= 0.99 (m, 3H), 1.34 - 1.79 (m, 5H), 1.84 - 1.98 (m, 1H), 2.04 - 2.21 (m, 2H), 2.25 - 2.40 (m, 3H), 2.50 (s, 3H), 2.81 - 2.93 (m, 2H), 3.09 (t, 2H), 3.51 - 3.64 (m, 2H), 6.27 (s., 1H), 7.31 (d, 1H), 7.47 - 7.56 (m, 2H), 7.68 (dt, 1H), 8.39 - 8.54 (m, 3H).

**Beispiel 22**

**17-(5-Fluorpyridin-3-yl)-*N*-(1*H*-tetrazol-5-ylmethyl)estra-1,3,5(10),16-tetraen-3-carboxamid**

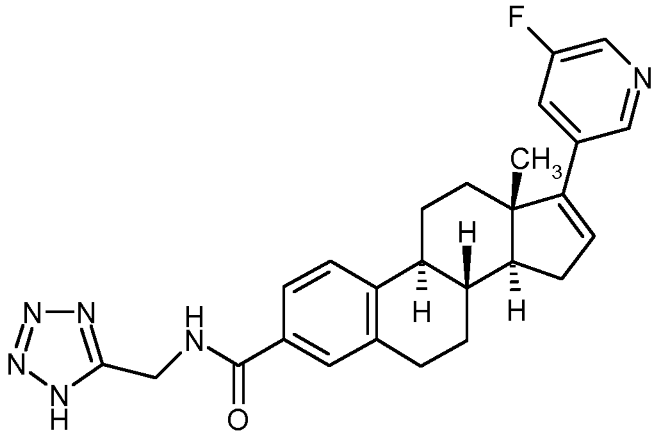

Analog zu Beispiel 8 wurden 100 mg 17-(5-Fluorpyridin-3-yl)estra-1,3,5(10),16-tetraen-3-carbonsäure mit 53 mg 1-(1*H*-Tetrazol-5-yl)methylamin zu 14 mg der Titelverbindung umgesetzt.
$C_{26}H_{27}FN_6O$ UPLC Analytik (Methode 1) Rt = 1.27 min, gefundene Masse ESI(+) 458.22.
$^1$H-NMR (400MHz, DMSO-$d_6$): δ [ppm]= 1.00 (s, 3H), 1.38 - 1.68 (m, 4H), 1.74 (td, 1H), 1.86 - 1.97 (m, 1H), 2.07 - 2.21 (m, 2H), 2.24 - 2.43 (m, 3H), 2.83 - 2.95 (m, 2H), 4.69 (d, 2H), 6.24 - 6.30 (m, 1H), 7.35 (d, 1H), 7.58 - 7.65 (m, 2H), 7.68 (dt, 1H), 8.43 (d, 1H), 8.49 (t, 1H), 9.05 (t, 1H).

**Beispiel 23**

**17-(3-Pyridyl)-*N*-(2-sulfamoylethyl)estra-1,3,5(10),16-tetraen-3-carboxamid**

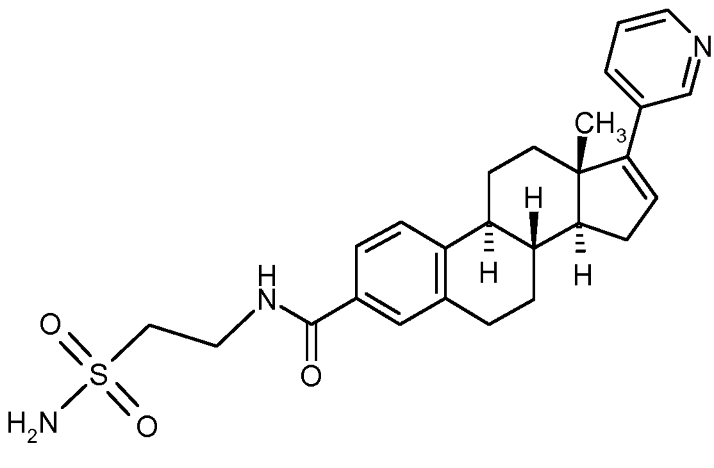

Analog zu Beispiel 8 wurden 100 mg 17-(3-Pyridyl)estra-1,3,5(10),16-tetraen-3-carbonsäure mit 89 mg 2-Aminoethan-1-sulfonamid Hydrochlorid (1:1) zu 84 mg der Titelverbindung umgesetzt.
UPLC Analytik (Methode 1) Rt = 0.93 min, gefundene Masse ESI(+) 465.21.
$^1$H-NMR (400MHz, DMSO-$d_6$): δ [ppm]= 0.99 (s, 3H), 1.37 - 1.67 (m, 4H), 1.73 (td, 1H), 1.86 - 1.96 (m, 1H), 2.05 - 2.17 (m, 2H), 2.24 - 2.44 (m, 3H), 2.82 - 2.96 (m, 2H), 3.14 - 3.23 (m, 2H), 3.54 - 3.66 (m, 2H), 6.12 (s, 1H), 6.91 (s, 2H), 7.29 - 7.36 (m, 2H), 7.51 - 7.59 (m, 2H), 7.77 (d, 1H), 8.39 - 8.45 (m, 1H), 8.48 (t, 1H), 8.58 - 8.61 (m, 1H).

**Beispiel 24**

***N*-(2-Sulfamoylethyl)-17-[5-(trifluormethyl)pyridin-3-yl]estra-1,3,5(10),16-tetraen-3-carboxamid**

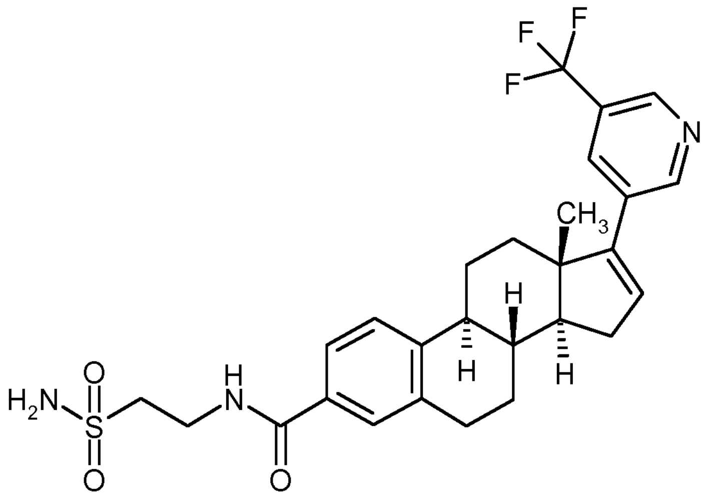

Analog zu Beispiel 8 wurden 70 mg 17-[5-(Trifluormethyl)pyridin-3-yl]estra-1,3,5(10),16-tetraen-3-carbonsäure mit 53 mg 2-Aminoethan-1-sulfonamid Hydrochlorid (1:1) zu 58 mg der Titelverbindung umgesetzt.
$C_{27}H_{30}F_3N_3O_3S$ UPLC Analytik (Methode 1) Rt = 1.39 min, gefundene Masse ESI(+) 533.20.
$^1$H-NMR (400MHz, DMSO-$d_6$): $\delta$ [ppm]= 1.01 (s, 3H), 1.38 - 1.67 (m, 4H), 1.76 (td, 1H), 1.86 - 1.97 (m, 1H), 2.07 - 2.20 (m, 2H), 2.27 - 2.43 (m, 3H), 2.82 - 2.95 (m, 2H), 3.19 (dd, 2H), 3.53 - 3.65 (m, 2H), 6.35 - 6.38 (m, 1H), 6.91 (s, 2H), 7.33 (d, 1H), 7.51 - 7.57 (m, 2H), 8.04 (s, 1H), 8.48 (t, 1H), 8.82 - 8.85 (m, 1H), 8.90 (d, 1H).

**Beispiel 25**

***N*-[2-(*N*-Methylsulfamoyl)ethyl]-17-[5-(trifluormethyl)pyridin-3-yl]estra-1,3,5(10),16-tetraen-3-carboxamid**

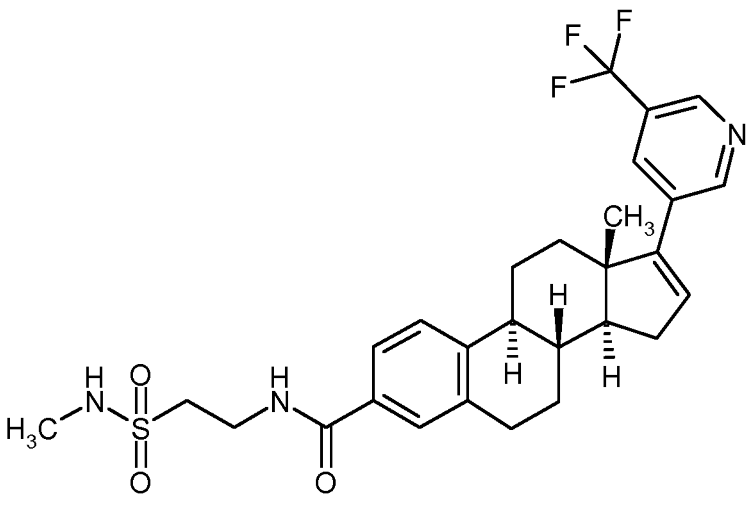

Analog zu Beispiel 8 wurden 70 mg 17-[5-(Trifluormethyl)pyridin-3-yl]estra-1,3,5(10),16-tetraen-3-carbonsäure mit 57 mg 2-Amino-*N*-methylethan-1-sulfonamid Hydrochlorid (1:1) zu 30 mg der Titelverbindung umgesetzt.
$C_{28}H_{32}F_3N_3O_3S$ UPLC Analytik (Methode 1) Rt = 1.45 min, gefundene Masse ESI(+) 547.21.
$^1$H-NMR (400MHz, DMSO-$d_6$): $\delta$ [ppm]= 1.01 (s, 3H), 1.38 - 1.68 (m, 4H), 1.76 (td, 1H), 1.88 - 1.96 (m, 1H), 2.08 - 2.20 (m, 2H), 2.27 - 2.39 (m, 2H), 2.39 - 2.44 (m), 2.55 (s, 3H), 2.84 - 2.96 (m, 2H), 3.21 (dd, 2H), 3.51 - 3.58 (m, 2H), 6.34

- 6.38 (m, 1H), 6.97 (s, 1H), 7.33 (d, 1H), 7.51 - 7.58 (m, 2H), 8.02 - 8.05 (m, 1H), 8.46 (t, 1H), 8.82 - 8.85 (m, 1H), 8.90 (d, 1H).

**Beispiel 26**

***N*-(3-Amino-3-oxopropyl)-17-(5-fluorpyridin-3-yl)estra-1,3,5(10),16-tetraen-3-carboxamid**

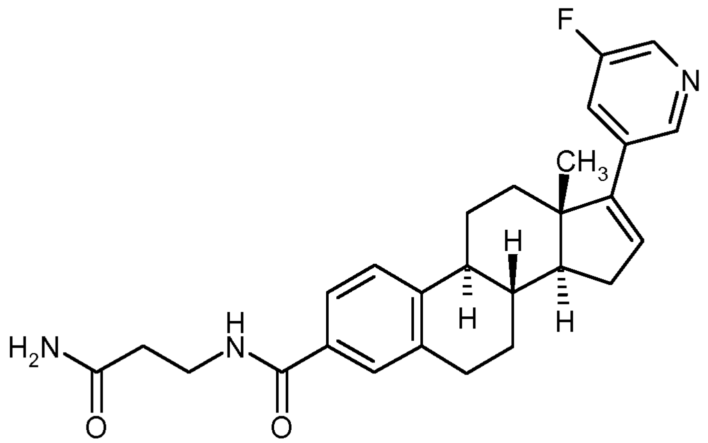

Analog zu Beispiel 8 wurden 100 mg 17-(5-Fluorpyridin-3-yl)estra-1,3,5(10),16-tetraen-3-carbonsäure mit 66 mg *β*-Alaninamid zu 87 mg der Titelverbindung umgesetzt.
$C_{27}H_{30}FN_3O_2$ UPLC Analytik (Methode 1) Rt = 1.22 min, gefundene Masse ESI(+) 447.23.
$^1$H-NMR (300MHz, DMSO-$d_6$): δ [ppm]= 0.99 (s, 3H), 1.34 - 1.80 (m, 5H), 1.84 - 1.96 (m, 1H), 2.06 - 2.44 (8H, enthält Triplet bei 2.30 ppm), 2.82 - 2.93 (m, 2H), 3.32 - 3.43 (m, 2H), 6.27 (s, 1H), 6.79 (br. s., 1H), 7.25 - 7.37 (m, 2H), 7.50 - 7.58 (m, 2H), 7.65 - 7.72 (m, 1H), 8.34 (t, 1H), 8.43 (d, 1H), 8.47 - 8.50 (m, 1H).

**Beispiel 27**

**17-(5-Fluorpyridin-3-yl)-*N*-[3-(methylamino)-3-oxopropyl]estra-1,3,5(10),16-tetraen-3-carboxamid**

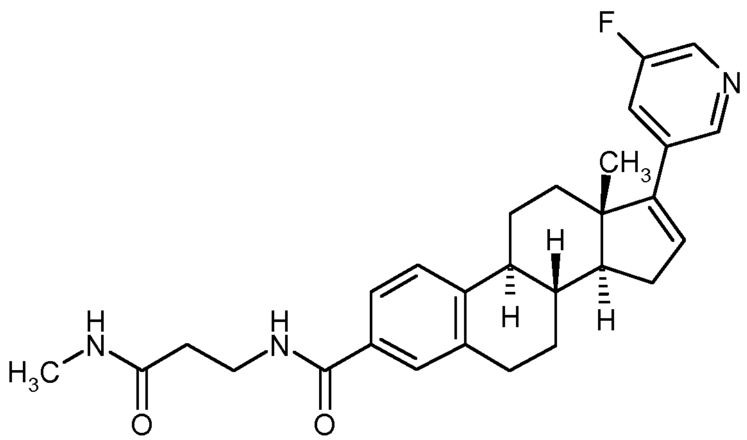

Analog zu Beispiel 8 wurden 100 mg 17-(5-Fluorpyridin-3-yl)estra-1,3,5(10),16-tetraen-3-carbonsäure mit 73 mg *N*-Methyl-*β*-alaninamid Hydrochlorid (1:1) zu 45 mg der Titelverbindung umgesetzt.
$C_{28}H_{32}FN_3O_2$ UPLC Analytik (Methode 1) Rt = 1.26 min, gefundene Masse ESI(+) 461.25.
$^1$H-NMR (300MHz, DMSO-$d_6$): δ [ppm]= 0.99 (s, 3H), 1.34 - 1.80 (m, 5H), 1.85 - 1.96 (m, 1H), 2.05 - 2.43 (m, 7H), 2.53 (d, 3H), 2.80 - 2.96 (m, 2H), 3.33 - 3.45 (m, 2H), 6.27 (s, 1H), 7.31 (d, 1H), 7.49 - 7.59 (m, 2H), 7.68 (dt, 1H), 7.73 - 7.83 (m, 1H), 8.36 (t, 1H), 8.43 (d, 1H), 8.46 - 8.52 (m, 1H).

**Beispiel 28**

**[17-(5-Fluorpyridin-3-yl)estra-1,3,5(10),16-tetraen-3-yl][(*S*)-2-(1*H*-tetrazol-5-yl)pyrrolidin-1-yl]keton**

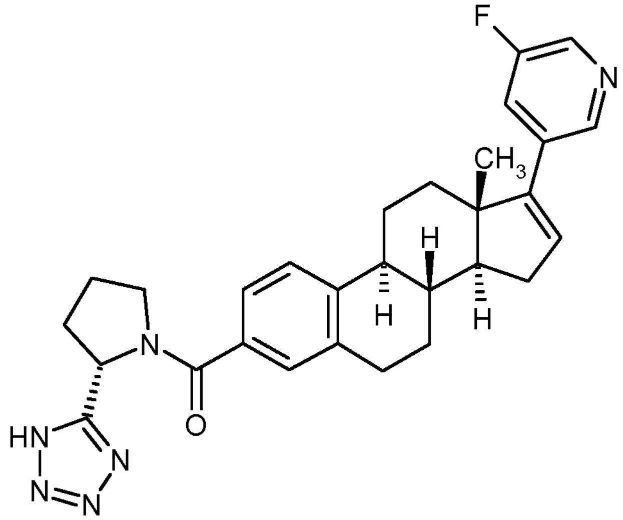

Analog zu Beispiel 8 wurden 100 mg 17-(5-Fluorpyridin-3-yl)estra-1,3,5(10),16-tetraen-3-carbonsäure mit 74 mg 5-[(2*S*)-Pyrrolidin-2-yl]-1*H*-tetrazol zu 83 mg der Titelverbindung umgesetzt.
UPLC Analytik (Methode 1) Rt = 1.35 min, gefundene Masse ESI(+) 498.25.
$^1$H-NMR (300MHz, DMSO-$d_6$): $\delta$ [ppm]= 1.00 (s, 3H), 1.35 - 2.42 (m), 2.78 - 2.96 (m, 2H), 3.44 - 3.59 (m, 1H), 3.62 - 3.75 (m, 1H), 5.19 (br. s., 0.1H), 5.32 - 5.49 (m, 0.9H), 6.27 (br. s., 1H), 6.68 - 6.92 (br. s., 0.2H), 7.01 - 7.20 (br. s., 0.2H), 7.20 - 7.43 (m, 2.6H), 7.63 - 7.75 (m, 1H), 8.40 - 8.55 (m, 2H).

**Beispiel 29**

**1-{[17-(5-Fluorpyridin-3-yl)estra-1,3,5(10),16-tetraen-3-yl]carbonyl}-*N*-methyl-L-prolinamid**

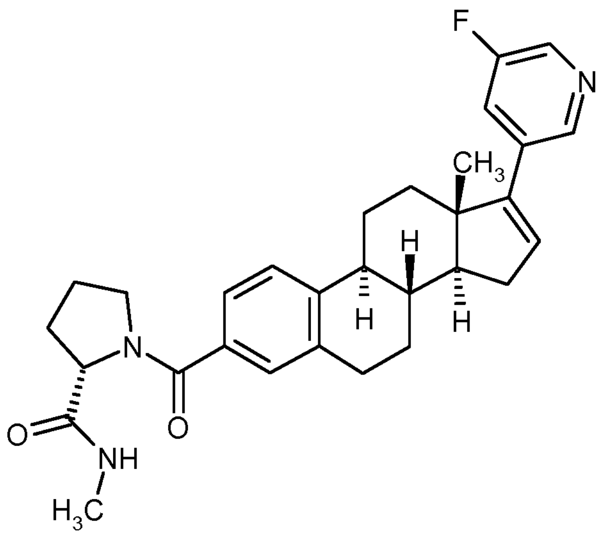

Analog zu Beispiel 8 wurden 100 mg 17-(5-Fluorpyridin-3-yl)estra-1,3,5(10),16-tetraen-3-carbonsäure mit 68 mg *N*-Methyl-L-prolinamid zu 85 mg der Titelverbindung umgesetzt.
UPLC Analytik (Methode 1) Rt = 1.32 min, gefundene Masse ESI(+) 487.26.

$^1$H-NMR (300MHz, DMSO-$d_6$): δ [ppm]= 1.00 (s, 3H), 1.32 - 2.42 (m, 15H), 2.58 (d, 3H), 2.75 - 2.95 (m, 2H), 3.32 - 3.62 (m), 4.00 - 4.08 (m), 4.29 - 4.39 (m), 6.23 - 6.32 (m, 1H), 6.96 - 7.06 (m, 0.5H), 7.21 - 7.36 (m, 2.5H), 7.64 - 7.82 (m, 2H), 8.43 (d, 1H), 8.47 - 8.51 (m, 1H).

**Beispiel 30**

**1-{[17-(5-Fluorpyridin-3-yl)estra-1,3,5(10),16-tetraen-3-yl]carbonyl}-L-prolinamid**

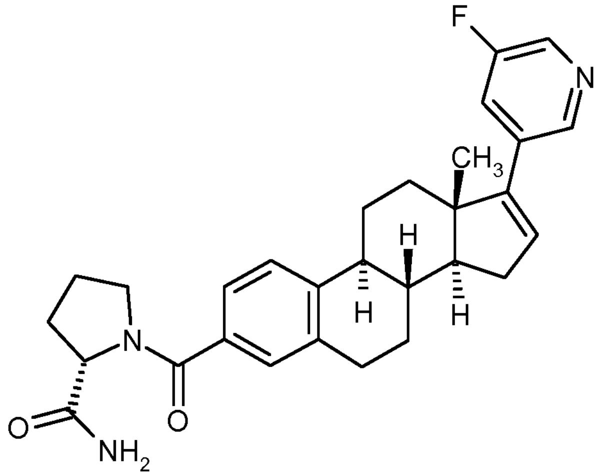

Analog zu Beispiel 8 wurden 100 mg 17-(5-Fluorpyridin-3-yl)estra-1,3,5(10),16-tetraen-3-carbonsäure mit 60 mg L-Prolinamid zu 92 mg der Titelverbindung umgesetzt.
UPLC Analytik (Methode 1) Rt = 1.29 min, gefundene Masse ESI(+) 473.25.
$^1$H-NMR (300MHz, DMSO-$d_6$): δ [ppm]= 1.00 (s, 3H), 1.33 - 1.96 (m, 9H), 2.00 - 2.42 (m), 2.77 - 2.94 (m, 2H), 3.31 - 3.42 (m), 3.43 - 3.62 (m), 4.07 - 4.16 (m, 0.3H), 4.26 - 4.36 (m, 0.7H), 6.27 (br. s., 1H), 6.85 - 7.00 (m, 1H), 7.02 - 7.12 (m, 0.5H), 7.20 - 7.40 (m, 3.5H), 7.68 (d, 1H), 8.43 (d, 1H), 8.49 (s, 1H).

**Beispiel 31**

**17-(5-Fluorpyridin-3-yl)-*N*-{2-methyl-2-[(methylsulfonyl)amino]propyl}estra-1,3,5(10),16-tetraen-3-carboxamid**

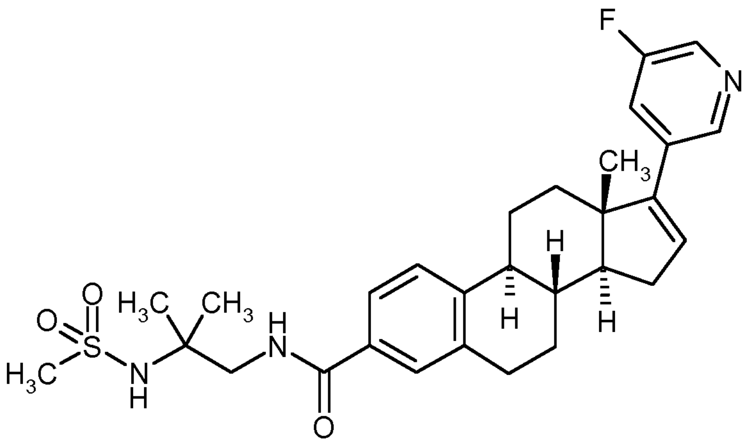

Analog zu Beispiel 8 wurden 100 mg 17-(5-Fluorpyridin-3-yl)estra-1,3,5(10),16-tetraen-3-carbonsäure mit 107 mg *N*-[1-(Aminomethyl)-1-methylethyl]methansulfonamid zu 95 mg der Titelverbindung umgesetzt.
$C_{29}H_{36}FN_3O_3S$ UPLC Analytik (Methode 1) Rt = 1.40 min, gefundene Masse ESI(+) 525.25.

$^1$H-NMR (300MHz, DMSO-$d_6$): δ [ppm]= 1.00 (s, 3H), 1.23 (s, 6H), 1.39 - 1.79 (m, 5H), 1.85 - 1.97 (m, 1H), 2.05 - 2.41 (m, 5H), 2.84 - 2.99 (5H, enthält Singulett bei 2.93 ppm), 3.34 (d, 2H), 6.27 (s, 1H), 6.93 (s, 1H), 7.34 (d, 1H), 7.53 - 7.61 (m, 2H), 7.68 (dt, 1H), 8.25 (t, 1H), 8.43 (d, 1H), 8.48 - 8.52 (m, 1H).

**Beispiel 32**

***N*-Ethyl-17-(5-fluorpyridin-3-yl)-*N*-(2-hydroxyethyl)estra-1,3,5(10),16-tetraen-3-carboxamid**

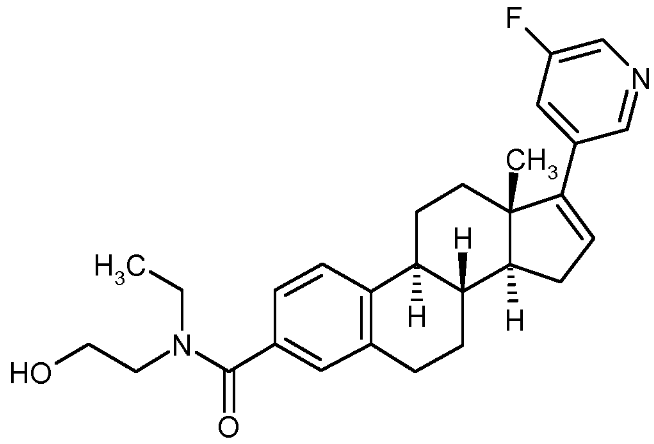

Analog zu Beispiel 8 wurden 100 mg 17-(5-Fluorpyridin-3-yl)estra-1,3,5(10),16-tetraen-3-carbonsäure mit 47 mg 2-(Ethylamino)ethan-1-ol zu 75 mg der Titelverbindung umgesetzt.
UPLC Analytik (Methode 1) Rt = 1.38 min, gefundene Masse ESI(+) 448.25.
$^1$H-NMR (400MHz, DMSO-$d_6$): δ [ppm]= 1.00 (s, 3H), 1.07 (br. s., 3H), 1.37 - 1.67 (m, 4H), 1.74 (td, 1H), 1.86 - 1.94 (m, 1H), 2.07 - 2.20 (m, 2H), 2.25 - 2.45 (m, 3H), 2.81 - 2.90 (m, 2H), 3.23 (br. s.), 3.42 (br. s.), 3.54 (br. s.), 4.71 (t, 1H), 6.24 - 6.29 (m, 1H), 7.00 - 7.11 (m, 2H), 7.28 (d, 1H), 7.65 - 7.71 (m, 1H), 8.43 (d, 1H), 8.49 (t, 1H).

**Beispiel 33**

***N*-[(*S*)-2,3-Dihydroxypropyl]-17-(5-fluorpyridin-3-yl)estra-1,3,5(10),16-tetraen-3-carboxamid**

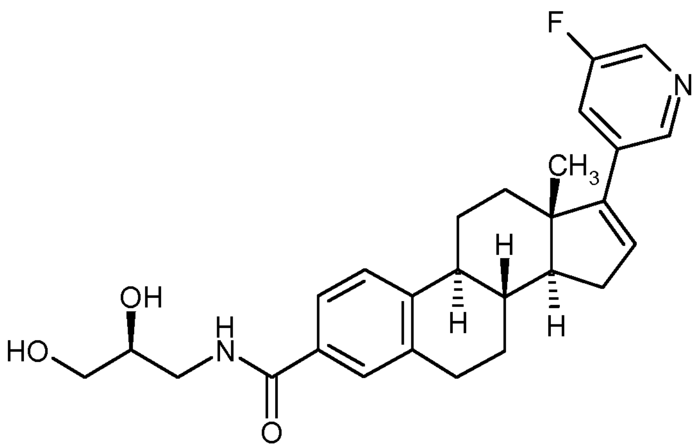

Analog zu Beispiel 8 wurden 100 mg 17-(5-Fluorpyridin-3-yl)estra-1,3,5(10),16-tetraen-3-carbonsäure mit 58 mg (2*S*)-3-Aminopropan-1,2-diol zu 80 mg der Titelverbindung umgesetzt.
UPLC Analytik (Methode 1) Rt = 1.22 min, gefundene Masse ESI(+) 450.23.

$^1H$-NMR (300MHz, DMSO-$d_6$): δ [ppm]= 0.99 (s, 3H), 1.34 - 1.79 (m, 5H), 1.85 - 1.96 (m, 1H), 2.05 - 2.44 (m, 5H), 2.82 - 2.97 (m, 2H), 3.09 - 3.23 (m, 1H), 3.23 - 3.40 (m, überlagert von Wassersignal), 3.53 - 3.63 (m, 1H), 4.53 (t, 1H), 4.76 (d, 1H), 6.27 (br. s., 1H), 7.31 (d, 1H), 7.53 - 7.62 (m, 2H), 7.68 (dt, 1H), 8.24 (t, 1H), 8.42 - 8.52 (m, 2H).

**Beispiel 34**

**17-(5-Fluorpyridin-3-yl)-*N*-(3-hydroxypropyl)-*N*-methylestra-1,3,5(10),16-tetraen-3-carboxamid**

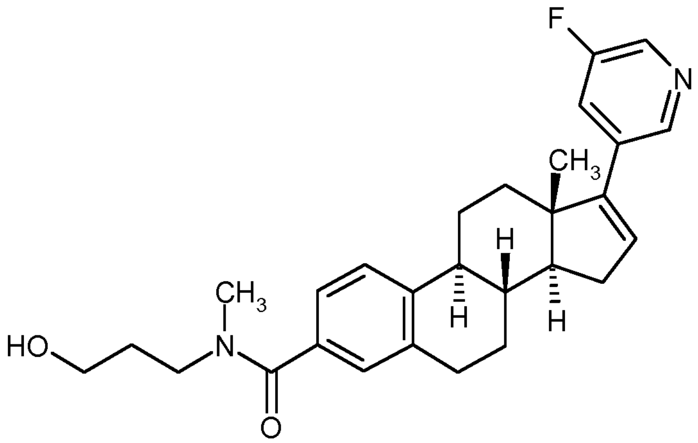

Analog zu Beispiel 8 wurden 100 mg 17-(5-Fluorpyridin-3-yl)estra-1,3,5(10),16-tetraen-3-carbonsäure mit 47 mg 3-(Methylamino)propan-1-ol zu 83 mg der Titelverbindung umgesetzt.
UPLC Analytik (Methode 1) Rt = 1.34 min, gefundene Masse ESI(+) 448.25.
$^1H$-NMR (400MHz, DMSO-$d_6$): δ [ppm]= 1.00 (s, 3H), 1.37 - 1.51 (m, 1H), 1.52 - 1.78 (m, 6H), 1.86 - 1.94 (m, 1H), 2.07 - 2.20 (m, 2H), 2.25 - 2.45 (m, 3H), 2.80 - 2.93 (m, 5H), 3.42 (br. s.), 4.42 (br. s., 1H), 6.27 (dd, 1H), 7.01 - 7.12 (m, 2H), 7.28 (d, 1H), 7.65 - 7.70 (m, 1H), 8.43 (d, 1H), 8.49 (t, 1H).

**Beispiel 35**

***N*-[(*RS*)-2,3-Dihydroxypropyl]-17-(5-fluorpyridin-3-yl)-*N*-methylestra-1,3,5(10),16-tetraen-3-carboxamid**

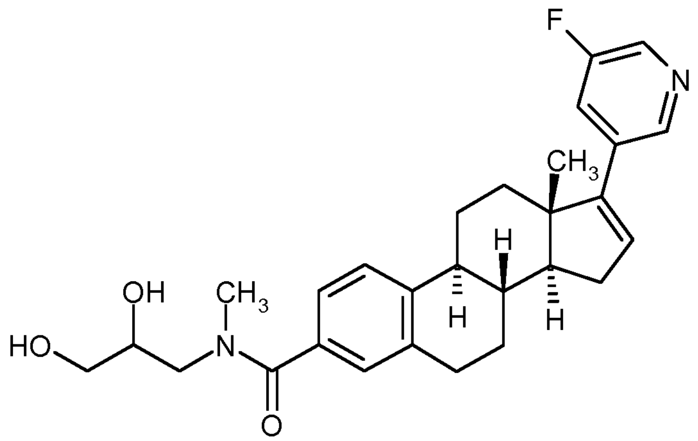

Analog zu Beispiel 8 wurden 100 mg 17-(5-Fluorpyridin-3-yl)estra-1,3,5(10),16-tetraen-3-carbonsäure mit 56 mg 3-(Methylamino)propan-1,2-diol zu 68 mg der Titelverbindung umgesetzt.
UPLC Analytik (Methode 1) Rt = 1.25 min, gefundene Masse ESI(+) 464.25.
$^1H$-NMR (400MHz, DMSO-$d_6$): δ [ppm]= 1.00 (s, 3H), 1.37 - 1.67 (m, 4H), 1.74 (td, 1H), 1.86 - 1.94 (m, 1H), 2.07 - 2.20

(m, 2H), 2.25 - 2.45 (m, 3H), 2.85 (br. s., 2H), 2.94 (s, 3H), 3.12 (br. s.), 3.23 (br. s.), 3.32 (br. s.), 3.51 (br. s.), 3.66 (br. s.), 3.75 (br. s.), 4.52 (br. s., 1H), 4.80 (br. s.), 4.86 (br. s.), 6.27 (br. s., 1H), 7.06 - 7.17 (m, 2H), 7.28 (br. s., 1H), 7.68 (dt, 1H), 8.43 (d, 1H), 8.47 - 8.51 (m, 1H).

**Beispiel 36**

***N*-[(*R*)-2,3-Dihydroxypropyl]-17-(5-fluorpyridin-3-yl)estra-1,3,5(10),16-tetraen-3-carboxamid**

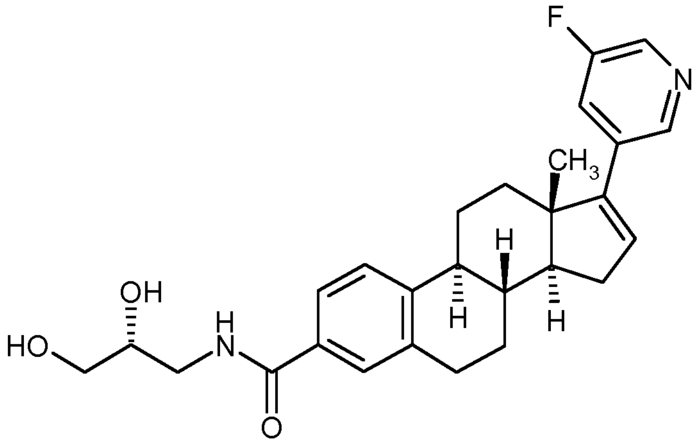

Analog zu Beispiel 8 wurden 100 mg 17-(5-Fluorpyridin-3-yl)estra-1,3,5(10),16-tetraen-3-carbonsäure mit 48 mg (2*R*)-3-Aminopropan-1,2-diol zu 65 mg der Titelverbindung umgesetzt.
UPLC Analytik (Methode 1) Rt = 1.22 min, gefundene Masse ESI(+) 450.23.
$^1$H-NMR (400MHz, DMSO-$d_6$): δ [ppm]= 1.00 (s, 3H), 1.38 - 1.66 (m, 4H), 1.74 (td, 1H), 1.87 - 1.95 (m, 1H), 2.08 - 2.19 (m, 2H), 2.26 - 2.45 (m, 3H), 2.84 - 2.93 (m, 2H), 3.12 - 3.20 (m, 1H), 3.25 - 3.37 (m, 3H), 3.55 - 3.62 (m, 1H), 4.51 (t, 1H), 4.75 (d, 1H), 6.27 (dd, 1H), 7.32 (d, 1H), 7.53 - 7.59 (m, 2H), 7.65 - 7.70 (m, 1H), 8.22 (t, 1H), 8.43 (d, 1H), 8.49 (t, 1H).

**Beispiel 37**

**17-(5-Fluorpyridin-3-yl)-*N*-[2-(methylsulfinyl)ethyl]estra-1,3,5(10),16-tetraen-3-carboxamid**

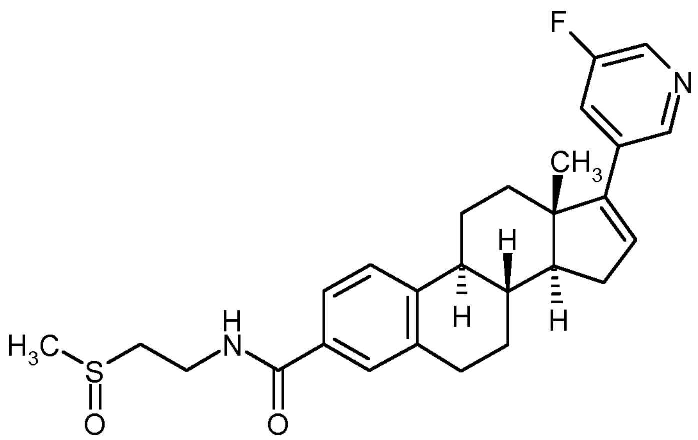

Analog zu Beispiel 8 wurden 100 mg 17-(5-Fluorpyridin-3-yl)estra-1,3,5(10),16-tetraen-3-carbonsäure mit 57 mg 2-(Methylsulfinyl)ethan-1-amin zu 85 mg der Titelverbindung umgesetzt.
UPLC Analytik (Methode 1) Rt = 1.24 min, gefundene Masse ESI(+) 466.21.
$^1$H-NMR (300MHz, DMSO-$d_6$): δ [ppm]= 1.00 (s, 3H), 1.35 - 1.80 (m, 5H), 1.86 - 1.96 (m, 1H), 2.06 - 2.41 (m, 6H), 2.55

(s, 3H), 2.78 - 2.92 (m, 3H), 2.92 - 3.06 (m, 1H), 3.47 - 3.67 (m, 2H), 6.24 - 6.30 (m, 1H), 7.33 (d, 1H), 7.52 - 7.59 (m, 2H), 7.64 - 7.71 (m, 1H), 8.41 - 8.51 (m, 2H), 8.59 (t, 1H).

**Beispiel 38**

**17-(5-Fluorpyridin-3-yl)-*N*-[(*R*)-2-hydroxypropyl]estra-1,3,5(10),16-tetraen-3-carboxamid**

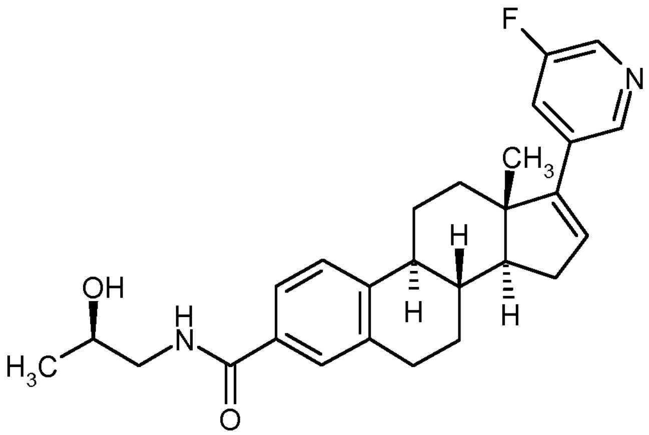

Analog zu Beispiel 8 wurden 100 mg 17-(5-Fluorpyridin-3-yl)estra-1,3,5(10),16-tetraen-3-carbonsäure mit 40 mg (2*R*)-1-Aminopropan-2-ol zu 75 mg der Titelverbindung umgesetzt.
UPLC Analytik (Methode 1) Rt = 1.32 min, gefundene Masse ESI(+) 434.24.
$^1$H-NMR (300MHz, DMSO-$d_6$): δ [ppm]= 0.95 - 1.07 (m, 6H), 1.35 - 1.81 (m, 5H), 1.86 - .96 (m, 1H), 2.06 - 2.44 (m, 5H), 2.50 (br. s., 1H), 2.83 - 2.93 (m, 2H), 3.15 (t, 2H), 3.68 - 3.79 (m, 1H), 4.67 (d, 1H), 6.23 - 6.29 (m, 1H), 7.31 (d, 1H), 7.52 - 7.60 (m, 2H), 7.67 (dt, 1H), 8.21 (t, 1H), 8.41 - 8.51 (m, 2H).

**Beispiel 39**

***N*-Ethyl-17-(5-fluorpyridin-3-yl)estra-1,3,5(10),16-tetraen-3-carboxamid**

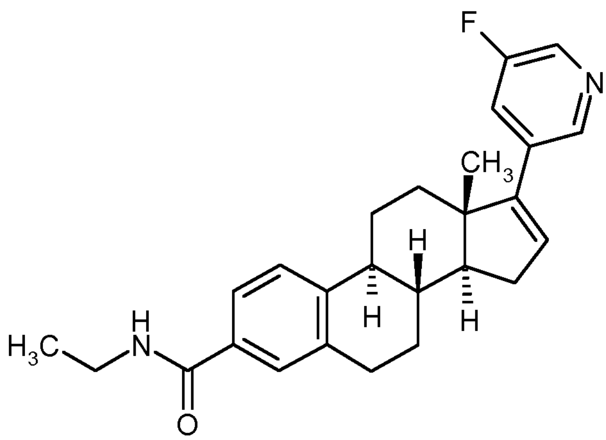

Analog zu Beispiel 8 wurden 100 mg 17-(5-Fluorpyridin-3-yl)estra-1,3,5(10),16-tetraen-3-carbonsäure mit 43 mg Ethan-1-amin Hydrochlorid(1:1) zu 75 mg der Titelverbindung umgesetzt.
UPLC Analytik (Methode 1) Rt = 1.44 min, gefundene Masse ESI(+) 404.23.
$^1$H-NMR (300MHz, DMSO-$d_6$): δ [ppm]= 0.99 (s, 3H), 1.07 (t, 3H), 1.35 - 1.79 (m, 5H), 1.86 - 1.96 (m, 1H), 2.06 - 2.44 (m, 5H), 2.82 - 2.93 (m, 2H), 3.17 - 3.26 (m), 6.23 - 6.29 (m, 1H), 7.30 (d, 1H), 7.50 - 7.58 (m, 2H), 7.64 - 7.71 (m, 1H), 8.28 (t, 1H), 8.41 - 8.51 (m, 2H).

**Beispiel 40**

**17-(5-Fluorpyridin-3-yl)-*N*-[(*S*)-1-(hydroxymethyl)ethyl]estra-1,3,5(10),16-tetraen-3-carboxamid**

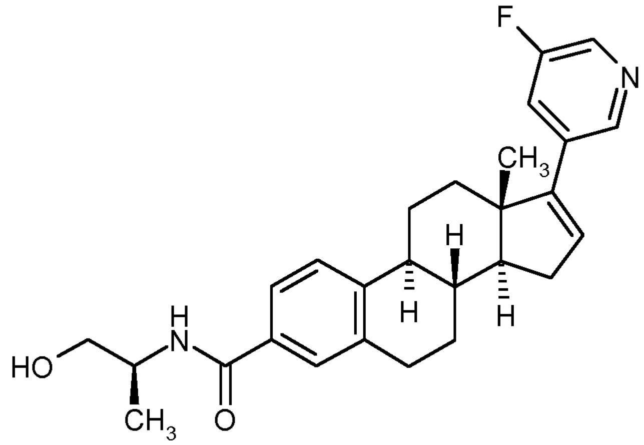

Analog zu Beispiel 8 wurden 100 mg 17-(5-Fluorpyridin-3-yl)estra-1,3,5(10),16-tetraen-3-carbonsäure mit 40 mg (2*S*)-2-Aminopropan-1-ol zu 79 mg der Titelverbindung umgesetzt.
UPLC Analytik (Methode 1) Rt = 1.32 min, gefundene Masse ESI(+) 434.24.
$^1$H-NMR (300MHz, DMSO-$d_6$): δ [ppm]= 1.00 (s, 3H), 1.08 (d, 3H), 1.35 - 1.67 (m, 4H), 1.74 (td, 1H), 1.86 - 1.96 (m, 1H), 2.06 - 2.20 (m, 2H), 2.25 - 2.44 (m, 3H), 2.83 - 2.93 (m, 2H), 3.23 - 3.33 (m, zum Teil verdeckt durch Wassersignal), 3.37 - 3.46 (m, 1H), 3.88 - 4.04 (m, 1H), 4.64 (t, 1H), 6.22 - 6.30 (m, 1H), 7.30 (d, 1H), 7.51 - 7.60 (m, 2H), 7.67 (dt, 1H), 7.90 (d, 1H), 8.40 - 8.51 (m, 2H).

**Beispiel 41**

**17-(5-Fluorpyridin-3-yl)-*N*-(2-methoxyethyl)estra-1,3,5(10),16-tetraen-3-carboxamid**

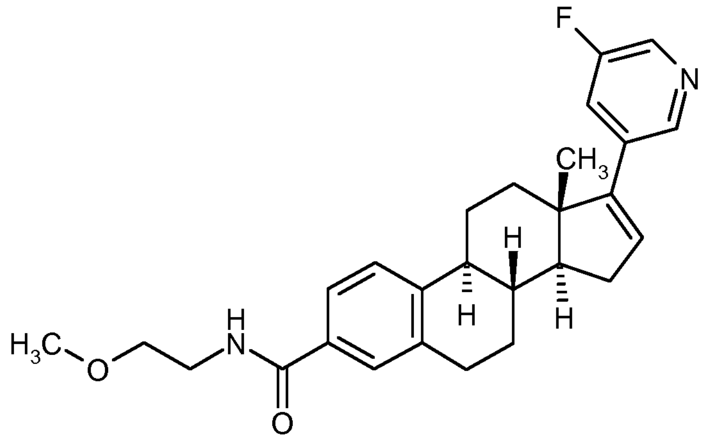

Analog zu Beispiel 8 wurden 100 mg 17-(5-Fluorpyridin-3-yl)estra-1,3,5(10),16-tetraen-3-carbonsäure mit 40 mg 2-Methoxyethan-1-amin zu 77 mg der Titelverbindung umgesetzt.
UPLC Analytik (Methode 1) Rt = 1.41 min, gefundene Masse ESI(+) 434.24.
$^1$H-NMR (300MHz, DMSO-$d_6$): δ [ppm]= 0.99 (s, 3H), 1.35 - 1.79 (m, 5H), 1.86 - 1.96 (m, 1H), 2.06 - 2.44 (m, 5H), 2.83 - 2.93 (m, 2H), 3.23 (s, 3H), 3.32 - 3.46 (m, 4H), 6.23 - 6.29 (m, 1H), 7.31 (d, 1H), 7.52 - 7.60 (m, 2H), 7.64 - 7.71 (m, 1H), 8.29 - 8.37 (m, 1H), 8.40 - 8.51 (m, 2H).

**Beispiel 42**

**17-(5-Fluorpyridin-3-yl)-*N*-[2-(isopropylsulfonyl)ethyl]estra-1,3,5(10),16-tetraen-3-carboxamid**

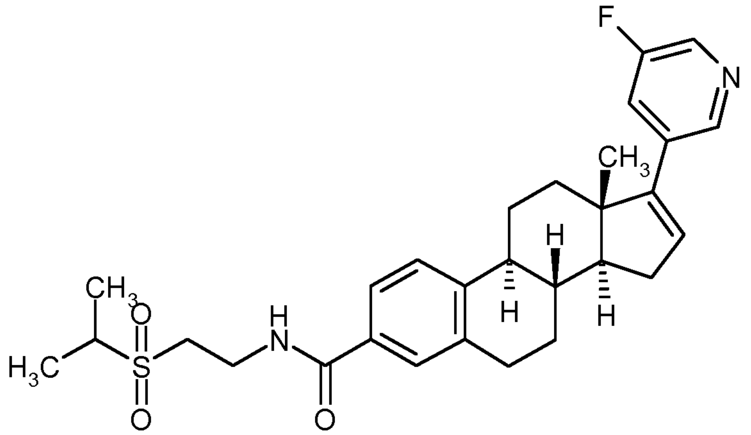

Analog zu Beispiel 8 wurden 100 mg 17-(5-Fluorpyridin-3-yl)estra-1,3,5(10),16-tetraen-3-carbonsäure mit 80 mg 2-(Isopropylsulfonyl)ethan-1-amin zu 71 mg der Titelverbindung umgesetzt.
$C_{29}H_{35}FN_2O_3S$ UPLC Analytik (Methode 1) Rt = 1.40 min, gefundene Masse ESI(+) 510.24.
$^1$H-NMR (300MHz, DMSO-$d_6$): $\delta$ [ppm]= 1.00 (s, 3H), 1.21 (d, 6H), 1.36 - 1.79 (m, 5H), 1.86 - 1.96 (m, 1H), 2.06 - 2.44 (m, 5H), 2.83 - 2.93 (m, 2H), 3.21 - 3.35 (m, teilweise verdeckt durch Wassersignal), 3.61 (q, 2H), 6.22 - 6.30 (m, 1H), 7.33 (d, 1H), 7.51 - 7.58 (m, 2H), 7.67 (dt, 1H), 8.39 -8.51 (m, 2H), 8.55 (t, 1H).

**Beispiel 43**

**17-(5-Fluorpyridin-3-yl)-*N*-[(3-methyloxetan-3-yl)methyl]estra-1,3,5(10),16-tetraen-3-carboxamid**

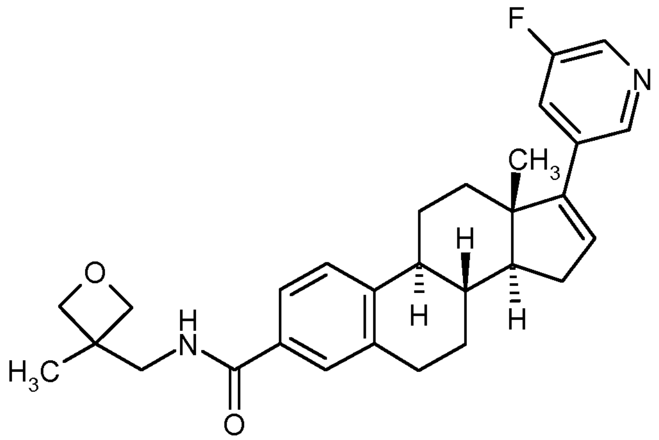

Analog zu Beispiel 8 wurden 100 mg 17-(5-Fluorpyridin-3-yl)estra-1,3,5(10),16-tetraen-3-carbonsäure mit 54 mg 1-(3-Methyloxetan-3-yl)methylamin zu 69 mg der Titelverbindung umgesetzt.
UPLC Analytik (Methode 1) Rt = 1.40 min, gefundene Masse ESI(+) 460.25.
$^1$H-NMR (300MHz, DMSO-$d_6$): $\delta$ [ppm]= 1.00 (s, 3H), 1.21 (s, 3H), 1.35 - 1.80 (m, 5H), 1.85 - 1.97 (m, 1H), 2.06 - 2.41 (m, 5H), 2.83 - 2.95 (m, 2H), 3.40 (d, 2H), 4.15 (d, 2H), 4.44 (d, 2H), 6.27 (br. s., 1H), 7.33 (d, 1H), 7.52 - 7.71 (m, 3H), 8.40 - 8.52 (m, 3H).

**Beispiel 44**

**17-(5-Fluorpyridin-3-yl)-*N*-(2-hydroxyethyl)-*N*-methylestra-1,3,5(10),16-tetraen-3-carboxamid**

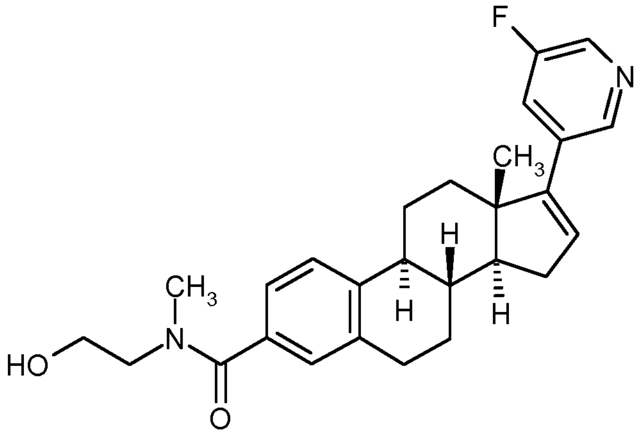

Analog zu Beispiel 8 wurden 100 mg 17-(5-Fluorpyridin-3-yl)estra-1,3,5(10),16-tetraen-3-carbonsäure mit 40 mg 2-(Methylamino)ethan-1-ol zu 55 mg der Titelverbindung umgesetzt.
UPLC Analytik (Methode 1) Rt = 1.31 min, gefundene Masse ESI(+) 434.24.
$^1$H-NMR (300MHz, DMSO-$d_6$): δ [ppm]= 1.00 (s, 3H), 1.34 - 1.80 (m, 5H), 1.84 - 1.95 (m, 1H), 2.05 - 2.42 (m, 5H), 2.79 - 2.97 (5H, enthält s bei 2.92 ppm), 3.45 (br. s.), 3.55 (br. s.), 4.71 (t, 1H), 6.21 - 6.31 (m, 1H), 7.03 - 7.15 (m, 2H), 7.28 (d, 1H), 7.68 (dt, 1H), 8.40 - 8.51 (m, 2H).

**Beispiel 45**

**17-(5-Fluorpyridin-3-yl)-*N*-[(*S*)-2-hydroxypropyl]estra-1,3,5(10),16-tetraen-3-carboxamid**

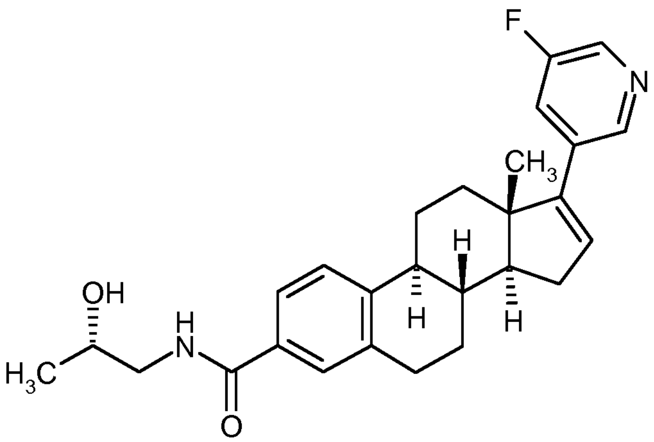

Analog zu Beispiel 8 wurden 100 mg 17-(5-Fluorpyridin-3-yl)estra-1,3,5(10),16-tetraen-3-carbonsäure mit 40 mg (*S*)-1-(Aminomethyl)ethan-1-ol zu 92 mg der Titelverbindung umgesetzt.
$C_{27}H_{31}FN_2O_2$ UPLC Analytik (Methode 1) Rt = 1.32 min, gefundene Masse ESI(+) 434.24.
$^1$H-NMR (300MHz, DMSO-$d_6$): δ [ppm]= 0.95 - 1.07 (m, 6H), 1.35 - 1.79 (m, 5H), 1.86 - 1.96 (m, 1H), 2.06 - 2.41 (m, 5H), 2.83 - 2.93 (m, 2H), 3.15 (t, 2H), 3.67 - 3.79 (m, 1H), 4.67 (d, 1H), 6.23 - 6.29 (m, 1H), 7.31 (d, 1H), 7.52 - 7.60 (m, 2H), 7.64 - 7.71 (m, 1H), 8.21 (t, 1H), 8.43 (d, 1H), 8.46 - 8.51 (m, 1H).

**Beispiel 46**

**17-(5-Fluorpyridin-3-yl)-*N*-[(*S*)-2-hydroxypropyl]estra-1,3,5(10),16-tetraen-3-carboxamid**

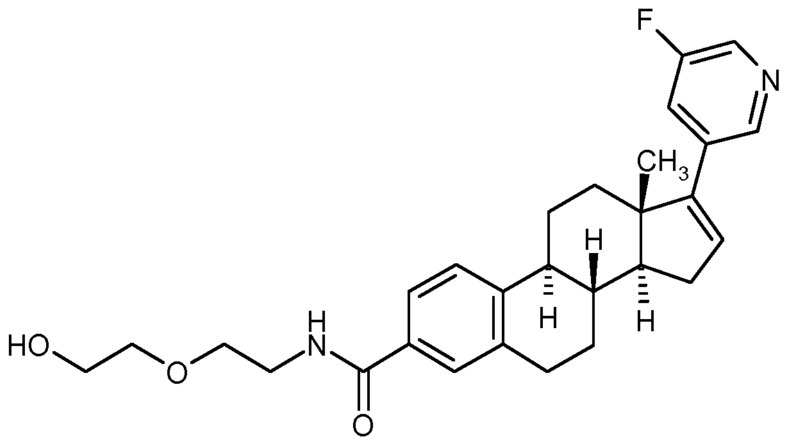

Analog zu Beispiel 8 wurden 100 mg 17-(5-Fluorpyridin-3-yl)estra-1,3,5(10),16-tetraen-3-carbonsäure mit 56 mg 2-(2-Aminoethoxy)ethan-1-ol zu 64 mg der Titelverbindung umgesetzt. $C_{28}H_{33}FN_2O_3$
UPLC Analytik (Methode 1) Rt = 1.28 min, gefundene Masse ESI(+) 464.25.
$^1$H-NMR (400MHz, DMSO-$d_6$): δ [ppm]= 1.00 (s, 3H), 1.37 - 1.66 (m, 4H), 1.74 (td, 1H), 1.87 - 1.95 (m, 1H), 2.08 - 2.19 (m, 2H), 2.26 - 2.45 (m, 3H), 2.84 - 2.92 (m, 2H), 3.29 - 3.52 (m, 8H), 4.54 (t, 1H), 6.25 - 6.28 (m, 1H), 7.31 (d, 1H), 7.52 - 7.59 (m, 2H), 7.65 - 7.70 (m, 1H), 8.32 (t, 1H), 8.43 (d, 1H), 8.49 (t, 1H).

**Beispiel 47**

**17-(Pyrimidin-5-yl)-*N*-(2-sulfamoylethyl)estra-1,3,5(10),16-tetraen-3-carboxamid**

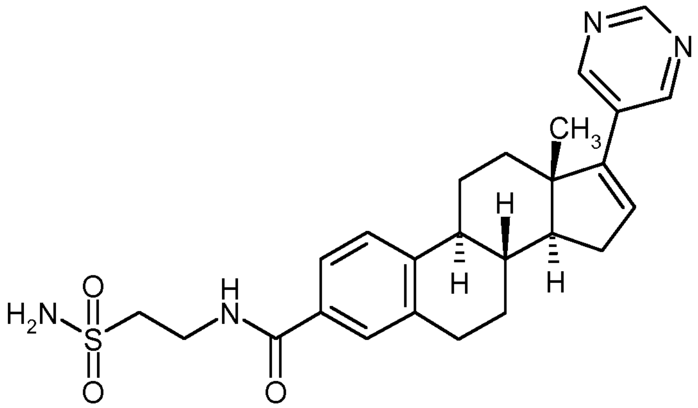

Analog zu Beispiel 8 wurden 100 mg 17-(Pyrimidin-5-yl)estra-1,3,5(10),16-tetraen-3-carbonsäure mit 89 mg 2-Aminoethan-1-sulfonamid Hydrochlorid (1:1) zu 47 mg der Titelverbindung umgesetzt. Die Titelverbindung wurde durch präparative HPLC aufgereinigt.

| | |
|---|---|
| *System:* | Waters Autopurificationsystem: Pump 254, Sample Manager 2767, CFO, DAD 2996, ELSD 2424, SQD 3100 |
| *Säule:* | XBrigde C18 5µm 100x30 mm |
| *Solvent:* | A = H2O + 0.2% Vol. NH3 (32%) |

(fortgesetzt)

| | | |
|---|---|---|
| | B = ACN | |
| *Gradient:* | 0-8 min 15-60% B | |
| *Fluss:* | 50 mL/min | |
| *Temperatur:* | Raumtemp. | |
| *Lösung:* | 126mg / 2.5 mL DMSO | |
| *Injektion:* | 5 x 0.5 mL | |
| *Detektion:* | DAD scan range 210-400 nm | |
| | MS ESI+, ESI-, scan range 160-1000 m/z | |
| | ELSD | |
| Fraktionen | Rt in min | Menge in mg |
| | 7.1 - 7.3 | 47 |
| *Aufarbeitung:* | Die Fraktionen wurden eingedampft, mit tBuOH versetzt, bei -65°C tiefgekühlt und abschließend gefriergetrocknet. | |

$^1$H-NMR (300MHz, DMSO-$d_6$): $\delta$ [ppm]= 0.99 (s, 3H), 1.34 - 1.80 (m, 5H), 1.85 - 1.97 (m, 1H), 2.05 - 2.41 (m, 6H), 2.83 - 2.93 (m, 2H), 3.14 - 3.23 (m, 2H), 3.54 - 3.64 (m, 2H), 6.31 (s, 1H), 6.90 (s, 2H), 7.33 (d, 1H), 7.50 - 7.60 (m, 2H), 8.47 (t, 1H), 8.83 (s, 2H), 9.04 (s, 1H).

**Pharmakologische Untersuchung der erfindungsgemäßen Verbindungen *in vitro***

**Beispiel 48 (AKR1C3-inhibitorische Wirkung)**

Die AKR1C3-inhibitorische Wirkung der Substanzen dieser Erfindung wurde in dem in den folgenden Absätzen beschriebenen AKR1C3-Assay gemessen.

Im Wesentlichen wird die Enzymaktivität durch Quantifizierung des gebildeten Coumberols aus Coumberon gemessen (Halim, M., Yee, D.J., and Sames, D., J. AM. CHEM. SOC. 130, 14123-14128 (2008) und Yee, D.J., Balsanek, V., Bauman, D.R., Penning, T.M., and Sames, D., Proc. Natl. Acad. Sci. USA 103, 13304 - 13309 (2006)). Bei diesem Versuch kann die Zunahme des stark fluoreszierenden Coumberols durch NADPH (Nicotinamid-Adenin-Dinukleotidphosphat)-abhängige Reduktion des nicht fluoreszierenden Coumberons durch AKR1 C3 bestimmt werden.

Als Enzym wurde rekombinantes humanes AKR1C3 (Aldo-keto reductase family 1 member C3) (GenBank Accession No. NM_003739) verwendet. Dieses wurde als GST (Glutathion-S-Transferase)-Fusionsprotein in *E.coli* exprimiert und mittels Gluthation-Sepharose-Affinitätschromatographie gereinigt. Durch Thrombinverdau mit anschließender Größenausschlusschromatographie wurde das GST entfernt (Dufort, I., Rheault, P., Huang, XF., Soucy, P., and Luu-The, V.,Endocrinology 140, 568-574 (1999)).

Für den Assay wurden 50 nl einer 100-fach konzentrierten Lösung der Testsubstanz in DMSO in eine schwarze low-volume 384well-Mikrotiterplatte (Greiner Bio-One, Frickenhausen, Deutschland) pipettiert, 2,0 $\mu$l einer Lösung von AKR1C3 in Assaypuffer [50 mM Kalium-Phosphatpuffer pH 7, 1 mM DTT, 0,0022% (w/v) Pluronic F-127, 0,01% BSA (w/v) und Proteaseinhibitor-Cocktail (Complete, EDTA-free Protease Inhibitor Cocktail von Roche)] hinzugegeben und die Mischung für 15 min inkubiert, um eine Vorbindung der Substanzen an das Enzym vor der Enzymreaktion zu ermöglichen. Dann wurde die Enzymreaktion durch Zugabe von 3 $\mu$l einer Lösung von NADPH (16,7 $\mu$M $\rightarrow$ Endkonzentration in 5 $\mu$l Assayvolumen ist 10 $\mu$M) und Coumberon (0,5 $\mu$M $\rightarrow$ Endkonzentration in 5 $\mu$l Assayvolumen ist 0,3 $\mu$M) in Assaypuffer gestartet und die resultierende Mischung für die Reaktionszeit von 90 min bei 22°C inkubiert. Die Konzentration der AKR1C3 wurde an die jeweilige Aktivität des Enzym-Präparats angepasst und so eingestellt, dass der Assay im linearen Bereich arbeitete. Typische Konzentrationen lagen im Bereich von 1 nM. Die Reaktion wurde durch Zugabe von 5 $\mu$l einer Stopplösung bestehend aus dem Inhibitor EM-1404 [F. Labrie et al. US Patent 6,541,463, 2003] (2 $\mu$M $\rightarrow$ Endkonzentration in 5 $\mu$l Assayvolumen ist 1 $\mu$M) gestoppt. Anschließend wurde die Fluoreszenz des Coumberols bei 520 nm (Anregung bei 380 nm) mit einem geeigneten Messgerät (Pherastar von BMG Labtechnologies) gemessen. Die Intensität der Fluoreszenz wurde als Maß für die Menge des gebildeten Coumberols und damit für die Enzymaktivität von AKR1C3 verwendet. Die Daten wurden normalisiert (Enzymreaktion ohne Inhibitor = 0 % Inhibition; alle anderen Assaykomponenten, aber kein Enzym = 100 % Inhibition). Üblicherweise wurden die Testsubstanzen auf

derselben Mikrotiterplatte bei 11 verschiedenen Konzentrationen im Bereich von 20 μM bis 96,8 pM (20 μM, 5,9 μM, 1,7 μM, 0,5 μM, 0,15 μM, 44 nM, 12,9 nM, 3,8 nM, 1,1 nM, 0,3 nM und 96,8 pM, die Verdünnungsreihen wurden vor dem Assay auf der Ebene der 100-fach konzentrierten Lösung durch serielle 1:3 Verdünnungen mit 100% DMSO hergestellt) in Doppelwerten für jede Konzentration getestet, und $IC_{50}$-Werte wurden mit einem 4-Parameter-Fit kalkuliert.

Wie beschrieben wurden die beanspruchten pharmakologischen Substanzen auf ihre inhibitorische Wirkung auf das AKR1C3 Enzym untersucht (siehe Tabelle 1). Die beanspruchten Verbindungen zeigen eine starke Inhibition von AKR1C3 *in vitro* ($IC_{50}$-Werte < 500 nM) und überwiegend sogar $IC_{50}$-Werte < 100 nM.

**Tabelle 1:** Inhibition von AKR1C3 der erfindungsgemäßen Verbindungen (angegeben sind für einen Teil der Verbindungen die Werte für zwei experimentelle Bestimmungen)

| Beispielverbindung | AKR1C3 Enzym Inhibition $IC_{50}$ [nmol/l] | Beispielverbindung | AKR1C3 Enzym Inhibition $IC_{50}$ [nmol/l] | Beispielverbindung | AKR1C3 Enzym Inhibition $IC_{50}$ [nmol/l] |
|---|---|---|---|---|---|
| 1 | 6 | 14 | 54 | 30 | 12 |
| 1 | 5 | 14 | 37 | 31 | 19 |
| 2 | 189 | 15 | 25 | 32 | 11 |
| 2 | 100 | 15 | 25 | 33 | 4 |
| 3 | 46 | 16 | 15 | 33 | 5 |
| 3 | 25 | 16 | 42 | 34 | 6 |
| 4 | 4 | 17 | 29 | 35 | 17 |
| 4 | 4 | 17 | 20 | 36 | 6 |
| 5 | 313 | 18 | 45 | 37 | 5 |
| 5 | 199 | 18 | 35 | 38 | 4 |
| 5 | 143 | 19 | 60 | 39 | 5 |
| 6 | 346 | 19 | 48 | 41 | 8 |
| 7 | 27 | 20 | 12 | 42 | 11 |
| 7 | 27 | 20 | 8 | 43 | 7 |
| 8 | 73 | 20 | 20 | 44 | 12 |
| 9 | 61 | 20 | 16 | 45 | 6 |
| 9 | 81 | 21 | 5 | 46 | 6 |
| 10 | 97 | 22 | 5 | 47 | 119 |
| 10 | 46 | 23 | 9 | | |
| 11 | 34 | 24 | 17 | | |
| 11 | 20 | 25 | 48 | | |
| 12 | 21 | 26 | 5 | | |
| 12 | 20 | 27 | 5 | | |
| 13 | 108 | 28 | 9 | | |
| 13 | 79 | 29 | 10 | | |

**Beispiel 49 (Test auf AKR1C3 Inhibition im zellbasierten System)**

Die Inhibierung der AKR1 C3 durch die in dieser Erfindung beschriebenen Substanzen wurde in einem zellbasierten Assay unter Verwendung von Coumberol als Substrat für die AKR1C3 (Halim, M., Yee, D.J., and Sames, D., J. AM. CHEM. SOC. 130, 14123-14128 (2008) und Yee, D.J., Balsanek, V., Bauman, D.R., Penning, T.M., and Sames, D., Proc. Natl. Acad. Sci. USA 103, 13304 -13309 (2006)) gemessen (vgl. Beispiel 48).

Als Zellsystem wurden HEK293 Zellen (ATCC, USA) verwendet (Zellkulturmedium: DMEM, 1.5 g Glukose, 10% FCS, PSG). Die Zellen wurden mit einem AKR1C3 Expressionsplasmid (pCMV6-AC-AKR1C3, GenBank Accession No. NM_003739.4) über Nacht transfiziert (X-tremeGENE HP, Roche). Am nächsten Morgen wurden die Zellen in schwarzen 96well Zellkulturplatten mit einer Zelldichte von 40000 Zellen/well ausgesät (Greiner Bio-One, Frickenhausen, Deutschland). 7 h später wurden die Zellen mit den Testsubstanzen (gelöst in 100x Konzentration in DMSO, Endkonzentration zwischen $10^{-11}$M und $10^{-5}$M) und Coumberol (gelöst in Zellkulturmedium, Endkonzentration $5x10^{-6}$M) über Nacht inkubiert. Am folgenden Morgen wurde die Fluoreszenz des Coumberols bei 535 nm (Anregung bei 355 nm) mit einem geeigneten Messgerät (Mithras, Fa. Berthold) gemessen. Die Intensität der Fluoreszenz wurde als Maß für die Menge des gebildeten Coumberols und damit für die Enzymaktivität von AKR1C3 verwendet. Die Daten wurden normalisiert (transfizierte Zellen ohne Inhibitor, nur DMSO = 0 % Inhibition; transfizierte Zellen, 10 µM Inhibitor EM-1404 [F. Labrie et al. US Patent 6,541,463, 2003] = 100 % Inhibition) und $IC_{50}$-Werte wurden mit einem 4-Parameter-Fit kalkuliert.

Die beanspruchten pharmakologischen Substanzen wurden mittels des oben beschriebenen zellbasierten Assays auf ihre inhibitorische Wirkung auf das AKR1C3 Enzym untersucht (siehe Tabelle 2). Die Verbindungen zeigten eine starke Inhibition der zellulären AKR1C3 *in vitro* ($IC_{50}$-Werte< 100 nM).

**Tabelle 2:** Inhibition von AKR1C3 der erfindungsgemäßen Verbindungen in einem zellulären Assay (angegeben sind die Werte für mindestens zwei experimentelle Bestimmungen)

| Beispielverbindung | Zelluläre AKR1C3 Inhibition IC50 [nmol/l]) | Beispielverbindung | Zelluläre AKR1C3 Inhibition IC50 [nmol/l]) | Beispielverbindung | Zelluläre AKR1C3 Inhibition IC50 [nmol/l]) |
|---|---|---|---|---|---|
| 1 | 13 | 12 | 68 | 17 | 34 |
| 1 | 27 | 12 | 96 | 17 | 28 |
| 1 | 28 | 12 | 43 | 20 | 32 |
| 1 | 66 | 12 | 23 | 20 | 42 |
| 7 | 83 | 16 | 97 | | |
| 7 | 150 | 16 | 74 | | |

**Beispiel 50 (Inhibition von Cyp17A1)**

CYP17A1 (Synonym 17α-Hydroxylase/17,20 Lyase) ist ein Enzym, das an Pregnenolon und an Progesteron eine Hydroxyl-Gruppe an Position 17 des steroidalen D-Rings addiert, wodurch 17α-Hydroxyprogesteron und 17α-Hydroxypregnenolon gebildet wird. Nachfolgend wird Dehydroepiandrosteron und Androstendion gebildet. Der bekannte CYP17A1 Inhibitor *Abirateron* wird zum Beispiel für die Therapie von metastasiertem, kastrationsrefraktärem Prostatakarzinom nach Versagen einer Docetaxel-basierten Chemotherapie eingesetzt (Urologe 2010, 49, 64-68). Abiraterone blockiert die Androgensynthese und Estrogensynthese im gesamten Körper und senkt demnach die Hormonprduktion nicht gewebespezifisch, was zu unerwünschten Nebenwirkungen führt (vgl. Pressemitteilung der *FDA, U.S. Food and Drug Admistration* vom 28. April 2011).

Überaschenderweise wurde gefunden, dass die erfindungsgemäßen Verbindungen, obwohl sie einen aromatischen stickstoffhaltigen Heterocyclus an Position 17 des steroidalen Gerüstes zeigen, CYP17A1 nicht oder nur sehr schwach inhibieren.

Assaybeschreibung:

Die Inhibierung der CYP17A1 durch die Testverbindungen wurde mittels rekombinantem Enzym evaluiert. Humane CYP17A1 wurde in E. coli exprimiert (Ehmer, P. B. et al.; J. Steroid Biochem. Mol. Biol., 75, 57-63 (2000)). Die Microsomale Fraktion und 140 µL Phosphatpuffer (50 mM Na-Phosphat, 1mM $MgCl_2$, 0.1 mM EDTA, 0.1 mM Dithiothreitol, pH 7.4) mit einer Mischung aus Progesteron (24.95 µM) und $^3$H-Progesterone (0.05 µM, 101.3 Ci/mmol), 50 µM eines NADPH Regenerationssystems (in Phosphatpuffer mit 10 mM NADP+, 100 mM Glukose-6-Phosphat und 2.5 U Glukose-6-Phosphat Dehydrogenase) und den entsprechenden Testsubstanzen (in 5 µL DMSO) wurden einzeln bei 37°C für 5 Minuten präinkubiert. Die Reaktion wurde durch Zugabe des Enzyms gestartet und nach 30 Minuten Inkubation bei 37°C durch Zugabe von 50 µL 1 N Salzsäure gestoppt.

Die Steroide wurden mit Ethylacetat extrahiert. Nach Verdampfen der organischen Phase wurden die Steroide

in Acetonitril aufgenommen. 16α-Hydroxyprogesteron, 17α-Hydroxyprogesteron und Progesteron wurden mit Acetonitril/Wasser (45:55) als mobile Phase auf einer C18 *reverse phase* Chromatographiesäule (Nucleodur C18 Gravity, 3 μm, Macherey-Nagel, Düren) auf einem HPLC System (Agilent 1100 Series, Agilent Technologies, Waldbronn) getrennt. Detektion und Quantifizierung der Steroide wurde mittels eines Radioflow Detektors (Berthold Technologies, Bad Wildbad) durchgeführt. Die Inhibition wurde nach folgender Formel berechnet:

$$\%Inhibition = \frac{\%(17\alpha - Hydroxyprogesteron + 16\alpha - Hydroxyprogesteron)}{\%(17\alpha - Hydroxyprogesteron + 16\alpha - Hydroxyprogesteron) + \mathrm{Pr}ogesteron} \cdot 100$$

Jeder Wert wurde aus mindestens drei unabhängigen Experimenten berechnet. Der finale $IC_{50}$ Wert wurde als Mittelwert aus 3 oder 4 unabhängigen $IC_{50}$ Werten berechnet.

Die erfindungsgemäßen Verbindungen zeigen keine Inhibition von Cyp17A1 (Tabelle 3), dagegen ist der bekannte Cyp17A1 Inhibitor Abirateron (als freie Base eingesetzt) im verwendeten Assay aktiv.

**Tabelle 3:** Inhibierung von *humaner* CYP17

| **Beispielverbindung** | **$IC_{50}$ ± SD (μM)** |
|---|---|
| | **CYP17** |
| Abirateron | 0.029 ± 0.004 |
| 4 | $IC_{50}$ > 20 μM |
| 20 | $IC_{50}$ > 20 μM |

**Beispiel 51 (AKR1C1,2,4-inhibitorische Wirkung)**

Die AKR1C1 / AKR1C2 / AKR1C4-inhibitorische Wirkung der erfindungsgemäßen Substanzen zur Bestimmung der Selektivität dieser Erfindung wurde in dem in den folgenden Absätzen beschriebenen Assay gemessen.

Im Wesentlichen wird die Enzymaktivität durch die Quantifizierung des NADPH (Nicotinamid-Adenin-Dinukleotidphosphat) Verbrauchs bei der Umsetzung von Phenanthrenquinon (PQ) durch die AKR1C-Enzyme gemessen.

Als Enzyme wurden rekombinantes humanes AKR1C1, AKR1C2 sowie AKR1C4 (Aldo-keto reductase family 1 member C1, 2, 4) (GenBank Accession No. NM_001353.5, NM_001354, NM_001818) verwendet. Dieses wurde als GST (Glutathion-S-Transferase)-Fusionsprotein in *E.coli* exprimiert und mittels Gluthation-Sepharose-Affinitätschromatographie gereinigt. Durch Thrombinverdau mit anschließender Größenausschlusschromatographie wurde das GST entfernt (Dufort, I., Rheault, P., Huang, XF., Soucy, P., and Luu-The, V.,Endocrinology 140, 568-574 (1999)).

Für den Assay wurden 50 nl einer 100-fach konzentrierten Lösung der Testsubstanz in DMSO sowie 90 μL Assaypuffer [50 mM Kalium-Phosphatpuffer pH 7, 0,0022% (w/v) Pluronic F-127, 0,02 % BSA (w/v), 170 μM NADPH, 100 nM PQ] in eine schwarze 96well-Mikrotiterplatte (Greiner Bio-One, Frickenhausen, Deutschland) pipettiert. Zum Starten der Reaktion wurden 10 μL Enzym [20 nM] hinzugegeben und die Fluoreszenz zum Zeitpunkt 0 bei 460 nm (Anregung bei 355 nm) mit einem geeigneten Messgerät bestimmt. Die Mischung bei 37°C für 3h inkubiert, und die Fluoreszenz am Reaktionsende bestimmt.

Die Differenz der Fluoreszenzintensitäten wurde als Maß für den Verbrauch an NADPH und damit für die Enzymaktivität von AKR1C1, -2 bzw. -4 verwendet. Die Daten wurden normalisiert (Enzymreaktion ohne Inhibitor = 0 % Inhibition; alle anderen Assaykomponenten, aber kein Enzym = 100 % Inhibition). Üblicherweise wurden die Testsubstanzen auf derselben Mikrotiterplatte bei 10 verschiedenen Konzentrationen im Bereich von 10 μM bis 1 pM getestet (10 μM, 1 μM, 100 nM, 10 nM, 1 nM, 100 pM, 10 pM, 1 pM). Wie beschrieben wurden die beanspruchten pharmakologischen Substanzen auf ihre inhibitorische Wirkung auf die AKR1C Enzyme -1, -2 und -4 untersucht (siehe Tabelle 4).

**Tabelle 4:** Inhibition von AKR1C1, -2 bzw. -4 der erfindungsgemäßen Verbindungen (angegeben sind die Werte für zwei experimentelle Bestimmungen)

| Beispielverbindung | AKR1C1 Enzym Inhibition $IC_{50}$ [nmol/l] | AKR1C2 Enzym Inhibition $IC_{50}$ [nmol/l] | AKR1C4 Enzym Inhibition $IC_{50}$ [nmol/l] |
|---|---|---|---|
| 1 | Kein Effekt | Kein Effekt | Kein Effekt |
| 1 | Kein Effekt | Kein Effekt | Kein Effekt |
| Phenolphthalein | 46 | 554 | 21 |

(fortgesetzt)

| Beispielverbindung | AKR1C1 Enzym Inhibition $IC_{50}$ [nmol/l] | AKR1C2 Enzym Inhibition $IC_{50}$ [nmol/l] | AKR1C4 Enzym Inhibition $IC_{50}$ [nmol/l] |
|---|---|---|---|
| Phenolphthalein | 60 | 438 | 17 |

**Beispiel 52 (kinetische Löslichkeit)**

Die kinetischen Löslichkeiten der erfindungsgemäßen Substanzen wurden durch Laser Nephelometrie bestimmt.

Zur Aufnahme der Daten wurden folgende Instrumente verwendet:

Liquid Handling System: Hamilton Star
Nephelometer: Nepheloskan Ascent

Folgende Chemikalien und Materialien wurden verwendet:

DMSO
Di-Natriumhydrogenphosphat Dihydrat
Kaliumdihydrogenphosphat
Gummi Arabicum

Herstellung der Phosphat Puffer pH 7.4:

Lösung 1: 9.71 g Di-Natriumhydrogenphosphat Dihydrat und 1.65 g Kaliumdihydrogenphosphat gelöst in 1 Liter Wasser
Lösung 2: 10 mg Gummi Arabicum gelöst in 1 Liter Wasser

150 ml Lösung 1 und 100 ml Lösung 2 wurden mit 750 ml Wasser verdünnt.

Experimentelles Protokoll:

150 µl einer 10 mM DMSO Stammlösung wurde in eine Platte (deep-well plate, Abgene, PP, 1.2 ml) pipettiert. Die Stammlösung wurde mit DMSO verdünnt was zu einer neuen Multi-Titerplatte (MTP, Greiner bio-one, PS, V-Form) führte mit acht Konzentrationen: 10/5/2.5/1.25/0.625/0.313/0.156 und 0.078 mM.

261 µl Phosphat Puffer wurden in eine weiter Platte (96 cliniplate, UB Thermo Electron Corporation) transferiert und mit 9 µl der DMSO Stammlösungen versetzt.

Die Konzentration des Ko-Solvens DMSO wurde bei 3% konstant gehalten.

Die erhaltenen Platten (cliniplates) wurden im Nephelometer vermessen.

Folgende nephelometrische Löslichkeiten wurden bestimmt:

| Beispiel | Nephelometrische Löslichkeit [mg/l] bei pH = 7.4 |
|---|---|
| 21 | 158 |
| 22 | 153 |
| 28 | 166 |
| 29 | 24 |
| 33 | 80 |
| 35 | 22 |
| 38 | 145 |
| 40 | 145 |
| 43 | 154 |

(fortgesetzt)

| Beispiel | Nephelometrische Löslichkeit [mg/l] bei pH = 7.4 |
|---|---|
| EM-1404 | 18 |

**Beispiel 53: Bestimmung der anti-androgenen Wirkung**

Die antiandrogene Wirkung der Substanz wurde in erwachsenen Affen (Macaca fascicularis) gemessen, als Surrogat für die antiproliferativen Effekte im Prostatakrebs und dessen Metastasen. Die Affen (4 pro Gruppe) wurden auf oralem Wege mittels Schlundsonde mit 1, 3 oder 10 mg/kg Substanz oder mit Vehikel während 4 Wochen behandelt. Die Größe der Prostata und der Samenblase wurde am Anfang des Experimentes und nach einer, zwei, drei und vier Wochen durch Ultraschall bestimmt. Die Abnahme des Gewichtes dieser Organe wurde als Nachweis für die Antiandrogenezität der Substanzen genommen. Zusätzlich wurden die Blutkonzentrationen (im Serum oder im Plasma) verschiedener Steroide (DHEA, Testosterone, Androstendione, Hydroxyprogesterone) und Prostaglandinen (PGD2, PGJ2, PGF2alpha) am Anfang des Experiments und nach einer, zwei, drei oder vier Wochen bestimmt. Da AKR1C3 sowohl im Steroid- wie auch im Prostaglandinsyntheseweg eine Rolle spielt, werden Änderungen in den Blutkonzentrationen dieser Steroide und Prostaglandine als Indiz für die *in vivo* Wirkung der Substanzen genommen.

## Patentansprüche

**1.** Verbindungen der allgemeinen Formel (I)

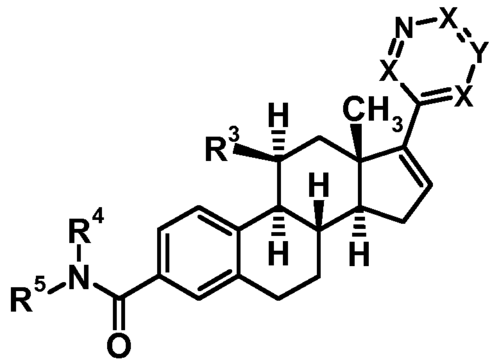

**(I)**

wobei

X unabhängig voneinander Kohlenstoff oder Stickstoff, wobei der Kohlenstoff mit $R^1$ substituiert sein kann,
Y Kohlenstoff oder Stickstoff, wobei der Kohlenstoff mit $R^2$ substituiert sein kann,
$R^1$ und $R^2$ unabhängig voneinander Wasserstoff, Halogen, $C_1$-$C_6$-Alkyl, $C_2$-$C_6$-Alkenyl, $C_2$-$C_4$-Alkinyl, $C_3$-$C_6$-Cycloalkyl, $C_1$-$C_6$-Alkoxy, $C_1$-$C_6$-Halogenalkyl, $C_1$-$C_6$-Halogenalkoxy, Nitril, Nitro, $-SO_2CH_3$, $-SO_2CH_2CH_3$, $-(C{=}O)CH_3$, Carboxyl, Hydroxyl, $-NH_2$, $-CH_2NH_2$, $-CH_2OH$, $-CH(OH)CH_3$, $-C(CH_3)_2OH$, $-(C{=}O)NH_2$, $-(C{=}O)NHCH_3$, $-(C{=}O)NHCH_2CH_3$, $-(C{=}O)N(CH_3)_2$, $-SO_2NH_2$, $-SO_2NHCH_3$ oder $-SO_2N(CH_3)_2$,
$R^3$ Wasserstoff oder Halogen,
$R^4$ Wasserstoff, $C_1$-$C_6$-Alkyl, $C_3$-$C_6$-Cycloalkyl oder $C_3$-$C_6$-Cycloalkyl-$C_1$-$C_6$-alkyl, welche gegebenenfalls mit bis zu 6 Halogenatomen substituiert sind und gegebenenfalls einfach oder zweifach substituiert sind mit Hydroxyl, $C_1$-$C_6$-Alkoxy, $C_1$-$C_6$-Halogenalkoxy,
$R^5$ Wasserstoff, $C_1$-$C_6$-Alkyl oder $C_3$-$C_6$-Cycloalkyl, wobei $C_1$-$C_6$-Alkyl und $C_3$-$C_6$-Cycloalkyl gegebenenfalls mit bis zu 6 Halogenatomen substituiert sind und gegebenenfalls einfach oder zweifach substituiert sind mit Hydroxyl, Hydroxy-$C_1$-$C_6$-alkyl, $C_1$-$C_6$-Alkyl, $C_1$-$C_6$-Alkoxy, Hydroxy-$C_2$-$C_6$-alkoxy, $C_1$-$C_6$-Halogenalkoxy, $C_3$-$C_6$-Cycloalkyl, Aryl, Heteroaryl, 3-10gliedriges Heterocycloalkyl, Aryl-$C_1$-$C_6$-alkyl, Heteroaryl-$C_1$-$C_6$-alkyl, -C(=O)R', -C(=O)$NH_2$, -C(=O)N(H)R', -C(=O)N(R')R", -$NH_2$, -NHR' -N(R')R", -N(H)C(=O)R', -N(R')C(=O)R', -N(H)C(=O)OR', -N(R')C(=O)OR', -$NO_2$, -N(H)S(=O)R', -N(R')S(=O)R', -N(H)S$(=O)_2$R', -N(R')S$(=O)_2$R', -N=S(=O)(R')R", -S(=O)R', -S$(=O)_2$R', -S$(=O)_2NH_2$, -S$(=O)_2$NHR', -S$(=O)_2$N(R')R", -S(=O)(=NR')R", wobei Aryl,

Heteroaryl, Aryl-$C_1$-$C_6$-alkyl und Heteroaryl-$C_1$-$C_6$-alkyl gegebenenfalls einfach oder mehrfach unabhängig voneinander substituiert sind mit $R^6$ und 3-10gliedriges Heterocycloalkyl gegebenenfalls einfach oder mehrfach unabhängig voneinander substituiert ist mit R'

oder

$R^4$ und $R^5$ gemeinsam mit dem direkt verknüpften Stickstoffatom einen 4-7gliedrigen Ring, welcher gegebenenfalls substituiert ist mit einem oder zwei Substituenten bestehend aus:

Halogen, Nitril, Hydroxyl, $C_1$-$C_6$-Alkyl, $C_1$-$C_6$-Halogenalkyl, $C_2$-$C_6$-Alkenyl, $C_2$-$C_4$-Alkinyl, $C_3$-$C_{10}$-Cycloalkyl, Aryl, Heteroaryl, -C(=O)$NH_2$ -C(=O)N(H)R', -C(=O)N(R')R'', -C(=O)OR', -$NH_2$, -NHR', -N(R')R'', -N(H)C(=O)R', -N(R')C(=O)R', -N(H)S(=O)R', -N(R')S(=O)R', -N(H)S(=O)$_2$R', -N(R')S(=O)$_2$R', -N=S(=O)(R')R'', -OH, $C_1$-$C_6$-Alkoxy, $C_1$-$C_6$-Halogenalkoxy, -OC(=O)R', -OC(=O)$NH_2$, -OC(=O)NHR', -OC(=O)N(R')R'', -SH, $C_1$-$C_6$-Alkyl-S-, -S(=O)R', -S(=O)$_2$R', -S(=O)$_2$$NH_2$, -S(=O)$_2$NHR', -S(=O)$_2$N(R')R'', wobei Aryl und Heteroaryl gegebenenfalls einfach oder mehrfach unabhängig voneinander substituiert sind mit $R^6$

und in welchem 5-, 6- oder -7gliedrigen Ring gegebenenfalls eine oder mehrere Methylengruppen durch NH, NR', O oder S ersetzt sind,

$R^6$ Halogen, Nitril, $C_1$-$C_6$-Alkyl, $C_1$-$C_6$-Halogenalkyl, $C_2$-$C_6$-Alkenyl, $C_2$-$C_4$-Alkinyl, $C_3$-$C_{10}$-Cycloalkyl, 3-10gliedriges Heterocycloalkyl, Aryl, Heteroaryl, -C(=O)R',-C(=O)$NH_2$, -C(=O)N(H)R',-C(=O)N(R')R'', -C(=O)OR', -$NH_2$, -NHR', -N(R')R'', -N(H)C(=O)R', -N(R')C(=O)R', -N(H)C(=O)$NH_2$, -N(H)C(=O)NHR', -N(H)C(=O)N(R')R'', -N(R')C(=O)$NH_2$, -N(R')C(=O)NHR', -N(R')C(=O)N(R')R'', -N(H)C(=O)OR', -N(R')C(=O)OR', -$NO_2$, -N(H)S(=O)R', -N(R')S(=O)R', -N(H)S(=O)$_2$R', -N(R')S(=O)$_2$R', -N=S(=O)(R')R'', -OH, $C_1$-$C_6$-Alkoxy, $C_1$-$C_6$-Halogenalkoxy, -OC(=O)R', -OC(=O)$NH_2$, -OC(=O)NHR', -OC(=O)N(R')R'', -SH, $C_1$-$C_6$-Alkyl-S-, -S(=O)R', -S(=O)$_2$R', -S(=O)$_2$$NH_2$, -S(=O)$_2$NHR', -S(=O)$_2$N(R')R'', S(=O)(=NR')R'',

R' und R'' unabhängig voneinander $C_1$-$C_6$-Alkyl, $C_3$-$C_{10}$-Cycloalkyl oder $C_1$-$C_6$-Halogenalkyl bedeuten oder deren Tautomere, N-Oxide, Hydrate, Solvate oder Salze oder eine Mischung bestehend aus den Vorgenannten, worin "Heterocycloalkyl" steht für einen gesättigten Heterocyclus mit insgesamt 3 bis 10 Ringatomen, der ein oder zwei Ring-Heteroatome aus der Reihe N, O, S, SO und/oder $SO_2$ enthält; "Aryl" steht für einen mono- bis tricyclischen aromatischen, carbocyclischen Rest mit in der Regel 6 bis 14 Kohlenstoffatomen; "Heteroaryl" steht für einen monocyclischen aromatischen Heterocyclus mit insgesamt 5 oder 6 Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist; der oben genannte monocyclische aromatische Heterocyclus ist gegebenenfalls mit Hydroxyl oder SH substituiert; Aryl-$C_1$-$C_6$-alkyl steht für einen mono- bis tricyclischen aromatischen, carbocyclischen Rest mit in der Regel 6 bis 14 Kohlenstoffatomen, der über einen linearen oder verzweigten Alkylrest mit der jeweils angegebenen Anzahl an Kohlenstoffatomen gebunden ist und "Heteroaryl-$C_{1-6}$-Alkyl" steht für einen monocyclischen aromatischen Heterocyclus mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist und der über einen linearen oder verzweigten Alkylrest mit der jeweils angegebenen Anzahl an Kohlenstoffatomen gebunden ist.

**2.** Verbindungen der allgemeinen Formel (I) gemäß Anspruch 1, wobei

X Kohlenstoff substituiert mit Wasserstoff,

Y Kohlenstoff oder Stickstoff, wobei der Kohlenstoff mit $R^2$ substituiert sein kann,

$R^2$ Wasserstoff, Fluor, Chlor, Nitril, Methoxy, Ethoxy, Trifluormethoxy, Methyl, Ethyl, Trifluormethyl, -(C=O)$CH_3$,

$R^3$ Wasserstoff oder Fluor,

$R^4$ Wasserstoff, Methyl, Ethyl, Isopropyl, Propyl, Butyl, Cyclopropyl oder 2,2,2-Trifluorethyl,

$R^5$ Wasserstoff, Methyl, Ethyl, Propyl, 2,2,2,-Trifluorethyl, 3,3,3-Trifluorpropyl, 2-Fluorethyl, 2-Sulfamoylethyl, 3-Sulfamoylpropyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1*H*-Tetrazol-5-yl)ethyl, 1*H*-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, 4-Hydroxybutyl, (2*RS*)-2,3-Dihydroxypropyl, (2*R*)-2,3-Dihydroxypropyl, 2,3-Dihydroxybutyl, 2-(Methylsulfinyl)ethyl, 3-(Methylsulfinyl)propyl, 2-(Methylsulfonyl)ethyl, 3-(Methylsulfonyl)propyl, 2-(*S*-Methylsulfonimidoyl)ethyl, (2*R*)-2-Hydroxypropyl, (2*S*)-1-Hydroxypropan-2-yl, 2-Methoxyethyl, 3-Methoxypropyl, 2-(Isopropylsulfonyl)ethyl, (3-Methyloxetan-3-yl)methyl, (2*S*)-2-Hydroxypropyl, 2-(2-Hydroxyethoxy)ethyl oder

$R^4$ und $R^5$ gemeinsam mit dem direkt verknüpften Stickstoffatom Piperidinyl, Pyrrolidinyl, Morpholinyl, N-Methylpiperazinyl, 1-Oxidothiomorpholinyl, 1,1-Dioxidothiomorpholin-4-yl, 4-Hydroxypiperidinyl, 4-(Trifluormethyl)piperidin-4-yl, (3*R*)-3-Hydroxypiperidinyl, (2*S*)-2-(1*H*-Tetrazol-5-yl)pyrrolidinyl, *N*-Methyl-L-prolinamidyl und L-Prolinamidyl

bedeuten oder deren Tautomere, N-Oxide, Hydrate, Solvate oder Salze oder eine Mischung bestehend aus den Vorgenannten.

**3.** Verbindungen der allgemeinen Formel (I) gemäß Anspruch 1-2, wobei

X Kohlenstoff substituiert mit Wasserstoff,
Y Kohlenstoff oder Stickstoff, wobei der Kohlenstoff mit $R^2$ substituiert sein kann,
$R^2$ Wasserstoff, Fluor, Chlor, Methyl, Nitril, Methoxy, Trifluormethyl,
$R^3$ Wasserstoff oder Fluor,
$R^4$ Wasserstoff, Methyl, Ethyl, Isopropyl, Propyl oder Cyclopropyl,
$R^5$ Wasserstoff, Methyl, Ethyl, 2-Sulfamoylethyl, 3-Sulfamoylpropyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1*H*-Tetrazol-5-yl)ethyl, 1*H*-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, (2*RS*)-2,3-Dihydroxypropyl, (2*R*)-2,3-Dihydroxypropyl, 2-(Methylsulfinyl)ethyl, (2*R*)-2-Hydroxypropyl, (2*S*)-1-Hydroxypropan-2-yl, 2-Methoxyethyl, 2-(Isopropylsulfonyl)ethyl, (3-Methyloxetan-3-yl)methyl, (2*S*)-2-Hydroxypropyl oder 2-(2-Hydroxyethoxy)ethyl
oder
$R^4$ und $R^5$ gemeinsam mit dem direkt verknüpften Stickstoffatom Piperidinyl, Pyrrolidinyl, Morpholinyl, 4-Hydroxypiperidinyl, (*3R*)-*3*-Hydroxypiperidinyl, (2*S*)-2-(1*H*-Tetrazol-5-yl)pyrrolidinyl, *N*-Methyl-L-prolinamidyl oder L-Prolinamidyl

bedeuten oder deren Tautomere, N-Oxide, Hydrate, Solvate oder Salze oder eine Mischung bestehend aus den Vorgenannten.

**4.** Verbindungen der allgemeinen Formel (I) gemäß Anspruch 1-3, wobei

X Kohlenstoff substituiert mit Wasserstoff,
Y Kohlenstoff oder Stickstoff, wobei der Kohlenstoff mit $R^2$ substituiert sein kann,
$R^2$ Wasserstoff, Fluor, Nitril, Methoxy oder Trifluormethyl,
$R^3$ Wasserstoff oder Fluor,
$R^4$ Wasserstoff, Methyl, Ethyl oder Isopropyl,
$R^5$ Wasserstoff, Ethyl, 2-Sulfamoylethyl, (1*S*,2*R*)-2-Hydroxycyclopentyl, 3-Hydroxy-2,2-dimethylpropyl, (1*S*,2*S*)-2-Hydroxycyclopentyl, (3*R*)-4-Hydroxy-3-methylbutyl, 1-(Hydroxymethyl)cyclopentyl, (2*S*)-1-Hydroxybutan-2-yl, (2*R*)-1-Hydroxy-3-methylbutan-2-yl, 3-Hydroxybutan-2-yl, 2-Hydroxyethyl, 3,3,3-Trifluor-2-hydroxypropyl, 2-(1H-Tetrazol-5-yl)ethyl, 1H-Tetrazol-5-ylmethyl, 2-(Methylsulfamoyl)ethyl, 3-Amino-3-oxopropyl, 3-(Methylamino)-3-oxopropyl, 2-Methyl-2-[(methylsulfonyl)amino]propyl, (2*S*)-2,3-Dihydroxypropyl, 3-Hydroxypropyl, (2*RS*)-2,3-Dihydroxypropyl, (2*R*)-2,3-Dihydroxypropyl, 2-(Methylsulfinyl)ethyl, (2*R*)-2-Hydroxypropyl, (2*S*)-1-Hydroxypropan-2-yl, 2-Methoxyethyl, 2-(Isopropylsulfonyl)ethyl, (3-Methyloxetan-3-yl)methyl, (2*S*)-2-Hydroxypropyl oder 2-(2-Hydroxyethoxy)ethyl
oder
$R^4$ und $R^5$ gemeinsam mit dem direkt verknüpften Stickstoffatom 4-Hydroxypiperidinyl, (3*R*)-3-Hydroxypiperidinyl, (2*S*)-2-(1*H*-Tetrazol-5-yl)pyrrolidinyl, *N*-Methyl-L-prolinamidyl oder L-Prolinamidyl

bedeuten oder deren Tautomere, N-Oxide, Hydrate, Solvate oder Salze oder eine Mischung bestehend aus den Vorgenannten.

**5.** Verbindungen gemäß Anspruch 1 - 4, ausgewählt aus einer Gruppe, die die folgenden Verbindungen enthält:

- 17-(3-Pyridyl)estra-1,3,5(10),16-tetraen-3-carboxamid
- 17-(5-Methoxypyridin-3-yl)estra-1,3,5(10),16-tetraen-3-carboxamid
- 17-[5-(Trifluormethyl)pyridin-3-yl]estra-1,3,5(10),16-tetraen-3-carboxamid

- 17-(5-Fluorpyridin-3-yl)estra-1,3,5(10),16-tetraen-3-carboxamid
- 17-(Pyrimidin-5-yl)estra-1,3,5(10),16-tetraen-3-carboxamid
- 17-(5-Cyanpyridin-3-yl)estra-1,3,5(10),16-tetraen-3-carboxamid
- 11$\beta$-Fluor-17-(5-fluorpyridin-3-yl)estra-1,3,5(10),16-tetraen-3-carboxamid
- 11$\beta$-Fluor-17-(5-fluorpyridin-3-yl)-*N*-(2-sulfamoylethyl)estra-1,3,5(10),16-tetraen-3-carboxamid
- 17-(5-Fluorpyridin-3-yl)-*N*-[(1*S*,2*R*)-2-hydroxycyclopentyl]estra-1,3,5(10),16-tetraen-3-carboxamid
- 17-(5-Fluorpyridin-3-yl)-*N*-[2-(hydroxymethyl)-2-methylpropyl]estra-1,3,5(10),16-tetraen-3-carboxamid
- 17-(5-Fluorpyridin-3-yl)-*N*-[(1*S*,2*S*)-2-hydroxycyclopentyl]estra-1,3,5(10),16-tetraen-3-carboxamid
- 17-(5-Fluorpyridin-3-yl)-*N*-[(*R*)-3-(hydroxymethyl)butyl]estra-1,3,5(10),16-tetraen-3-carboxamid
- 17-(5-Fluorpyridin-3-yl)-*N*-[1-(hydroxymethyl)cyclopentyl]estra-1,3,5(10),16-tetraen-3-carboxamid
- [17-(5-Fluorpyridin-3-yl)estra-1,3,5(10),16-tetraen-3-yl](4-hydroxypiperidin-1-yl)keton
- 17-(5-Fluorpyridin-3-yl)-*N*-[(5)-1-(hydroxymethyl)propyl]estra-1,3,5(10),16-tetraen-3-carboxamid
- 17-(5-Fluorpyridin-3-yl)-*N*-[(*R*)-1-(hydroxymethyl)-2-methylpropyl]estra-1,3,5(10),16-tetraen-3-carboxamid
- [17-(5-Fluorpyridin-3-yl)estra-1,3,5(10),16-tetraen-3-yl][(*R*)-3-hydroxypiperidin-1-yl]keton
- rel-17-(5-Fluorpyridin-3-yl)-*N*-[(1*R*,2*R*)-2-hydroxy-1-methylpropyl]estra-1,3,5(10),16-tetraen-3-carboxamid
- 17-(5-Fluorpyridin-3-yl)-*N*-(2-hydroxyethyl)-*N*-isopropylestra-1,3,5(10),16-tetraen-3-carboxamid
- 17-(5-Fluorpyridin-3-yl)-*N*-[(*RS*)-3,3,3-trifluor-2-hydroxypropyl]estra-1,3,5(10),16-tetraen-3-carboxamid
- 17-(5-Fluorpyridin-3-yl)-*N*-[2-(1H-tetrazol-5-yl)ethyl]estra-1,3,5(10),16-tetraen-3-carboxamid
- 17-(5-Fluorpyridin-3-yl)-*N*-(1*H*-tetrazol-5-ylmethyl)estra-1,3,5(10),16-tetraen-3-carboxamid
- 17-(3-Pyridyl)-*N*-(2-sulfamoylethyl)estra-1,3,5(10),16-tetraen-3-carboxamid
- *N*-(2-Sulfamoylethyl)-17-[5-(trifluormethyl)pyridin-3-yl]estra-1,3,5(10),16-tetraen-3-carboxamid
- *N*-[2-(*N*-Methylsulfamoyl)ethyl]-17-[5-(trifluormethyl)pyridin-3-yl]estra-1,3,5(10),16-tetraen-3-carboxamid
- *N*-(3-Amino-3-oxopropyl)-17-(5-fluorpyridin-3-yl)estra-1,3,5(10),16-tetraen-3-carboxamid
- 17-(5-Fluorpyridin-3-yl)-*N*-[3-(methylamino)-3-oxopropyl]estra-1,3,5(10),16-tetraen-3-carboxamid
- [17-(5-Fluorpyridin-3-yl)estra-1,3,5(10),16-tetraen-3-yl][(*S*)-2-(1*H*-tetrazol-5-yl)pyrrolidin-1-yl]keton
- 1-{[17-(5-Fluorpyridin-3-yl)estra-1,3,5(10),16-tetraen-3-yl]carbonyl}-*N*-methyl-L-prolinamid
- 1-{[17-(5-Fluorpyridin-3-yl)estra-1,3,5(10),16-tetraen-3-yl]carbonyl}-L-prolinamid
- 17-(5-Fluorpyridin-3-yl)-*N*-{2-methyl-2-[(methylsulfonyl)amino]propyl}estra-1,3,5(10),16-tetraen-3-carboxamid
- *N*-Ethyl-17-(5-fluorpyridin-3-yl)-*N*-(2-hydroxyethyl)estra-1,3,5(10),16-tetraen-3-carboxamid
- *N*-[(*S*)-2,3-Dihydroxypropyl]-17-(5-fluorpyridin-3-yl)estra-1,3,5(10),16-tetraen-3-carboxamid
- 17-(5-Fluorpyridin-3-yl)-*N*-(3-hydroxypropyl)-*N*-methylestra-1,3,5(10),16-tetraen-3-carboxamid
- *N*-[(*RS*)-2,3-Dihydroxypropyl]-17-(5-fluorpyridin-3-yl)-*N*-methylestra-1,3,5(10),16-tetraen-3-carboxamid
- *N*-[(*R*)-2,3-Dihydroxypropyl]-17-(5-fluorpyridin-3-yl)estra-1,3,5(10),16-tetraen-3-carboxamid
- 17-(5-Fluorpyridin-3-yl)-*N*-[2-(methylsulfinyl)ethyl]estra-1,3,5(10),16-tetraen-3-carboxamid
- 17-(5-Fluorpyridin-3-yl)-*N*-[(*R*)-2-hydroxypropyl]estra-1,3,5(10),16-tetraen-3-carboxamid
- *N*-Ethyl-17-(5-fluorpyridin-3-yl)estra-1,3,5(10),16-tetraen-3-carboxamid
- 17-(5-Fluorpyridin-3-yl)-*N*-[(5)-1-(hydroxymethyl)ethyl]estra-1,3,5(10),16-tetraen-3-carboxamid
- 17-(5-Fluorpyridin-3-yl)-*N*-(2-methoxyethyl)estra-1,3,5(10),16-tetraen-3-carboxamid
- 17-(5-Fluorpyridin-3-yl)-*N*-[2-(isopropylsulfonyl)ethyl]estra-1,3,5(10),16-tetraen-3-carboxamid
- 17-(5-Fluorpyridin-3-yl)-*N*-[(3-methyloxetan-3-yl)methyl]estra-1,3,5(10),16-tetraen-3-carboxamid
- 17-(5-Fluorpyridin-3-yl)-*N*-(2-hydroxyethyl)-*N*-methylestra-1,3,5(10),16-tetraen-3-carboxamid
- 17-(5-Fluorpyridin-3-yl)-*N*-[(*S*)-2-hydroxypropyl]estra-1,3,5(10),16-tetraen-3-carboxamid
- 17-(5-Fluorpyridin-3-yl)-*N*-[2-(2-hydroxyethoxy)ethyl]estra-1,3,5(10),16-tetraen-3-carboxamid
- 17-(Pyrimidin-5-yl)-*N*-(2-sulfamoylethyl)estra-1,3,5(10),16-tetraen-3-carboxamid

und deren Tautomere, N-Oxide, Hydrate, Solvate oder Salze oder eine Mischung bestehend aus den Vorgenannten.

**6.** Verbindung der Formel (I), wie in einem der Ansprüche 1 bis 5 definiert, zur Behandlung und/oder Prophylaxe von Krankheiten.

**7.** Verbindung der Formel (I), wie in einem der Ansprüche 1 bis 5 definiert, zur Verwendung in einem Verfahren zur Behandlung und/oder Prophylaxe von Endometriose, von Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

**8.** Verwendung einer Verbindung nach Anspruch 1, 2, 3, 4 oder 5 zur Herstellung eines Arzneimittels zur Behandlung und/oder Prophylaxe von Krankheiten.

**9.** Verwendung einer Verbindung, wie in einem der Ansprüche 1 bis 5 definiert, zur Herstellung eines Arzneimittels zur Prophylaxe von Endometriose, von Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

**10.** Arzneimittel enthaltend eine Verbindung, wie in einem der Ansprüche 1 bis 5 definiert, in Kombination mit einem oder mehreren weiteren Wirkstoffen insbesondere mit selektiven Estrogen Rezeptor Modulatoren (SERMs), Estrogenrezeptor (ER) Antagonisten, Aromataseinhibitoren, 17 HSD1 Inhibitoren, Steroid Sulfatase (STS)-Inhibitoren, GnRH-Agonisten und -Antagonisten, Kisspeptin Rezeptor (KISSR)-Antagonisten, selektiven Androgen Rezeptor Modulatoren (SARMs), Androgenen, 5 -Reduktase Inhibitoren, selektiven Progesteron Rezeptor Modulatoren (SPRMs), Gestagenen, Antigestagenen, orale Kontrazeptiva, Inhibitoren der Mitogen Aktivierten Protein (MAP) Kinasen sowie Inhibitoren der MAP Kinasen Kinasen (Mkk3/6, Mek1/2, Erk1/2), Inhibitoren der Proteinkinasen B (PKB$\alpha/\beta/\gamma$; Akt1/2/3), Inhibitoren der Phosphoinositid-3-Kinasen (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten und Nichtsteroidale Enzündungshemmern (NSAIDs).

**11.** Arzneimittel enthaltend eine Verbindung der Formel (I) wie in einem der Ansprüche 1 bis 5 definiert, in Kombination mit einem inerten, nichttoxischen, pharmazeutisch geeigneten Hilfsstoff.

**12.** Arzneimittel nach Anspruch 10 oder 11 zur Behandlung und Prophylaxe von Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

**13.** Verbindungen der allgemeinen Formel (I), gemäß den Ansprüchen 1-5, zur Verwendung in Form eines pharmazeutischen Präparates für die enterale, parenterale, vaginale, intrauterine und orale Applikation.

## Claims

**1.** Compounds of the general formula (I)

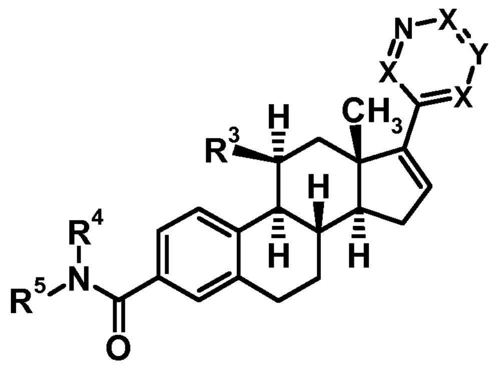

(I)

where

X is independently carbon or nitrogen, where the carbon may be substituted by $R^1$,

Y is carbon or nitrogen, where the carbon may be substituted by $R^2$,
$R^1$ and $R^2$ are each independently hydrogen, halogen, $C_1$-$C_6$-alkyl, $C_2$-$C_6$-alkenyl, $C_2$-$C_6$-alkynyl, $C_3$-$C_6$-cycloalkyl, $C_1$-$C_6$-alkoxy, $C_1$-$C_6$-haloalkyl, $C_1$-$C_6$-haloalkoxy, nitrile, nitro, $-SO_2CH_3$, $-SO_2CH_2CH_3$,-(C=O)$CH_3$, carboxyl, hydroxyl, $-NH_2$, $-CH_2NH_2$, $-CH_2OH$, -CH(OH)$CH_3$, -C($CH_3$)$_2$OH, -(C=O)$NH_2$, -(C=O)$NHCH_3$, -(C=O)$NHCH_2CH_3$, -(C=O)N($CH_3$)$_2$,$-SO_2NH_2$, $-SO_2NHCH_3$ or $-SO_2N(CH_3)_2$,
$R^3$ is hydrogen or halogen,
$R^4$ is hydrogen, $C_1$-$C_6$-alkyl, $C_3$-$C_6$-cycloalkyl or $C_3$-$C_6$-cycloalkyl-$C_1$-$C_6$-alkyl, which are optionally substituted by up to 6 halogen atoms and are optionally mono- or disubstituted by hydroxyl, $C_1$-$C_6$-alkoxy, $C_1$-$C_6$-haloalkoxy,
$R^5$ is hydrogen, $C_1$-$C_6$-alkyl or $C_3$-$C_6$-cycloalkyl, where $C_1$-$C_6$-alkyl and $C_3$-$C_6$-cycloalkyl are optionally substituted by up to 6 halogen atoms and are optionally mono- or disubstituted by hydroxyl, hydroxy-$C_1$-$C_6$-alkyl, $C_1$-$C_6$-alkyl, $C_1$-$C_6$-alkoxy, hydroxy-$C_2$-$C_6$-alkoxy, $C_1$-$C_6$-haloalkoxy, $C_3$-$C_6$-cycloalkyl, aryl, heteroaryl, 3-10-membered heterocycloalkyl, aryl-$C_1$-$C_6$-alkyl, heteroaryl-$C_1$-$C_6$-alkyl, -C(=O)R', -C(=O)$NH_2$, -C(=O)N(H)R', -C(=O)N(R')R", $-NH_2$, -NHR' -N(R')R", -N(H)C(=O)R',-N(R')C(=O)R', -N(H)C(=O)OR',-N(R')C(=O)OR', $-NO_2$, -N(H)S(=O)R',-N(R')S(=O)R', -N(H)S(=O)$_2$R', -N(R')S(=O)$_2$R', -N=S(=O)(R')R", -S(=O)R', -S(=O)$_2$R',-S(=O)$_2$$NH_2$, -S(=O)$_2$NHR', -S(=O)$_2$N(R')R",-S(=O)(=NR')R", where aryl, heteroaryl, aryl-$C_1$-$C_6$-alkyl and heteroaryl-$C_1$-$C_6$-alkyl are optionally each independently mono- or polysubstituted by $R^6$, and 3-10-membered heterocycloalkyl is optionally independently mono- or polysubstituted by R',
or
$R^4$ and $R^5$ together with the directly joining nitrogen atom are a 4-7-membered ring which is optionally substituted by one or two substituents from the group consisting of:

halogen, nitrile, hydroxyl, $C_1$-$C_6$-alkyl, $C_1$-$C_6$-haloalkyl, $C_2$-$C_6$-alkenyl, $C_2$-$C_6$-alkynyl, $C_3$-$C_{10}$-cycloalkyl, aryl, heteroaryl,
-C(=O)$NH_2$, -C(=O)N(H)R', -C(=O)N(R')R", -C(=O)OR', $-NH_2$, -NHR', -N(R')R", -N(H)C(=O)R',-N(R')C(=O)R', -N(H)S(=O)R', -N(R')S(=O)R', -N(H)S(=O)$_2$R',-N(R')S(=O)$_2$R', -N=S(=O)(R')R", -OH, $C_1$-$C_6$-alkoxy, $C_1$-$C_6$-haloalkoxy, -OC(=O)R', -OC(=O)$NH_2$, -OC(=O)NHR', -OC(=O)N(R')R", -SH, $C_1$-$C_6$-alkyl-S-, -S(=O)R', -S(=O)$_2$R',-S(=O)$_2$$NH_2$, -S(=O)$_2$NHR', -S(=O)$_2$N(R')R", where aryl and heteroaryl are optionally each independently mono- or polysubstituted by $R^6$,

and in which 5-, 6- or 7-membered ring one or more methylene groups are optionally replaced by NH, NR', O or S,
$R^6$ is halogen, nitrile, $C_1$-$C_6$-alkyl, $C_1$-$C_6$-haloalkyl, $C_2$-$C_6$-alkenyl, $C_2$-$C_6$-alkynyl, $C_3$-$C_{10}$-cycloalkyl, 3-10-membered heterocycloalkyl, aryl, heteroaryl,-C(=O)R', -C(=O)$NH_2$, -C(=O)N(H)R',-C(=O)N(R')R", -C(=O)OR', $-NH_2$, -NHR', -N(R')R", -N(H)C(=O)R', -N(R')C(=O)R',-N(H)C(=O)$NH_2$, -N(H)C(=O)NHR', -N(H)C(=O)N(R')R",-N(R')C(=O)$NH_2$, -N(R')C(=O)NHR', -N(R')C(=O)N(R')R",-N(H)C(=O)OR', -N(R')C(=O)OR', $-NO_2$, -N(H)S(=O)R',-N(R')S(=O)R', -N(H)S(=O)$_2$R', -N(R')S(=O)$_2$R', -N=S(=O)(R')R", -OH, $C_1$-$C_6$-alkoxy, $C_1$-$C_6$-haloalkoxy, -OC(=O)R', -OC(=O)$NH_2$, -OC(=O)NHR',-OC(=O)N(R')R", -SH, $C_1$-$C_6$-alkyl-S-, -S(=O)R', -S(=O)$_2$R',-S(=O)$_2$$NH_2$, -S(=O)$_2$NHR', -S(=O)$_2$N(R')R", -S(=O)(=NR')R",

R' and R" are each independently $C_1$-$C_6$-alkyl, $C_3$-$C_{10}$-cycloalkyl or $C_1$-$C_6$-haloalkyl, or the tautomers, N-oxides, hydrates, solvates or salts thereof, or a mixture consisting of the above, in which "heterocycloalkyl" is a saturated heterocycle which has a total of 3 to 10 ring atoms and contains one or two ring heteroatoms from the group consisting of N, O, S, SO and/or $SO_2$; "aryl" is a mono- to tricyclic aromatic, carbocyclic radical having generally 6 to 14 carbon atoms; "heteroaryl" is a monocyclic aromatic heterocycle which has a total of 5 or 6 ring atoms, contains up to four identical or different ring heteroatoms from the group of N, O and/or S and is attached via a ring carbon atom or optionally via a ring nitrogen atom; the abovementioned monocyclic aromatic heterocycle is optionally substituted by hydroxyl or SH; aryl-$C_1$-$C_6$-alkyl is a mono- to tricyclic aromatic, carbocyclic radical having generally 6 to 14 carbon atoms which is bonded via a linear or branched alkyl radical having the number of carbon atoms specified in each case and "heteroaryl-$C_{1-6}$-alkyl" is a monocyclic aromatic heterocycle having a total of 5 or 6 ring atoms which contains up to three identical or different ring heteroatoms from the group of N, O and/or S and is bonded via a ring carbon atom or, if present, via a ring nitrogen atom and which is bonded via a linear or branched alkyl radical having the number of carbon atoms specified in each case.

**2.** Compounds of the general formula (I) according to Claim 1, where

X is carbon substituted by hydrogen,
Y is carbon or nitrogen, where the carbon may be substituted by $R^2$,
$R^2$ is hydrogen, fluorine, chlorine, nitrile, methoxy, ethoxy, trifluoromethoxy, methyl, ethyl, trifluoromethyl,

$-(C{=}O)CH_3$,
$R^3$ is hydrogen or fluorine,
$R^4$ is hydrogen, methyl, ethyl, isopropyl, propyl, butyl, cyclopropyl or 2,2,2-trifluoroethyl,
$R^5$ is hydrogen, methyl, ethyl, propyl, 2,2,2-trifluoroethyl, 3,3,3-trifluoropropyl, 2-fluoroethyl, 2-sulphamoylethyl, 3-sulphamoylpropyl, (1*S*,2*R*)-2-hydroxycyclopentyl, 3-hydroxy-2,2-dimethylpropyl, (1*S*,2*S*)-2-hydroxycyclopentyl, (3*R*)-4-hydroxy-3-methylbutyl, 1-(hydroxymethyl)cyclopentyl, (2*S*)-1-hydroxybutan-2-yl, (2*R*)-1-hydroxy-3-methylbutan-2-yl, 3-hydroxybutan-2-yl, 2-hydroxyethyl, 3,3,3-trifluoro-2-hydroxypropyl, 2-(1*H*-tetrazol-5-yl)ethyl, 1*H*-tetrazol-5-ylmethyl, 2-(methylsulphamoyl)ethyl, 3-amino-3-oxopropyl, 3-(methylamino)-3-oxopropyl, 2-methyl-2-[(methylsulphonyl)amino]propyl, (2*S*)-2,3-dihydroxypropyl, 3-hydroxypropyl, 4-hydroxybutyl, (2*RS*)-2,3-dihydroxypropyl, (2*R*)-2,3-dihydroxypropyl, 2,3-dihydroxybutyl, 2-(methylsulphinyl)ethyl, 3-(methylsulphinyl)propyl, 2-(methylsulphonyl)ethyl, 3-(methylsulphonyl)propyl, 2-(*S*-methylsulphonimidoyl)ethyl, (2*R*)-2-hydroxypropyl, (2*S*)-1-hydroxypropan-2-yl, 2-methoxyethyl, 3-methoxypropyl, 2-(isopropylsulphonyl)ethyl, (3-methyloxetan-3-yl)methyl, (2*S*)-2-hydroxypropyl, 2-(2-hydroxyethoxy)ethyl, or
$R^4$ and $R^5$ together with the directly joining nitrogen atom are piperidinyl, pyrrolidinyl, morpholinyl, N-methylpiperazinyl, 1-oxidothiomorpholinyl, 1,1-dioxidothiomorpholin-4-yl, 4-hydroxypiperidinyl, 4-(trifluoromethyl)piperidin-4-yl, (3*R*)-3-hydroxypiperidinyl, (2*S*)-2-(1*H*-tetrazol-5-yl)pyrrolidinyl, *N*-methyl-L-prolinamidyl and L-prolinamidyl,

or the tautomers, N-oxides, hydrates, solvates or salts thereof, or a mixture consisting of the above.

**3.** Compounds of the general formula (I) according to Claims 1-2, where

X is carbon substituted by hydrogen,
Y is carbon or nitrogen, where the carbon may be substituted by $R^2$,
$R^2$ is hydrogen, fluorine, chlorine, methyl, nitrile, methoxy, trifluoromethyl,
$R^3$ is hydrogen or fluorine,
$R^4$ is hydrogen, methyl, ethyl, isopropyl, propyl or cyclopropyl,
$R^5$ is hydrogen, methyl, ethyl, 2-sulphamoylethyl, 3-sulphamoylpropyl, (1*S*, 2*R*)-2-hydroxycyclopentyl, 3-hydroxy-2,2-dimethylpropyl, (1*S*, 2*S*)-2-hydroxycyclopentyl, (3*R*)-4-hydroxy-3-methylbutyl, 1-(hydroxymethyl)cyclopentyl, (2*S*)-1-hydroxybutan-2-yl, (2*R*)-1-hydroxy-3-methylbutan-2-yl, 3-hydroxybutan-2-yl, 2-hydroxyethyl, 3,3,3-trifluoro-2-hydroxypropyl, 2-(1*H*-tetrazol-5-yl)ethyl, 1*H*-tetrazol-5-ylmethyl, 2-(methylsulphamoyl)ethyl, 3-amino-3-oxopropyl, 3-(methylamino)-3-oxopropyl, 2-methyl-2-[(methylsulphonyl)amino]propyl, (2*S*)-2,3-dihydroxypropyl, 3-hydroxypropyl, (2*RS*)-2,3-dihydroxypropyl, (2*R*)-2,3-dihydroxypropyl, 2-(methylsulphinyl)ethyl, (2*R*)-2-hydroxypropyl, (2*S*)-1-hydroxypropan-2-yl, 2-methoxyethyl, 2-(isopropylsulphonyl)ethyl, (3-methyloxetan-3-yl)methyl, (2*S*)-2-hydroxypropyl or 2-(2-hydroxyethoxy)ethyl
or
$R^4$ and $R^5$ together with the directly joining nitrogen atom are piperidinyl, pyrrolidinyl, morpholinyl, 4-hydroxypiperidinyl, (3*R*)-3-hydroxypiperidinyl, (2*S*)-2-(1*H*-tetrazol-5-yl)pyrrolidinyl, *N*-methyl-L-prolinamidyl or L-prolinamidyl

or the tautomers, N-oxides, hydrates, solvates or salts thereof, or a mixture consisting of the above.

**4.** Compounds of the general formula (I) according to Claims 1-3, where

X is carbon substituted by hydrogen,
Y is carbon or nitrogen, where the carbon may be substituted by $R^2$,
$R^2$ is hydrogen, fluorine, nitrile, methoxy or trifluoromethyl,
$R^3$ is hydrogen or fluorine,
$R^4$ is hydrogen, methyl, ethyl or isopropyl,
$R^5$ is hydrogen, ethyl, 2-sulphamoylethyl, (1*S*,2*R*)-2-hydroxycyclopentyl, 3-hydroxy-2,2-dimethylpropyl, (1*S*,2*S*)-2-hydroxycyclopentyl, (3*R*)-4-hydroxy-3-methylbutyl, 1-(hydroxymethyl)cyclopentyl, (2*S*)-1-hydroxybutan-2-yl, (2*R*)-1-hydroxy-3-methylbutan-2-yl, 3-hydroxybutan-2-yl, 2-hydroxyethyl, 3,3,3-trifluoro-2-hydroxypropyl, 2-(1*H*-tetrazol-5-yl)ethyl, 1H-tetrazol-5-ylmethyl, 2-(methylsulphamoyl)ethyl, 3-amino-3-oxopropyl, 3-(methylamino)-3-oxopropyl, 2-methyl-2-[(methylsulphonyl)amino]propyl, (2*S*)-2,3-dihydroxypropyl, 3-hydroxypropyl, (2*RS*)-2,3-dihydroxypropyl, (2*R*)-2,3-dihydroxypropyl, 2-(methylsulphinyl)ethyl, (2*R*)-2-hydroxypropyl, (2*S*)-1-hydroxypropan-2-yl, 2-methoxyethyl, 2-(isopropylsulphonyl)ethyl, (3-methyloxetan-3-yl)methyl, (2*S*)-2-hydroxypropyl or 2-(2-hydroxyethoxy)ethyl, or

$R^4$ and $R^5$ together with the directly joining nitrogen atom are 4-hydroxypiperidinyl, (3*R*)-3-hydroxypiperidinyl, (2*S*)-2-(1*H*-tetrazol-5-yl)pyrrolidinyl, *N*-methyl-L-prolinamidyl or L-prolinamidyl

or the tautomers, N-oxides, hydrates, solvates or salts thereof, or a mixture consisting of the above.

**5.** Compounds according to Claims 1 - 4, selected from a group comprising the following compounds:

• 17-(3-pyridyl)oestra-1,3,5(10),16-tetraene-3-carboxamide
• 17-(5-methoxypyridin-3-yl)oestra-1,3,5(10),16-tetraene-3-carboxamide
• 17-[5-(trifluoromethyl)pyridin-3-yl]oestra-1,3,5(10),16-tetraene-3-carboxamide
• 17-(5-fluoropyridin-3-yl)oestra-1,3,5(10), 6-tetraene-3-carboxamide
• 17-(pyrimidin-5-yl)oestra-1,3,5(10),16-tetraene-3-carboxamide
• 17-(5-cyanopyridin-3-yl)oestra-1,3,5(10),16-tetraene-3-carboxamide
• 11*β*-fluoro-17-(5-fluoropyridin-3-yl)oestra-1,3,5(10),16-tetraene-3-carboxamide
• 11*β*-fluoro-17-(5-fluoropyridin-3-yl)-*N*-(2-sulphamoylethyl)oestra-1,3,5(10),16-tetraene-3-carboxamide
• 17-(5-fluoropyridin-3-yl)-*N*-[(1*S*,2*R*)-2-hydroxycyclopentyl]oestra-1,3,5(10),16-tetraene-3-carboxamide
• 17-(5-fluoropyridin-3-yl)-*N*-[2-(hydroxymethyl)-2-methylpropyl]oestra-1,3,5(10),16-tetraene-3-carboxamide
• 17-(5-fluoropyridin-3-yl)-*N*-[(1*S*,2*S*)-2-hydroxycyclopentyl]oestra-1,3,5(10),16-tetraene-3-carboxamide
• 17-(5-fluoropyridin-3-yl)-*N*-[(*R*)-3-(hydroxymethyl)butyl]oestra-1,3,5(10),16-tetraene-3-carboxamide
• 17-(5-fluoropyridin-3-yl)-*N*-[1-(hydroxymethyl)cyclopentyl]oestra-1,3,5(10),16-tetraene-3-carboxamide
• 17-(5-fluoropyridin-3-yl)oestra-1,3,5(10),16-tetraen-3-yl 4-hydroxypiperidin-1-yl ketone
• 17-(5-fluoropyridin-3-yl)-*N*-[(*S*)-1-(hydroxymethyl)propyl]oestra-1,3,5(10),16-tetraene-3-carboxamide
• 17-(5-fluoropyridin-3-yl)-*N*-[(*R*)-1-(hydroxymethyl)-2-methylpropyl]oestra-1,3,5(10),16-tetraene-3-carboxamide
• 17-(5-fluoropyridin-3-yl)oestra-1,3,5(10),16-tetraen-3-yl (*R*)-3-hydroxypiperidin-1-yl ketone
• rel-17-(5-fluoropyridin-3-yl)-N-[(1*R*,2*R*)-2-hydroxy-1-methylpropyl]oestra-1,3,5(10),16-tetraene-3-carboxamide
• 17-(5-fluoropyridin-3-yl)-*N*-(2-hydroxyethyl)-*N*-isopropyloestra-1,3,5(10),16-tetraene-3-carboxamide
• 17-(5-fluoropyridin-3-yl)-*N*-[(*RS*)-3,3,3-trifluoro-2-hydroxypropyl]oestra-1,3,5(10),16-tetraene-3-carboxamide
• 17-(5-fluoropyridin-3-yl)-*N*-[2-(1*H*-tetrazol-5-yl)ethyl]oestra-1,3,5(10),16-tetraene-3-carboxamide
• 17-(5-fluoropyridin-3-yl)-*N*-(1*H*-tetrazol-5-ylmethyl)oestra-1,3,5(10),16-tetraene-3-carboxamide
• 17-(3-pyridyl)-*N*-(2-sulphamoylethyl)oestra-1,3,5(10),16-tetraene-3-carboxamide
• *N*-(2-sulphamoylethyl)-17-[5-(trifluoromethyl)pyridin-3-yl]oestra-1,3,5(10),16-tetraene-3-carboxamide
• *N*-[2-(*N*-methylsulphamoyl)ethyl]-17-[5-(trifluoromethyl)pyridin-3-yl]oestra-1,3,5(10),16-tetraene-3-carboxamide
• *N*-(3-amino-3-oxopropyl)-17-(5-fluoropyridin-3-yl)oestra-1,3,5(10),16-tetraene-3-carboxamide
• 17-(5-fluoropyridin-3-yl)-*N*-[3-(methylamino)-3-oxopropyl]oestra-1,3,5(10),16-tetraene-3-carboxamide
• 17-(5-fluoropyridin-3-yl)oestra-1,3,5(10),16-tetraen-3-yl (*S*)-2-(1*H*-tetrazol-5-yl)pyrrolidin-1-yl ketone
• 1-{[17-(5-fluoropyridin-3-yl)oestra-1,3,5(10),16-tetraen-3-yl]carbonyl}-*N*-methyl-L-prolinamide
• 1-{[17-(5-fluoropyridin-3-yl)oestra-1,3,5(10),16-tetraen-3-yl]carbonyl}-L-prolinamide
• 17-(5-fluoropyridin-3-yl)-*N*-{2-methyl-2-[(methylsulphonyl)amino]propyl}oestra-1,3,5(10),16-tetraene-3-carboxamide
• *N*-ethyl-17-(5-fluoropyridin-3-yl)-*N*-(2-hydroxyethyl)oestra-1,3,5(10),16-tetraene-3-carboxamide
• *N*-[(*S*)-2,3-dihydroxypropyl]-17-(5-fluoropyridin-3-yl)oestra-1,3,5(10),16-tetraene-3-carboxamide
• 17-(5-fluoropyridin-3-yl)-*N*-(3-hydroxypropyl)-*N*-methyloestra-1,3,5(10),16-tetraene-3-carboxamide
• *N*-[(*RS*)-2,3-dihydroxypropyl]-17-(5-fluoropyridin-3-yl)-*N*-methyloestra-1,3,5(10),16-tetraene-3-carboxamide
• *N*-[(*R*)-2,3-dihydroxypropyl]-17-(5-fluoropyridin-3-yl)oestra-1,3,5(10),16-tetraene-3-carboxamide
• 17-(5-fluoropyridin-3-yl)-*N*-[2-(methylsulphinyl)ethyl]oestra-1,3,5(10),16-tetraene-3-carboxamide
• 17-(5-fluoropyridin-3-yl)-*N*-[(*R*)-2-hydroxypropyl]oestra-1,3,5(10),16-tetraene-3-carboxamide
• *N*-ethyl-17-(5-fluoropyridin-3-yl)oestra-1,3,5(10),16-tetraene-3-carboxamide
• 17-(5-fluoropyridin-3-yl)-*N*-[(*S*)-1-(hydroxymethyl)ethyl]oestra-1,3,5(10),16-tetraene-3-carboxamide
• 17-(5-fluoropyridin-3-yl)-*N*-(2-methoxyethyl)oestra-1,3,5(10),16-tetraene-3-carboxamide
• 17-(5-fluoropyridin-3-yl)-*N*-[2-(isopropylsulphonyl)ethyl]oestra-1,3,5(10),16-tetraene-3-carboxamide
• 17-(5-fluoropyridin-3-yl)-*N*-[(3-methyloxetan-3-yl)methyl]oestra-1,3,5(10),16-tetraene-3-carboxamide
• 17-(5-fluoropyridin-3-yl)-*N*-(2-hydroxyethyl)-*N*-methyloestra-1,3,5(10),16-tetraene-3-carboxamide
• 17-(5-fluoropyridin-3-yl)-*N*-[(*S*)-2-hydroxypropyl]oestra-1,3,5(10),16-tetraene-3-carboxamide
• 17-(5-fluoropyridin-3-yl)-*N*-[2-(2-hydroxyethoxy)ethyl]oestra-1,3,5(10),16-tetraene-3-carboxamide
• 17-(pyrimidin-5-yl)-*N*-(2-sulphamoylethyl)oestra-1,3,5(10),16-tetraene-3-carboxamide

and the tautomers, N-oxides, hydrates, solvates or salts thereof, or a mixture consisting of the above.

**6.** Compound of the formula (I) as defined in any of Claims 1 to 5 for treatment and/or prophylaxis of diseases.

**7.** Compound of the formula (I) as defined in any of Claims 1 to 5 for use in a method for treatment and/or prophylaxis of endometriosis, of leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

**8.** Use of a compound according to Claim 1, 2, 3, 4 or 5 for production of a medicament for treatment and/or prophylaxis of diseases.

**9.** Use of a compound as defined in any of Claims 1 to 5 for production of a medicament for prophylaxis of endometriosis, of leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

**10.** Medicament comprising a compound as defined in any of Claims 1 to 5 in combination with one or more further active ingredients, especially with selective oestrogen receptor modulators (SERMs), oestrogen receptor (ER) antagonists, aromatase inhibitors, 17-HSD1 inhibitors, steroid sulphatase (STS) inhibitors, GnRH agonists and antagonists, kisspeptin receptor (KISSR) antagonists, selective androgen receptor modulators (SARMs), androgens, 5-reductase inhibitors, selective progesterone receptor modulators (SPRMs), gestagens, antigestagens, oral contraceptives, inhibitors of mitogen-activated protein (MAP) kinases and inhibitors of the MAP kinases (Mkk3/6, Mek1/2, Erk1/2), inhibitors of the protein kinases B (PKB$\alpha$/$\beta$/$\gamma$; Akt1/2/3), inhibitors of the phosphoinositide 3-kinases (PI3K), inhibitors of cyclin-dependent kinase (CDK1/2), inhibitors of the hypoxia-induced signalling pathway (HIF1alpha inhibitors, activators of prolylhydroxylases), histone deacetylase (HDAC) inhibitors, prostaglandin F receptor (FP) (PTGFR) antagonists, and non-steroidal inflammation inhibitors (NSAIDs).

**11.** Medicament comprising a compound of the formula (I) as defined in any of Claims 1 to 5 in combination with an inert, nontoxic, pharmaceutically suitable excipient.

**12.** Medicament according to Claim 10 or 11 for treatment and prophylaxis of endometriosis, of uterine leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

**13.** Compounds of the general formula (I), according to Claims 1-5, for use in the form of a pharmaceutical formulation for enteral, parenteral, vaginal, intrauterine and oral administration.

## Revendications

**1.** Composés de formule générale (I)

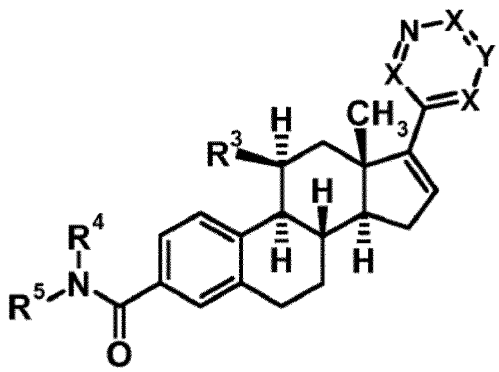

(I)

dans lesquels

les X signifient indépendamment les uns des autres carbone ou azote, le carbone pouvant être substitué avec $R^1$,
Y signifie carbone ou azote, le carbone pouvant être substitué avec $R^2$,
$R^1$ et $R^2$
signifient indépendamment l'un de l'autre hydrogène, halogène, alkyle en $C_1$-$C_6$, alcényle en $C_2$-$C_6$, alcynyle en $C_2$-$C_4$, cycloalkyle en $C_3$-$C_6$, alcoxy en $C_1$-$C_6$, halogénoalkyle en $C_1$-$C_6$, halogénoalcoxy en $C_1$-$C_6$, nitrile, nitro, $-SO_2CH_3$, $-SO_2CH_2CH_3$, $-(C{=}O)CH_3$, carboxyle, hydroxyle, $-NH_2$, $-CH_2NH_2$, $-CH_2OH$, $-CH(OH)CH_3$, $-C(CH_3)_2OH$, $-(C{=}O)NH_2$, $-(C{=}O)NHCH_3$, - $(C{=}O)NHCH_2CH_3$,$-(C{=}O)N(CH_3)_2$, $-SO_2NH_2$, $-SO_2NHCH_3$ ou $-SO_2N(CH_3)_2$,
$R^3$ signifie hydrogène ou halogène,
$R^4$ signifie hydrogène, alkyle en $C_1$-$C_6$, cycloalkyle en $C_3$-$C_6$ ou cycloalkyle en $C_3$-$C_6$-alkyle en $C_1$-$C_6$, qui sont éventuellement substitués avec jusqu'à 6 atomes d'halogène, et qui sont éventuellement substitués une ou deux fois avec hydroxyle, alcoxy en $C_1$-$C_6$, halogénoalcoxy en $C_1$-$C_6$,
$R^5$ signifie hydrogène, alkyle en $C_1$-$C_6$ ou cycloalkyle en $C_3$-$C_6$, l'alkyle en $C_1$-$C_6$ et le cycloalkyle en $C_3$-$C_6$ étant éventuellement substitués avec jusqu'à 6 atomes d'halogène et étant éventuellement substitués une ou deux fois avec hydroxyle, hydroxy-alkyle en $C_1$-$C_6$, alkyle en $C_1$-$C_6$, alcoxy en $C_1$-$C_6$, hydroxy-alcoxy en $C_2$-$C_6$, halogénoalcoxy en $C_1$-$C_6$, cycloalkyle en $C_3$-$C_6$, aryle, hétéroaryle, hétérocycloalkyle de 3 à 10 chaînons, aryl-alkyle en $C_1$-$C_6$, hétéroaryl-alkyle en $C_1$-$C_6$,-C(=O)R', -C(=O)$NH_2$, -C(=O)N(H)R', -C(=O)N(R')R", $-NH_2$, -NHR', -N(R')R", -N(H)C(=O)R', -N(R')C(=O)R',-N(H)C(=O)OR', -N(R')C(=O)OR', $-NO_2$, -N(H)S(=O)R',-N(R')S(=O)R', -N(H)S$(=O)_2$R', -N(R')S$(=O)_2$R',-N=S (=O)(R')R", -S(=O)R', -S$(=O)_2$R', -S$(=O)_2NH_2$,-S$(=O)_2$NHR', -S$(=O)_2$N(R')R", -S(=O)(=NR')R", l'aryle, l'hétéroaryle, l'aryle-alkyle en $C_1$-$C_6$ et l'hétéroaryl-alkyle en $C_1$-$C_6$ étant éventuellement substitués une ou plusieurs fois indépendamment les unes des autres avec $R^6$ et l'hétérocycloalkyle de 3 à 10 éléments étant éventuellement substitué une ou plusieurs fois indépendamment les unes des autres avec R',
ou
$R^4$ et $R^5$ signifient ensemble avec l'atome d'azote directement relié un cycle de 4 à 7 chaînons, qui est éventuellement substitué avec un ou deux substituants constitués par :

halogène, nitrile, hydroxyle, alkyle en $C_1$-$C_6$, halogénoalkyle en $C_1$-$C_6$, alcényle en $C_2$-$C_6$, alcynyle en $C_2$-$C_4$, cycloalkyle en $C_3$-$C_{10}$, aryle, hétéroaryle,-C(=O)$NH_2$, -C(=O)N(H)R', -C(=O)N(R')R", -C(=O)OR', $-NH_2$, -NHR', -N(R')R", -N(H)C(=O)R', -N(R')C(=O)R',-N(H)S(=O)R', -N(R')S(=O)R', -N(H)S$(=O)_2$R',-N(R')S$(=O)_2$R', -N=S (=O)(R')R", -OH, alcoxy en $C_1$-$C_6$, halogénoalcoxy en $C_1$-$C_6$, -OC(=O)R', -OC(=O)$NH_2$,-OC(=O)NHR', -OC(=O)N(R')R", -SH, alkyle en $C_1$-$C_6$-S-,-S(=O)R', -S$(=O)_2$R', -S$(=O)_2NH_2$, -S$(=O)_2$NHR',-S$(=O)_2$N(R')R", l'aryle et l'hétéroaryle étant éventuellement substitués une ou plusieurs fois indépendamment l'une de l'autre avec $R^6$,
et dans lequel cycle à 5, 6 ou 7 chaînons un ou plusieurs groupes méthylène sont éventuellement remplacés par NH, NR', O ou S,
$R^6$ signifie halogène, nitrile, alkyle en $C_1$-$C_6$, halogénoalkyle en $C_1$-$C_6$, alcényle en $C_2$-$C_6$, alcynyle en $C_2$-$C_4$, cycloalkyle en $C_3$-$C_{10}$, hétérocycloalkyle de 3 à 10 chaînons, aryle, hétéroaryle, -C(=O)R', -C(=O)$NH_2$,-C(=O)N(H)R', -C(=O)N(R')R", -C(=O)OR', $-NH_2$, -NHR',-N(R')R", -N(H)C(=O)R', -N(R')C(=O)R', -N(H)C(=O)$NH_2$,-N(H)C(=O)NHR', -N(H)C(=O)N(R')R", -N(R')C(=O)$NH_2$,-N(R')C(=O)NHR', -N(R')C(=O)N(R')R", -N(H)C(=O)OR',-N(R')C(=O)OR', $-NO_2$,

-N(H)S(=O)R', -N(R')S(=O)R',-N(H)S(=O)$_2$R', -N(R')S(=O)$_2$R', -N=S(=O)(R')R", -OH, alcoxy en $C_1$-$C_6$, halogénoalcoxy en $C_1$-$C_6$, -OC(=O)R',-OC(=O)$NH_2$, -OC(=O)NHR', -OC(=O)N(R')R", -SH, alkyle en $C_1$-$C_6$-S-, -S (=O)R', -S(=O)$_2$R', -S(=O)$_2$$NH_2$, -S(=O)$_2$NHR',-S(=O)$_2$N(R')R", - S(=O)(=NR')R",
R' et R" signifient indépendamment l'un de l'autre alkyle en $C_1$-$C_6$, cycloalkyle en $C_3$-$C_{10}$ ou halogénoalkyle en $C_1$-$C_6$,
ou leurs tautomères, N-oxydes, hydrates, solvates ou sels ou un mélange constitué des précédents, « hétérocycloalkyle » représentant un hétérocycle saturé contenant au total 3 à 10 atomes de cycle, qui contient un ou deux hétéroatomes de cycle de la série constituée par N, O, S, SO et/ou $SO_2$ ; « aryle » représentant un radical carbocyclique aromatique, mono-à tricyclique, contenant généralement 6 à 14 atomes de carbone ; « hétéroaryle » représentant un hétérocycle aromatique monocyclique contenant au total 5 ou 6 atomes de cycle, qui contient jusqu'à quatre hétéroatomes de cycle identiques ou différents de la série constituée par N, O et/ou S, et qui est relié par un atome de carbone de cycle ou éventuellement par un atome d'azote de cycle ; l'hétérocycle aromatique monocyclique susmentionné étant éventuellement substitué avec hydroxyle ou SH ; « aryl-alkyle en $C_1$-$C_6$ » représentant un radical carbocyclique aromatique mono- à tricyclique contenant généralement 6 à 14 atomes de carbone, qui est relié par un radical alkyle linéaire ou ramifié contenant le nombre indiqué à chaque fois d'atomes de carbone, et « hétéroaryl-alkyle en $C_1$-$C_6$ » représentant un hétérocycle aromatique monocyclique contenant au total 5 ou 6 atomes de cycle, qui contient jusqu'à trois hétéroatomes de cycle identiques ou différents de la série constituée par N, O et/ou S, et est relié par un atome de carbone de cycle ou éventuellement par un atome d'azote de cycle, et qui est relié par un radical alkyle linéaire ou ramifié contenant le nombre indiqué à chaque fois d'atomes de carbone.

**2.** Composés de formule générale (I) selon la revendication 1, dans lesquels
les X signifient carbone substitué avec hydrogène,
Y signifie carbone ou azote, le carbone pouvant être substitué avec $R^2$,
$R^2$ signifie hydrogène, fluor, chlore, nitrile, méthoxy, éthoxy, trifluorométhoxy, méthyle, éthyle, trifluorométhyle, -(C=O)$CH_3$,
$R^3$ signifie hydrogène ou fluor,
$R^4$ signifie hydrogène, méthyle, éthyle, isopropyle, propyle, butyle, cyclopropyle ou 2,2,2-trifluoroéthyle, $R^5$ signifie hydrogène, méthyle, éthyle, propyle, 2,2,2,-trifluoroéthyle, 3,3,3-trifluoropropyle, 2-fluoroéthyle, 2-sulfamoyléthyle, 3-sulfamoylpropyle, (1*S*,2*R*)-2-hydroxycyclopentyle, 3-hydroxy-2,2-diméthylpropyle, (1*S*,2*S*)-2-hydroxycyclopentyle, (3*R*)-4-hydroxy-3-méthylbutyle, 1-(hydroxyméthyl)cyclopentyle, (2*S*)-1-hydroxybutan-2-yle, (2*R*)-1-hydroxy-3-méthylbutan-2-yle, 3-hydroxybutan-2-yle, 2-hydroxyéthyle, 3,3,3-trifluoro-2-hydroxypropyle, 2-(1*H*-tétrazol-5-yl)éthyle, 1*H*-tétrazol-5-ylméthyle, 2-(méthylsulfamoyl)éthyle, 3-amino-3-oxopropyle, 3-(méthylamino)-3-oxopropyle, 2-méthyl-2-[(méthylsulfonyl)amino]propyle, (2*S*)-2,3-dihydroxypropyle, 3-hydroxypropyle, 4-hydroxybutyle, (2*RS*)-2,3-dihydroxypropyle, (2*R*)-2,3-dihydroxypropyle, 2,3-dihydroxybutyle, 2-(méthylsulfinyl)éthyle, 3-(méthylsulfinyl)propyle, 2-(méthylsulfonyl)éthyle, 3-(méthylsulfonyl)propyle, 2-(*S*-méthylsulfonimidoyl)éthyle, (2*R*)-2-hydroxypropyle, (2*S*)-1-hydroxypropan-2-yle, 2-méthoxyéthyle, 3-méthoxypropyle, 2-(isopropylsulfonyl)éthyle, (3-méthyloxétan-3-yl)méthyle, (2*S*)-2-hydroxypropyle, 2-(2-hydroxyéthoxy)éthyle,
ou
$R^4$ et $R^5$ signifient ensemble avec l'atome d'azote directement relié pipéridinyle, pyrrolidinyle, morpholinyle, N-méthylpipérazinyle, 1-oxydothiomorpholinyle, 1,1-dioxydothiomorpholin-4-yle, 4-hydroxypipéridinyle, 4-(trifluorométhyl)pipéridin-4-yle, (3*R*)-3-hydroxypipéridinyle, (2*S*)-2-(1*H*-tétrazol-5-yl)pyrrolidinyle, N-méthyl-L-prolinamidyle et L-prolinamidyle,
ou leurs tautomères, N-oxydes, hydrates, solvates ou sels ou un mélange constitué des précédents.

**3.** Composés de formule générale (I) selon les revendications 1 à 2, dans lesquels
les X signifient carbone substitué avec hydrogène,
Y signifie carbone ou azote, le carbone pouvant être substitué avec $R^2$,
$R^2$ signifie hydrogène, fluor, chlore, méthyle, nitrile, méthoxy, trifluorométhyle,
$R^3$ signifie hydrogène ou fluor,
$R^4$ signifie hydrogène, méthyle, éthyle, isopropyle, propyle ou cyclopropyle,
$R^5$ signifie hydrogène, méthyle, éthyle, 2-sulfamoyléthyle, 3-sulfamoylpropyle, (1*S*,2*R*)-2-hydroxycyclopentyle, 3-hydroxy-2,2-diméthylpropyle, (1*S*,2*S*)-2-hydroxycyclopentyle, (3*R*)-4-hydroxy-3-méthylbutyle, 1-(hydroxyméthyl)cyclopentyle, (2*S*)-1-hydroxybutan-2-yle, (2*R*)-1-hydroxy-3-méthylbutan-2-yle, 3-hydroxybutan-2-yle, 2-hydroxyéthyle, 3,3,3-trifluoro-2-hydroxypropyle, 2-(1*H*-tétrazol-5-yl)éthyle, 1*H*-tétrazol-5-ylméthyle, 2-(méthylsulfamoyl)éthyle, 3-amino-3-oxopropyle, 3-(méthylamino)-3-oxopropyle, 2-méthyl-2-[(méthylsulfonyl)amino]propyle, (2*S*)-2,3-dihydroxypropyle, 3-hydroxypropyle, (2*RS*)-2,3-dihydroxypropyle, (2*R*)-2,3-dihydroxypropyle, 2-(méthylsulfinyl)éthyle, (2*R*)-2-hydroxypropyle, (2*S*)-1-hydroxypropan-2-yle, 2-méthoxyéthyle, 2-(isopropylsulfonyl)éthyle, (3-méthyloxétan-3-yl)méthyle, (2*S*)-2-hydroxypropyle ou 2-(2-hydroxyéthoxy)éthyle,
ou
$R^4$ et $R^5$ signifient ensemble avec l'atome d'azote directement relié pipéridinyle, pyrrolidinyle, morpholinyle, 4-hydroxypipéridinyle, (3*R*)-3-hydroxypipéridinyle, (2*S*)-2-(1*H*-tétrazol-5-yl)pyrrolidinyle, N-méthyl-L-prolinamidyle ou L-prolinamidyle,
ou leurs tautomères, N-oxydes, hydrates, solvates ou sels ou un mélange constitué des précédents.

**4.** Composés de formule générale (I) selon les revendications 1 à 3, dans lesquels
les X signifient carbone substitué avec hydrogène,
Y signifie carbone ou azote, le carbone pouvant être substitué avec $R^2$,
$R^2$ signifie hydrogène, fluor, nitrile, méthoxy ou trifluorométhyle,
$R^3$ signifie hydrogène ou fluor,
$R^4$ signifie hydrogène, méthyle, éthyle ou isopropyle,
$R^5$ signifie hydrogène, éthyle, 2-sulfamoyléthyle, (1*S*,2*R*)-2-hydroxycyclopentyle, 3-hydroxy-2,2-diméthylpropyle, (1*S*,2*S*)-2-hydroxycyclopentyle, (3*R*)-4-hydroxy-3-méthylbutyle, 1-(hydroxyméthyl)cyclopentyle, (2*S*)-1-hydroxybutan-2-yle, (2*R*)-1-hydroxy-3-méthylbutan-2-yle, 3-hydroxybutan-2-yle, 2-hydroxyéthyle, 3,3,3-trifluoro-2-hydroxypropyle, 2-(1*H*-tétrazol-5-yl)éthyle, 1*H*-tétrazol-5-ylméthyle, 2-(méthylsulfamoyl)éthyle, 3-amino-3-oxopropyle, 3-(méthylamino)-3-oxopropyle, 2-méthyl-2-[(méthylsulfonyl)amino]propyle, (2*S*)-2,3-dihydroxypropyle, 3-hydroxypropyle, (2*RS*)-2,3-dihydroxypropyle, (2*R*)-2,3-dihydroxypropyle, 2-(méthylsulfinyl)éthyle, (2*R*)-2-hydroxypropyle, (2*S*)-1-hydroxypropan-2-yle, 2-méthoxyéthyle, 2-(isopropylsulfonyl)éthyle, (3-méthyloxétan-3-yl)méthyle, (2*S*)-2-hydroxypropyle ou 2-(2-hydroxyéthoxy)éthyle,
ou
$R^4$ et $R^5$ signifient ensemble avec l'atome d'azote directement relié 4-hydroxypipéridinyle, (3*R*)-3-hydroxypipéridinyle, (2*S*)-2-(1*H*-tétrazol-5-yl)pyrrolidinyle, N-méthyl-L-prolinamidyle ou L-prolinamidyle,
ou leurs tautomères, N-oxydes, hydrates, solvates ou sels ou un mélange constitué des précédents.

**5.** Composés selon les revendications 1 à 4, choisis dans un groupe qui contient les composés suivants :

- 17-(3-pyridyl)estra-1,3,5(10),16-tétraène-3-carboxamide
- 17-(5-méthoxypyridin-3-yl)estra-1,3,5(10),16-tétraène-3-carboxamide
- 17-[5-(trifluorométhyl)pyridin-3-yl]estra-1,3,5(10),16-tétraène-3-carboxamide
- 17-(5-fluoropyridin-3-yl)estra-1,3,5(10),16-tétraène-3-carboxamide
- 17-(pyrimidin-5-yl)estra-1,3,5(10),16-tétraène-3-carboxamide
- 17-(5-cyanopyridin-3-yl)estra-1,3,5(10),16-tétraène-3-carboxamide
- 11β-fluoro-17-(5-fluoropyridin-3-yl)estra-1,3,5(10),16-tétraène-3-carboxamide
- 11β-fluoro-17-(5-fluoropyridin-3-yl)-N-(2-sulfamoyléthyl)estra-1,3,5(10),16-tétraène-3-carboxamide
- 17-(5-fluoropyridin-3-yl)-N-[(1*S*,2*R*)-2-hydroxycyclopentyl]estra-1,3,5(10),16-tétraène-3-carboxamide
- 17-(5-fluoropyridin-3-yl)-N-[2-(hydroxyméthyl)-2-méthylpropyl]estra-1,3,5(10),16-tétraène-3-carboxamide
- 17-(5-fluoropyridin-3-yl)-N-[(1*S*,2*S*)-2-hydroxycyclopentyl]estra-1,3,5(10),16-tétraène-3-carboxamide
- 17-(5-fluoropyridin-3-yl)-N-[(*R*)-3-(hydroxyméthyl)butyl]estra-1,3,5(10),16-tétraène-3-carboxamide
- 17-(5-fluoropyridin-3-yl)-N-[1-(hydroxyméthyl)cyclopentyl]estra-1,3,5(10),16-tétraène-3-carboxamide
- [17-(5-fluoropyridin-3-yl)estra-1,3,5(10),16-tétraén-3-yl](4-hydroxypipéridin-1-yl)cétone
- 17-(5-fluoropyridin-3-yl)-N-[(*S*)-1-(hydroxyméthyl)propyl]estra-1,3,5(10),16-tétraène-3-carboxamide
- 17-(5-fluoropyridin-3-yl)-N-[(*R*)-1-(hydroxyméthyl)-2-méthylpropyl]estra-1,3,5(10),16-tétraène-3-carboxamide
- [17-(5-fluoropyridin-3-yl)estra-1,3,5(10),16-tétraène-3-yl][(*R*)-3-hydroxypipéridin-1-yl]cétone
- rel-17-(5-fluoropyridin-3-yl)-N-[(1*R*,2*R*)-2-hydroxy-1-méthylpropyllestra-1,3,5(10),16-tétraène-3-carboxamide
- 17-(5-fluoropyridin-3-yl)-N-(2-hydroxyéthyl)-N-isopropylestra-1,3,5(10),16-tétraène-3-carboxamide
- 17-(5-fluoropyridin-3-yl)-N-[(RS)-3,3,3-trifluoro-2-hydroxypropyl]estra-1,3,5(10),16-tétraène-3-carboxamide
- 17-(5-fluoropyridin-3-yl)-N-[2-(1*H*-tétrazol-5-yl)éthyl]estra-1,3,5(10),16-tétraène-3-carboxamide
- 17-(5-fluoropyridin-3-yl)-N-(1*H*-tétrazol-5-ylméthyl)estra-1,3,5(10),16-tétraène-3-carboxamide
- 17-(3-pyridyl)-N-(2-sulfamoyléthyl)estra-1,3,5(10),16-tétraène-3-carboxamide
- N-(2-sulfamoyléthyl)-17-[5-(trifluorométhyl)pyridin-3-yl]estra-1,3,5(10),16-tétraène-3-carboxamide
- N-[2-(N-méthylsulfamoyl)éthyl]-17-[5-(trifluorométhyl)pyridin-3-yl]estra-1,3,5(10),16-tétraène-3-carboxamide
- N-(3-amino-3-oxopropyl)-17-(5-fluoropyridin-3-yl)estra-1,3,5(10),16-tétraène-3-carboxamide
- 17-(5-fluoropyridin-3-yl)-N-[3-(méthylamino)-3-oxopropyl]estra-1,3,5(10),16-tétraène-3-carboxamide
- [17-(5-fluoropyridin-3-yl)estra-1,3,5(10),16-tétraén-3-yl][(*S*)-2-(1*H*-tétrazol-5-yl)pyrrolidin-1-yl]cétone

- 1-{[17-(5-fluoropyridin-3-yl)estra-1,3,5(10),16-tétraén-3-yl]carbonyl}-N-méthyl-L-prolinamide
- 1-{[17-(5-fluoropyridin-3-yl)estra-1,3,5(10),16-tétraén-3-yl]carbonyl}-L-prolinamide
- 17-(5-fluoropyridin-3-yl)-N-{2-méthyl-2-[(méthylsulfonyl)amino]propyl}estra-1,3,5(10),16-tétraène-3-carboxamide
- N-éthyl-17-(5-fluoropyridin-3-yl)-N-(2-hydroxyéthyl)estra-1,3,5(10),16-tétraène-3-carboxamide
- N-[(*S*)-2,3-dihydroxypropyl]-17-(5-fluoropyridin-3-yl)estra-1,3,5(10),16-tétraène-3-carboxamide
- 17-(5-fluoropyridin-3-yl)-N-(3-hydroxypropyl)-N-méthylestra-1,3,5(10),16-tétraène-3-carboxamide
- N-[(*RS*)-2,3-dihydroxypropyl]-17-(5-fluoropyridin-3-yl)-N-méthylestra-1,3,5(10),16-tétraène-3-carboxamide
- N-[(*R*)-2,3-dihydroxypropyl]-17-(5-fluoropyridin-3-yl)estra-1,3,5(10),16-tétraène-3-carboxamide
- 17-(5-fluoropyridin-3-yl)-N-[2-(méthylsulfinyl)éthyl]estra-1,3,5(10),16-tétraène-3-carboxamide
- 17-(5-fluoropyridin-3-yl)-N-[(*R*)-2-hydroxypropyl]estra-1,3,5(10),16-tétraène-3-carboxamide
- N-éthyl-17-(5-fluoropyridin-3-yl)estra-1,3,5(10),16-tétraène-3-carboxamide
- 17-(5-fluoropyridin-3-yl)-N-[(*S*)-1-(hydroxyméthyl)éthyl]estra-1,3,5(10),16-tétraène-3-carboxamide
- 17-(5-fluoropyridine-3-yl)-N-(2-méthoxyéthyl)estra-1,3,5(10),16-tétraène-3-carboxamide
- 17-(5-fluoropyridin-3-yl)-N-[2-(isopropylsulfonyl)éthyl]estra-1,3,5(10),16-tétraène-3-carboxamide
- 17-(5-fluoropyridin-3-yl)-N-[(3-méthyloxétan-3-yl)méthyl]estra-1,3,5(10),16-tétraène-3-carboxamide
- 17-(5-fluoropyridin-3-yl)-N-(2-hydroxyéthyl)-N-méthylestra-1,3,5(10),16-tétraène-3-carboxamide
- 17-(5-fluoropyridin-3-yl)-N-[(*S*)-2-hydroxypropyl]estra-1,3,5(10),16-tétraène-3-carboxamide
- 17-(5-fluoropyridin-3-yl)-N-[2-(2-hydroxyéthoxy)éthyl]estra-1,3,5(10),16-tétraène-3-carboxamide
- 17-(pyrimidin-5-yl)-N-(2-sulfamoyléthyl)estra-1,3,5(10),16-tétraène-3-carboxamide,

et leurs tautomères, N-oxydes, hydrates, solvates ou sels ou un mélange constitué par les précédents.

**6.** Composé de formule (I), tel que défini dans l'une quelconque des revendications 1 à 5, pour le traitement et/ou la prophylaxie de maladies.

**7.** Composé de formule (I), tel que défini dans l'une quelconque des revendications 1 à 5, destiné à une utilisation dans un procédé de traitement et/ou de prophylaxie de l'endométriose, de léiomyomes, de troubles de saignement utérin, de la dysménorrhée, du cancer de la prostate, de l'hyperplasie de la prostate, de l'acné, de la séborrhée, de la chute de cheveux, de la maturité sexuelle précoce, du syndrome polycystique ovarien, du cancer du sein, du cancer du poumon, du cancer de l'endomètre, de l'hypernéphrome, du cancer de la vessie, du lymphome non-hodgkinien, des bronchopneumopathies obstructives chroniques (BPCO), de l'adiposité ou de la douleur inflammatoire.

**8.** Utilisation d'un composé selon la revendication 1, 2, 3, 4 ou 5 pour la fabrication d'un médicament pour le traitement et/ou la prophylaxie de maladies.

**9.** Utilisation d'un composé, tel que défini dans l'une quelconque des revendications 1 à 5, pour la fabrication d'un médicament pour la prophylaxie de l'endométriose, de léiomyomes, de troubles de saignement utérin, de la dysménorrhée, du cancer de la prostate, de l'hyperplasie de la prostate, de l'acné, de la séborrhée, de la chute de cheveux, de la maturité sexuelle précoce, du syndrome polycystique ovarien, du cancer du sein, du cancer du poumon, du cancer de l'endomètre, de l'hypernéphrome, du cancer de la vessie, du lymphome non-hodgkinien, des bronchopneumopathies obstructives chroniques (BPCO), de l'adiposité ou de la douleur inflammatoire.

**10.** Médicament contenant un composé, tel que défini dans l'une quelconque des revendications 1 à 5, en combinaison avec un ou plusieurs autres agents actifs, notamment avec des modulateurs sélectifs des récepteurs aux oestrogènes (SERM), des antagonistes des récepteurs aux oestrogènes (ER), des inhibiteurs d'aromatase, des inhibiteurs de 17 HSD1, des inhibiteurs de stéroïde sulfatase (STS), des agonistes et antagonistes de GnRH, des antagonistes des récepteurs de kisspeptine (KISSR), des modulateurs sélectifs des récepteurs aux androgènes (SARM), des androgènes, des inhibiteurs de 5-réductase, des modulateurs sélectifs des récepteurs de la progestérone (SPRM), des gestagènes, des antigestagènes, des contraceptifs oraux, des inhibiteurs des protéines kinases activées par des mitogènes (MAP), ainsi que des inhibiteurs des MAP kinases kinases (Mkk3/6, Mek1/2, Erk1/2), des inhibiteurs des protéine kinases B (PKB$\alpha/\beta/\gamma$; Akt1/2/3), des inhibiteurs des phosphoinositide-3-kinases (PI3K), des inhibiteurs de la kinase dépendante de la cycline (CDK1/2), des inhibiteurs de la voie de signalisation induite par l'hypoxie (inhibiteurs d'HIF1alpha, activateurs des prolylhydroxylases), des inhibiteurs d'histone désacétylase (HDAC), des antagonistes des récepteurs de prostaglandine F (PF) (PTGFR) et des anti-inflammatoires non stéroïdiens (AINS).

**11.** Médicament contenant un composé de formule (I) tel que défini dans l'une quelconque des revendications 1 à 5,

en combinaison avec un adjuvant inerte, non toxique, pharmaceutiquement approprié.

**12.** Médicament selon la revendication 10 ou 11 pour le traitement et la prophylaxie de l'endométriose, de léiomyomes, de troubles de saignement utérin, de la dysménorrhée, du cancer de la prostate, de l'hyperplasie de la prostate, de l'acné, de la séborrhée, de la chute de cheveux, de la maturité sexuelle précoce, du syndrome polycystique ovarien, du cancer du sein, du cancer du poumon, du cancer de l'endomètre, de l'hypernéphrome, du cancer de la vessie, du lymphome non-hodgkinien, des bronchopneumopathies obstructives chroniques (BPCO), de l'adiposité ou de la douleur inflammatoire.

**13.** Composés de formule générale (I) selon les revendications 1 à 5, destinés à une utilisation sous la forme d'une préparation pharmaceutique pour l'application entérale, parentérale, vaginale, intrautérine et orale.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20100190826 A
- WO 2007100066 A
- US 6541463 B, F. Labrie
- US 5604213 A, S. E. Barrie
- US 20050203075 A


### In der Beschreibung aufgeführte Nicht-Patentliteratur


- **T. M. PENNING.** Aldo-keto reductase (AKR) 1C3: Role in prostate disease and the development of specific inhibitors. *Molecular and Cellular Endocrinology,* 2006, vol. 248 (1-2), 182-191
- **A. OSTER.** *J. Med. Chem.,* 2010, vol. 53, 8176-8186
- **B. DELVOUX et al.** *J Clin Endocrinol Metab.,* 2009, vol. 94, 876-883
- **T. SMUC et al.** *Mol Cell Endocrinol.,* 25. Marz 2009, vol. 301 (1-2), 59-64
- **M.C. BYRNS ; Y. JIN ; T.M. PENNING.** *Journal of Steroid Biochemistry and Molecular Biology,* 2010
- **A. L. LOVERING.** *Cancer Res,* vol. 64 (5), 1802-1810
- **K. M. FUNG et al.** *Endocr Relat Cancer,* vol. 13 (1), 169-180
- **R. O. ROBERTS et al.** *Prostate,* vol. 66 (4), 392-404
- **T. L. RIZNER et al.** *Mol Cell Endocrinol,* 2006, vol. 248 (1-2), 126-135
- **K. QIN et al.** *J Endocrinol Metab,* 2006, vol. 91 (1), 270-276
- **Q. LAN et al.** *Carcinogenesis,* 2004, vol. 25 (11), 2177-2181
- **Q. LAN et al.** *Hum Genet,* 2007, vol. 121 (2), 161-168
- **L. COLOMBE et al.** *Exp Dermatol,* 2007, vol. 16 (9), 762-769
- **P. A. SVENSSON et al.** *Cell Mol Biol Lett,* 2008, vol. 13 (4), 599-613
- **J. D. FIGUEROA.** *Carcinogenesis,* 2008, vol. 29 (10), 1955-1962
- **J. BIRTWISTLE.** *Mutat Res,* 2009, vol. 662 (1-2), 67-74
- **J. T. AZZARELLO.** *Int J Clin Exp Pathol,* 2009, vol. 3 (2), 147-155
- **M. C. BYRNS.** *J Steroid Biochem Mol Biol,* 2010, vol. 118 (3), 177-187
- **C. HE.** *Hum Genet,* 2010, vol. 128 (5), 515-527
- **S. PIERROU.** *Am J Respir Crit Care,* 2007, vol. 175 (6), 577-586
- **JOANNA M DAY ; HELENA J TUTILL ; ATUL PUROHIT ; MICHAEL J REED.** *Endocrine-Related Cancer,* 2008, vol. 15, 665-692
- **P. BROŽIČ et al.** *J. Med. Chem.,* 2012, vol. 55, 7417-7424
- **A. O. ADENIJII et al.** *J. Med. Chem.,* 2012, vol. 55, 2311-2323
- **S. M. F. JAMIESON et al.** *J. Med. Chem.,* 2012, vol. 55, 7746-7758
- **P. BYDAL ; VAN LUU-THE ; F. LABRIE ; D. POIRIER.** *European Journal of Medicinal Chemistry,* 2009, vol. 44, 632-644
- **D. DELUCA ; G. MOLLER ; A. ROSINUS ; W. ELGER ; A. HILLISCH ; J. ADAMSKI.** *Mol. Cell. Endocrinol.,* 2006, vol. 248, 218-224
- **V. M. MOREIRA et al.** *Current Medicinal Chemistry,* 2008, vol. 15 (9
- *Steroids,* 1995, vol. 60 (3), 299-306
- *J. Med. Chem.,* 1995, vol. 38, 2463-2471
- *J. Org. Chem.,* 1985, vol. 50, 1990-1992
- *JACS,* 2009, vol. 131, 9014-9019
- *Archiv der Pharmazie,* 2001, vol. 334 (12), 373-374
- **D. M. KNAPP et al.** *J. Am. Chem. Soc.,* 2009, vol. 131, 6961
- **G. A. MOLANDER et al.** *J. Org. Chem.,* 2009, vol. 74, 973
- *CHEMICAL ABSTRACTS,* 905459-27-0
- **D. M. KNAPP.** *J. Am. Chem. Soc.,* 2009, vol. 131, 6961
- **G. A. MOLANDER.** *J. Org. Chem.,* 2009, vol. 74, 973
- *CHEMICAL ABSTRACTS,* 25952-53-8
- *CHEMICAL ABSTRACTS,* 123333-53-9
- *CHEMICAL ABSTRACTS,* 5210-15-1
- **E. MORERA ; G. ORTAR.** *THL,* 1998, vol. 39, 2835-2838
- *Journal of the Chemical Society,* 1964, 5889

- **A. MCKILLOP ; D. KEMP.** *Tetrahedron,* 1989, vol. 45 (11), 3299-3306
- **HALIM, M. ; YEE, D.J. ; SAMES, D.** *J. AM. CHEM. SOC.,* 2008, vol. 130, 14123-14128
- **YEE, D.J. ; BALSANEK, V. ; BAUMAN, D.R. ; PENNING, T.M. ; SAMES, D.** *Proc. Natl. Acad. Sci. USA,* 2006, vol. 103, 13304-13309
- **DUFORT, I. ; RHEAULT, P. ; HUANG, XF. ; SOUCY, P. ; LUU-THE, V.** *Endocrinology,* 1999, vol. 140, 568-574
- **YEE, D.J. ; BALSANEK, V. ; BAUMAN, D.R. ; PENNING, T.M. ; SAMES, D.** *Proc. Natl. Acad. Sci. USA,* 2006, vol. 103, 13304-13309
- *Urologe,* 2010, vol. 49, 64-68
- **EHMER, P. B. et al.** *J. Steroid Biochem. Mol. Biol.,* 2000, vol. 75, 57-63